# EUROPEAN PATENT APPLICATION

(11) **EP 4 169 513 A1**
(43) Date of publication of application: **26.04.2023**
(21) Application number: 21203571.1
(22) Date of filing: 19.10.2021
(51) Int. Cl.: A61K 31/4155, A61K 31/4184, A61K 39/39

(54) **ADJUVANT COMPOSITION COMPRISING STING AGONISTS**

(71) Applicant: GlaxoSmithKline Biologicals S.A., 1330 Rixensart (BE)
(72) Inventor: The designation of the inventor has not yet been filed
(74) Representative: Matley, Joshua Ellis

(57) **Abstract**

The present application relates to an adjuvant composition comprising:
(i) a STING agonist of Formula (I) or a pharmaceutically acceptable salt thereof 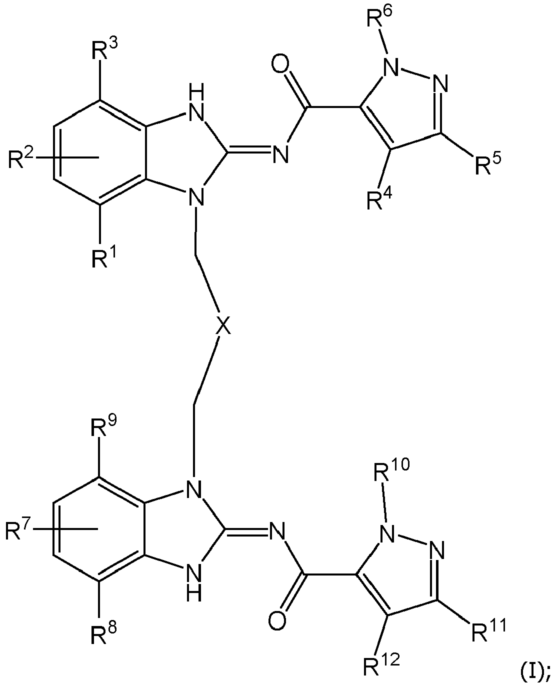 and
(ii) aluminium hydroxide, aluminium phosphate, aluminium oxyhydroxide, or aluminium hydroxyphosphate;
immunogenic compositions comprising the adjuvant composition, their use in methods of immunising a subject, and related aspects thereof.

## Description

### FIELD OF THE INVENTION

The present application relates to the field of adjuvant compositions, immunogenic compositions including the adjuvant composition, their use in a method of immunising a subject, and to related aspects.

### BACKGROUND TO THE INVENTION

Vaccine adjuvants are included in formulations to enhance humoral and cellular immune responses, particularly in the case of poorly immunogenic subunit vaccines or patients with challenging immune status (e.g., infants, elderly adults, and immune comprised patients). Similar to natural infections by pathogens, adjuvants rely on the activation of the innate immune system to promote long-lasting adaptive immunity. As simultaneous activation of multiple innate immune pathways is a feature of natural infections, adjuvants may combine multiple immunostimulants in order to promote adaptive immune responses to vaccination. The Adjuvant System 01 (AS01) is of particular interest due to its ability to promote antigen-specific CD4+ T cells, as well as antigen-specific antibodies. The AS01 adjuvant system relies on the synergistic activities of two immunoenhancers, 3-O-desacyl-4'-monophosphoryl lipid A (3D-MPL) and QS-21, and is formulated as a liposome-based formulation (Garcon and Van Mechelen, 2011; Didierlaurent et al., 2017). QS-21 is a purified plant extract, and is therefore complex to synthesize and dependent from a natural source. It is therefore desirable to develop adjuvant systems that induce similar or even better immune responses and/or show a similar or improved reactogenicity profile, but are easier to prepare.

Recently developed STING (STimulator of InterferoN Genes) agonists have been proposed as vaccine adjuvants. Specifically, WO 2017/175147 (PCT/IB2017/051945) discloses a series of dimeric amidobenzimidazole (diABZI) based compounds and their use as vaccine adjuvants. However, there remains a need to develop STING agonists formulations that are capable of providing adjuvant compositions having an improved immunogenicity and/or reduced reactogenicity.

### SUMMARY OF THE INVENTION

In a first aspect, the present invention provides an adjuvant composition comprising:
(i) a STING agonist of Formula (I) or a pharmaceutically acceptable salt thereof 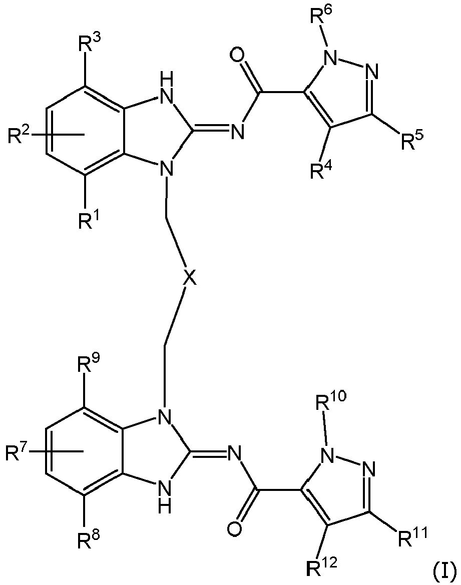 wherein
   X is ―halo(C₁-C₅)alkyl, unsubstituted ―C₁-C₅ alkyl, or unsubstituted ―C₂-C₅ alkenyl;
   R¹ and R⁹ are independently H, halogen, hydroxyl, ―O―P(O)(OH)₂, ―O―P(O)(R^{I}R^{II})₂, optionally substituted C₁-C₆ alkyl or optionally substituted C₁-C₆ alkyloxy,
      wherein optionally substituted means substituted with 1-4 substituents each independently selected from the group consisting of hydroxyl, ―O―P(O)(OH)₂, ―O-P(O)(R^{I}R^{II})₂, C₁-C₄ alkoxyl, -N(R^{A})₂, -CO₂(R^{B}), optionally substituted phenyl, and optionally substituted 5-6 membered heterocycloalkyl, wherein said optionally substituted phenyl, or optionally substituted 5-6 membered heterocycloalkyl is optionally substituted by 1-4 substituents each independently selected from halogen, hydroxy, ―O―P(O)(OH)₂, ―O― P(O)(R^{I}R^{II})₂, amino, (C₁-C₆ alkyl)amino-, (C₁-C₆ alkyl)(C₁-C₆ alkyl)amino―, halo(C₁-C₆ alkyl), hydroxy-(C₁-C₄ alkyl)―, ―(C₁-C₄ alkyl)-O-P(O)(OH)₂, -(C₁-C₄alkyl)-O-P(O)(R^{I}R^{II})₂, halo(C₁-C₄ alkoxy)―, C₁-C₄ alkoxy―, hydroxy―(C₂-C₄ alkoxy)―, ―(C₂-C₄ alkoxy)―O―P(O)(OH)₂, ―(C₂-C₄ alkoxy)-O-P(O)(R^{I}R^{II})₂, -(C₁-C₆ alkyl)-NH₂, -C1-C4 alkyl―(C₁-C₄ alkoxyl) and C₁-C₄ alkoxy-(C₁-C₄ alkoxy)―, wherein R^{A} and R^{B} are each independently selected from hydrogen, ―C₁-C₄ alkyl, ―CO(C₁-C₄ alkyl), ―OCO(C₁-C₄ alkyl), ―(C₁-C₄ alkyl)-NH2, ―(C₁-C₄ alkyl)-C₁-C₄ alkoxyl, or ―CO₂(C₁-C₄ alkyl),
   R² and R⁷ are each independently hydrogen, ―CON(R^{C})₂, ―COOH, or CO₂(R^{D}), or one of R² and R⁷ is ―CON(R^{C})(R^{D}) and the other is H, ―COOH, or CO₂(R^{E}), wherein R^{C} and R^{D} are each independently selected from hydrogen, ―C₁-C₄ alkyl, ―CO(C₁-C₄ alkyl), ― OCO(C₁-C₄ alkyl), ―(C₁-C₄ alkyl)-NH₂, ―(C₁-C₄ alkyl)-C₁-C₄ alkoxyl, or ―CO₂(C₁-C₄ alkyl);
   R³ and R⁷ are each independently H, halo(C₁-C₆alkyl), halo(C₁-C₆alkoxy)-, hydroxy, -O-P(O)(OH)₂, -O-P(O)(R^{I}R^{II})₂, -NR^{c}R^{d}, -COR^{c}, -CO₂R^{c}, -N(R^{d})COR^{c}, -N(R^{d})S O₂R^{c}, -N(R^{g})SO₂(C₁-C₂alkyl)-N(R^{h})(R^{f}), -N(R^{g})CO(C₁-C₂alkyl)-N(R^{h})(R^{f});
   R^{e}, R^{f}, R^{g}, and R^{h} are each independently H or C₁-C₄ alkyl;
   R⁴, R⁵, R¹¹ and R¹² are each independently H or C₁-C₄ alkyl;
   R⁶ and R¹⁰ are each C₁-C₄ alkyl; and
   each occurrence of R^{I} and R^{II} is independently C₁-C₆ alkyloxy-; and
(ii) aluminium hydroxide, aluminium phosphate, aluminium oxyhydroxide, or aluminium hydroxyphosphate.

In a second aspect, the present invention provides an immunogenic composition comprising the adjuvant composition of the invention and an antigen.

In a third aspect, the present invention provides the adjuvant composition of the invention for use in therapy.

In a further aspect, the present invention provides the adjuvant composition of the invention for use in a method of immunizing a subject comprising administering to the subject the adjuvant composition of the present invention and an antigen.

In a fourth aspect, the present invention provides a method of immunising a host comprising administering to the host an adjuvant composition as defined herein and an antigen.

In a further aspect, the present invention provides a method of adjuvanting an immune response in a subject, said method comprising administering to the subject the adjuvant composition of the invention and an antigen.

In a further aspect, the present invention provides the use of the adjuvant composition in the manufacture of a medicament for adjuvanting an immune response in a subject.

In a further aspect, the present invention provides a kit comprising i) a first container comprising the adjuvant composition of the invention, and ii) a second container comprising an antigen.

### BRIEF DESCRIPTION OF THE DRAWINGS

The invention is further described by reference to the accompanying drawings, which are non-limiting.

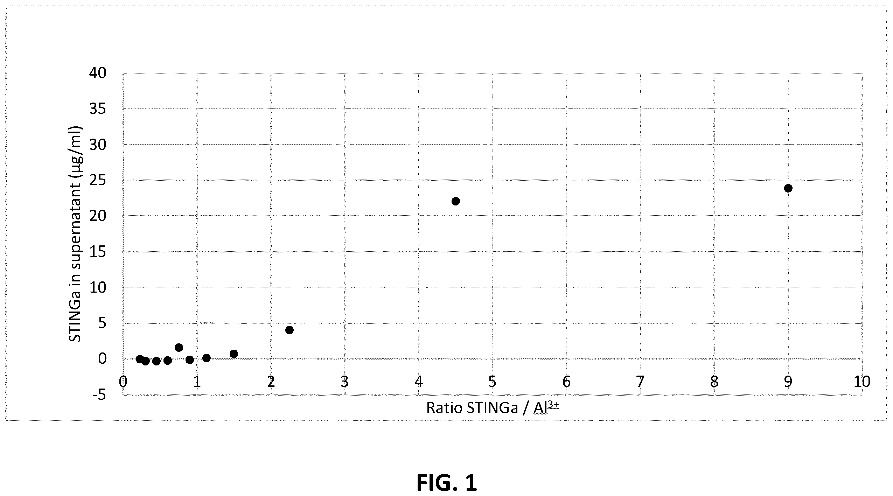

Fig. 1 represents the adsorption of a STING agonist on Al(OH)₃ (by reference to Al³⁺) at different ratios of STING agonist to Al(OH)₃ tested (as indicated).

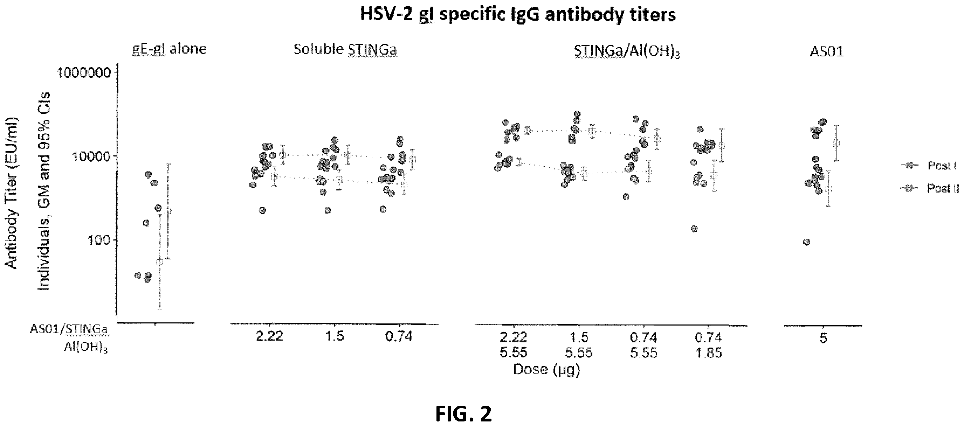

Fig. 2 represents the HSV-2 gI specific IgG antibody response induced by different vaccine formulations including the antigen HSV-2 gE-gI adjuvanted either with different doses of a soluble STING agonist or different doses of STING agonist and Al(OH)₃ at different ratios (as indicated) in mice. IgG titers were measured 2 weeks post-immunization I (Post I) and 2 weeks post-immunization II (Post II). The antigen HSV-2 gE-gI alone was used as a control. A vaccine formulation including HSV-2 gE-gI adjuvanted with AS01 was used as a comparator. Individual geometric means and 95% Confidence Intervals (Cis) are provided.

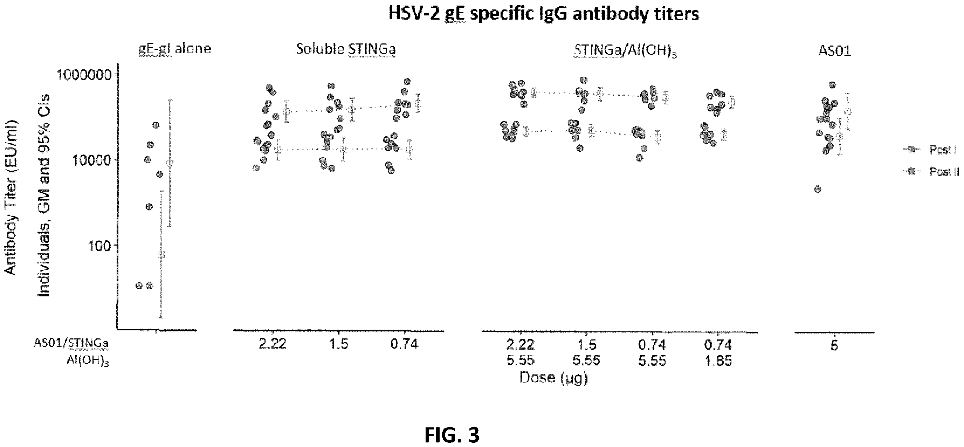

Fig. 3 represents the HSV-2 gE specific IgG antibody response induced by different vaccine formulations including the antigen HSV-2 gE-gI adjuvanted either with different doses of a soluble STING agonist or different doses of STING agonist to Al(OH)₃ at different ratios (as indicated) in mice. IgG titers were measured 2 weeks post-immunization I (Post I) and 2 weeks post-immunization II (Post II). The antigen HSV-2 gE-gI alone was used as a control. A vaccine formulation including HSV-2 gE-gI adjuvanted with AS01 was used as a comparator. Individual geometric means and 95% CIs are provided.

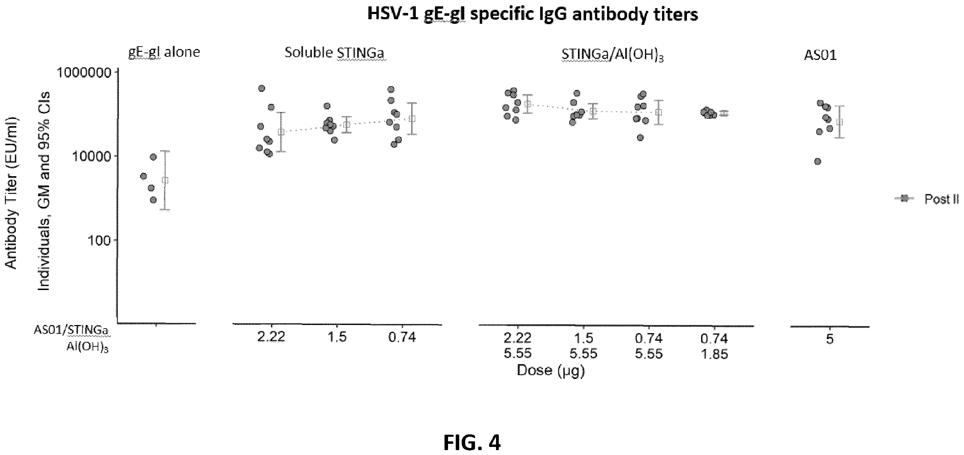

Fig. 4 represents the HSV-1 gE-gI specific IgG antibody response induced by different vaccine formulations including the antigen HSV-2 gE-gI adjuvanted either with different doses of a soluble STING agonist or different doses of STING agonist to Al(OH)₃ at different ratios (as indicated) in mice. IgG titers were measured 2 weeks post-immunization II (Post II). The antigen HSV-2 gE-gI alone was used as a control. A vaccine formulation including HSV-2 gE-gI adjuvanted with AS01 was used as a comparator. Individual geometric means and 95% CIs are provided.

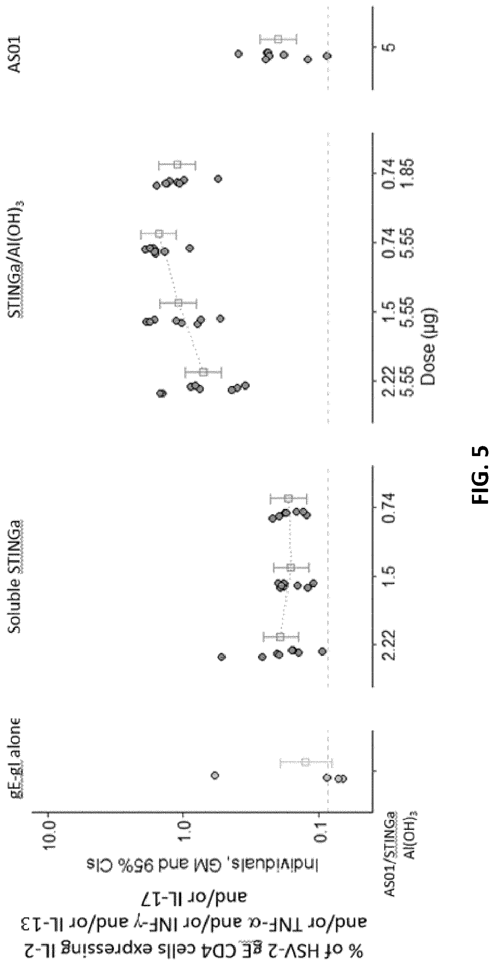

Fig. 5 represents the frequencies of vaccine-specific CD4+ T cells expressing IL-2 and/or INF-g and/or TNF-a and/ or IL-13 and/or IL-17 from splenocytes collected 2 weeks post-immunization II after *ex vivo* stimulation with HSV-2 gE peptide pools in mice. The vaccines administered included the antigen HSV-2 gE-gI adjuvanted either with different doses of a soluble STING agonist or different doses of STING agonist to Al(OH)₃ at different ratios (as indicated). The antigen HSV-2 gE-gI alone was used as a control. A vaccine formulation including HSV-2 gE-gI adjuvanted with AS01 was used as a comparator. Individual geometric means and 95% CIs are provided.

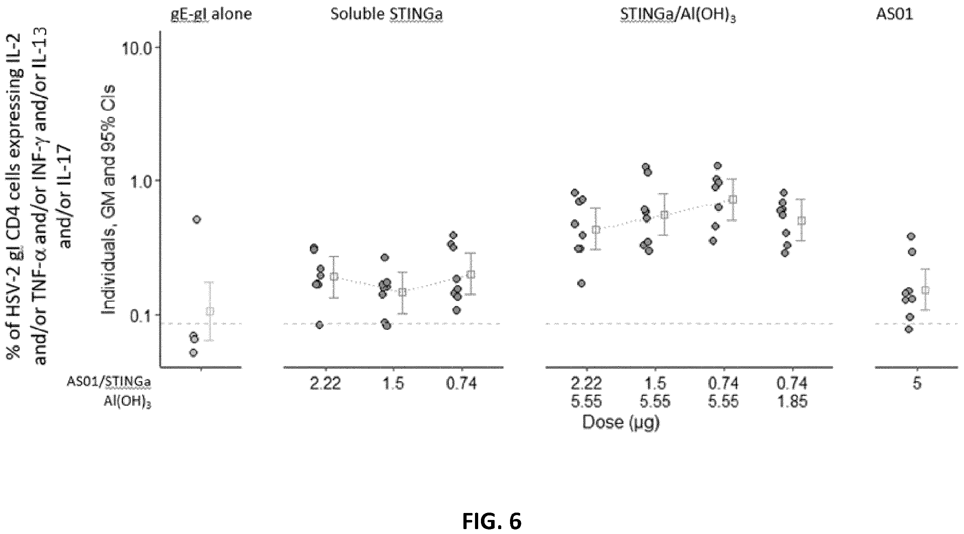

Fig. 6 represents the frequencies of vaccine-specific CD4+ T cells expressing IL-2 and/or INF-g and/or TNF-a and/ or IL-13 and/or IL-17 from splenocytes collected 2 weeks post-immunization II after *ex vivo* stimulation with HSV-2 gI peptides pool or with HSV-1 gE or gI pools in mice. The vaccines administered included the antigen HSV-2 gE-gI adjuvanted either with different doses of a soluble STING agonist or different doses of STING agonist to Al(OH)₃ at different ratios (as indicated). The antigen HSV-2 gE-gI alone was used as a control. A vaccine formulation including HSV-2 gE-gI adjuvanted with AS01 was used as a comparator. Individual geometric means and 95% CIs are provided.

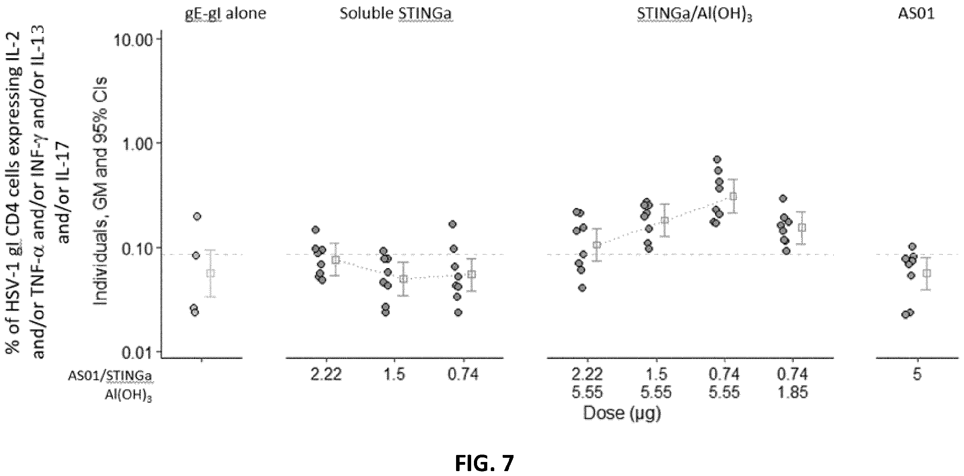

Fig. 7 represents the frequencies of vaccine-specific CD4+ T cells expressing IL-2 and/or INF-g and/or TNF-a and/ or IL-13 and/or IL-17 from splenocytes collected 2 weeks post-immunization II after *ex vivo* stimulation with HSV-1 gI peptide pools in mice. The vaccines administered included the antigen HSV-2 gE-gI adjuvanted either with different doses of a soluble STING agonist or different doses of STING agonist to Al(OH)₃ at different ratios (as indicated). The antigen HSV-2 gE-gI alone was used as a control. A vaccine formulation including HSV-2 gE-gI adjuvanted with AS01 was used as a comparator. Individual geometric means and 95% CIs are provided.

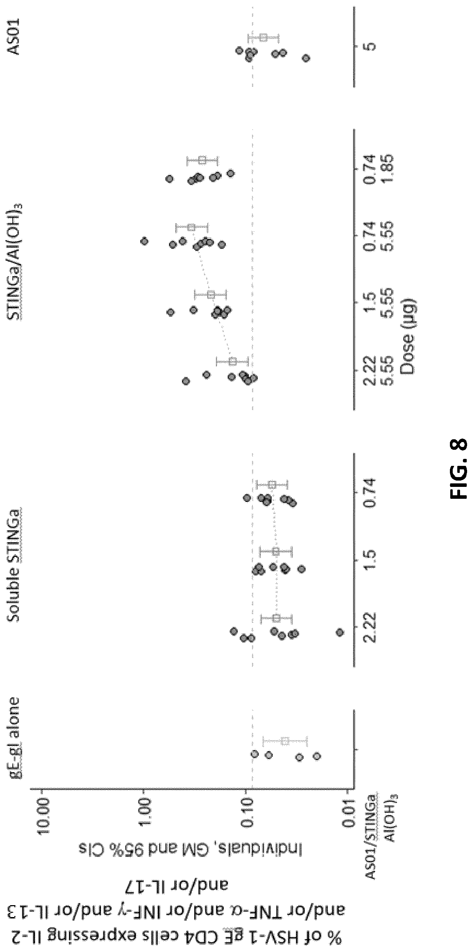

Fig. 8 represents the frequencies of vaccine-specific CD4+ T cells expressing IL-2 and/or INF-g and/or TNF-a and/ or IL-13 and/or IL-17 from splenocytes collected 2 weeks post-immunization II after *ex vivo* stimulation with HSV-1 gE peptide pools in mice. The vaccines administered included the antigen HSV-2 gE-gI adjuvanted either with different doses of a soluble STING agonist or different doses of STING agonist to Al(OH)₃ at different ratios (as indicated). The antigen HSV-2 gE-gI alone was used as a control. A vaccine formulation including HSV-2 gE-gI adjuvanted with AS01 was used as a comparator. Individual geometric means and 95% CIs are provided.

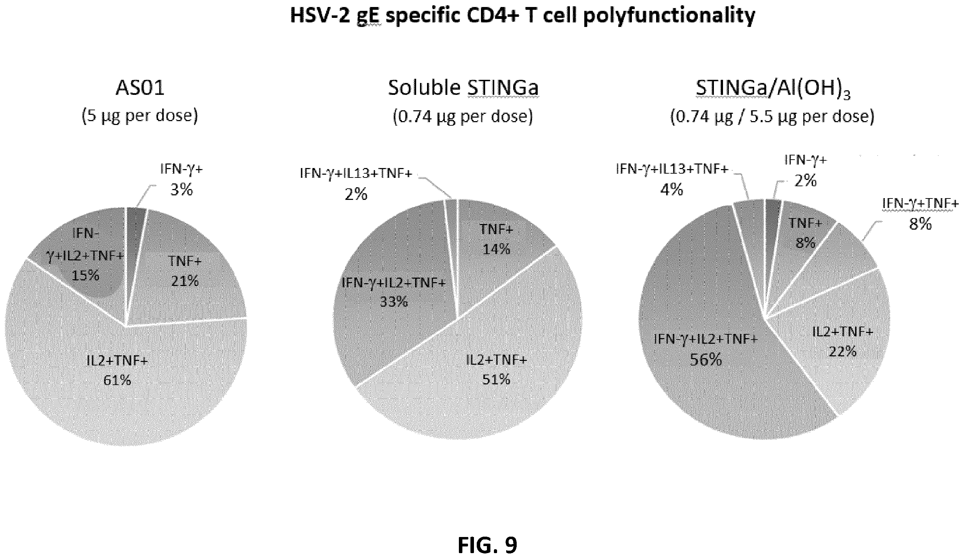

Fig. 9 represents the HSV-2 gE specific CD4+ T cell polyfunctionality analysis after *in vitro* stimulation. Proportions of HSV-2 gE specific CD4+ T cells expressing 1, 2, 3 or 4 cytokines are represented. Pie charts represent the mean proportions of cells expressing single markers and any combination of INF-g, IL-2, TNF-a, IL-13 and IL-17 marker-positive CD4+ T cells out of the total HSV-2 specific CD4+ T cells. The vaccines used in this analysis were as follows (as indicated): HSV-2 gE-gI antigen adjuvanted with either (i) AS01, (ii) 0.74 µg of soluble STING agonist, or (iii) 0.74 µg STING agonist and 5.55 µg AL(OH)₃.

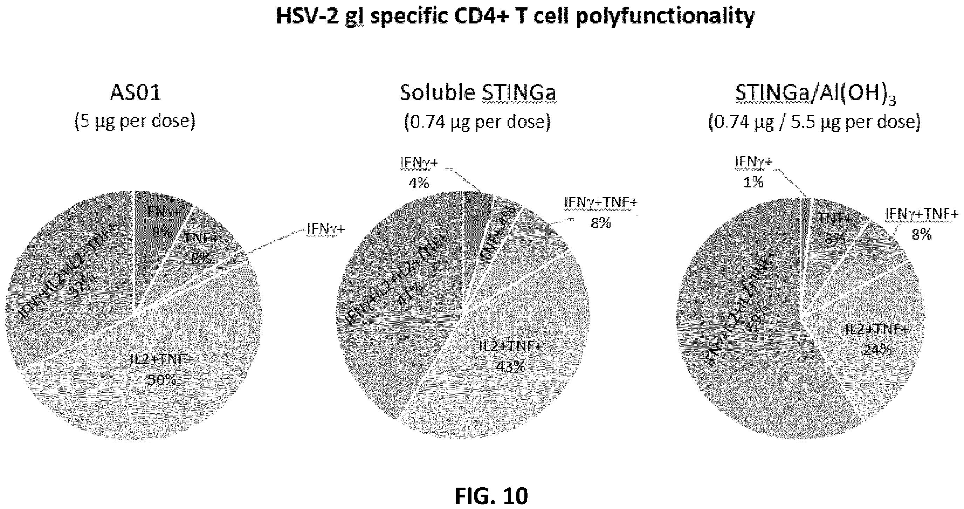

Fig. 10 represents the HSV-2 gI specific CD4+ T cell polyfunctionality analysis after *in vitro* stimulation. Proportions of HSV-2 gI specific CD4+ T cells expressing 1, 2, 3 or 4 cytokines are represented. Pie charts represent the mean proportions of cells expressing single markers and any combination of INF-g, IL-2, TNF-a, IL-13 and IL-17 marker-positive CD4+ T cells out of the total HSV-2 specific CD4+ T cells. The vaccines used in this analysis were as follows (as indicated): HSV-2 gE-gI antigen adjuvanted with either (i) AS01, (ii) 0.74 µg of soluble STING agonist, or (iii) 0.74 µg STING agonist and 5.55 µg AL(OH)₃.

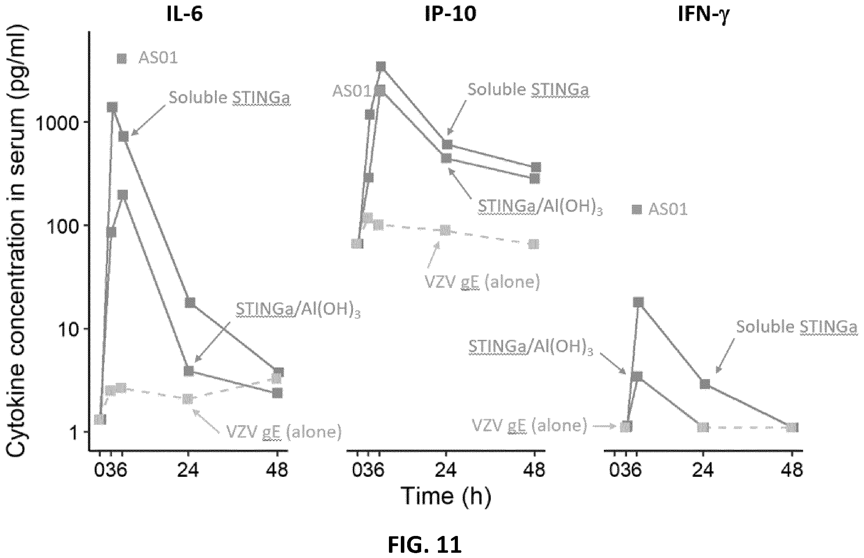

Fig. 11 represents an analysis of cytokines (IL-6, IP10 and IFN-γ, as indicated) measurement in the serum of mice, 3h, 6h, 24h and 48 h post-immunization I with either the VZV gE antigen alone, VZV gE adjuvanted with soluble STING agonist or adjuvanted with STING agonist and Al(OH)₃. A vaccine formulation including VZV gE adjuvanted with AS01 was used as a comparator.

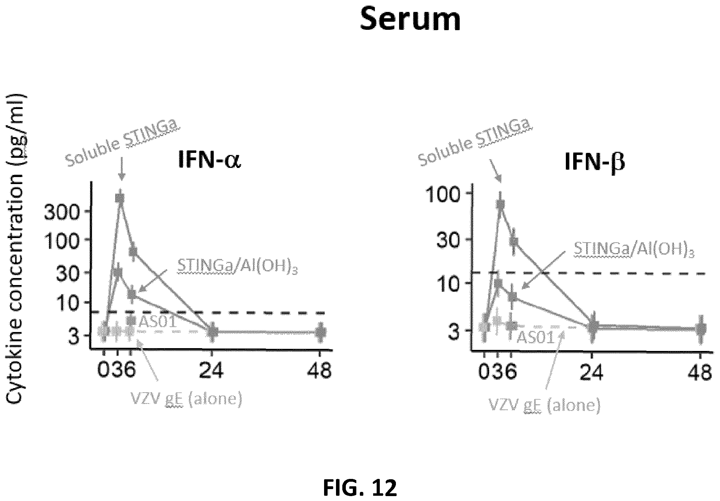

Fig. 12 represents an analysis of cytokines (IFN-a, IFN-b and IFN-γ, as indicated) measurement in the serum of mice, 3h, 6h, 24h and 48 h post-immunization I with either the VZV gE antigen alone, VZV gE adjuvanted with soluble STING agonist or adjuvanted with STING agonist and Al(OH)₃. A vaccine formulation including VZV gE adjuvanted with AS01 was used as a comparator.

### BRIEF DESCRIPTION OF THE SEQUENCES

SEQ ID NO. 1: SARS-CoV-2 S protein.
SEQ ID NO. 2: SARS-CoV-2 S protein ectodomain.
SEQ ID NO. 3: SARS-CoV-2 S protein receptor binding domain.
SEQ ID NO. 4: Pre-fusion stabilised SARS-CoV-2 S protein ectodomain.
SEQ ID NO. 5: Polypeptide sequence of HSV-2 gE P317R.
SEQ ID NO. 6: Polypeptide sequence of HSV-2 gI.
SEQ ID NO. 7: Polypeptide sequence for VZV gE.

### DETAILED DESCRIPTION OF THE EMBODIMENTS

### STATEMENT OF THE INVENTION

As described above, in one aspect of the invention, there is provided an adjuvant composition comprising:
(i) a STING agonist of Formula (I) or a pharmaceutically acceptable salt thereof 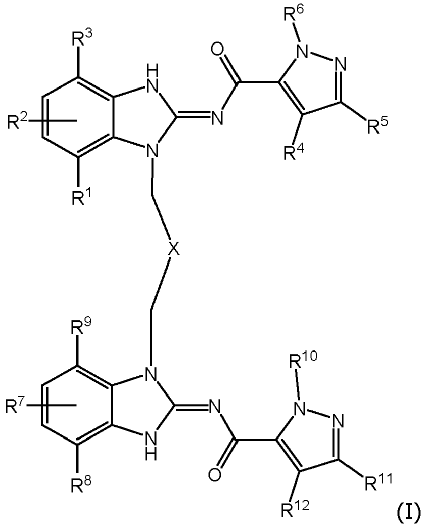 wherein
   X is ―halo(C₁-C₅)alkyl, unsubstituted ―C₁-C₅ alkyl, or unsubstituted ―C₂-C₅ alkenyl;
   R¹ and R⁹ are independently H, halogen, hydroxyl, ―O―P(O)(OH)₂, ―O―P(O)(R^{I}R^{II})₂, optionally substituted C₁-C₆ alkyl or optionally substituted C₁-C₆ alkyloxy,
      wherein optionally substituted means substituted with 1-4 substituents each independently selected from the group consisting of hydroxyl, ―O―P(O)(OH)₂, ―O-P(O)(R^{I}R^{II})₂, C₁-C₄ alkoxyl, -N(R^{A})₂, -CO₂(R^{B}), optionally substituted phenyl, and optionally substituted 5-6 membered heterocycloalkyl, wherein said optionally substituted phenyl, or optionally substituted 5-6 membered heterocycloalkyl is optionally substituted by 1-4 substituents each independently selected from halogen, hydroxy, ―O―P(O)(OH)₂, ―O― P(O)(R^{I}R^{II})₂, amino, (C₁-C₆ alkyl)amino-, (C₁-C₆ alkyl)(C₁-C₆ alkyl)amino―, halo(C₁-C₆ alkyl), hydroxy-(C₁-C₄ alkyl)―, ―(C₁-C₄ alkyl)-O-P(O)(OH)₂, -(C₁-C₄alkyl)-O-P(O)(R^{I}R^{II})₂, halo(C₁-C₄ alkoxy)―, C₁-C₄ alkoxy―, hydroxy―(C₂-C₄ alkoxy)―, ―(C₂-C₄ alkoxy)―O―P(O)(OH)₂, ―(C₂-C₄ alkoxy)-O-P(O)(R^{I}R^{II})₂, -(C₁-C₆ alkyl)-NH₂, -C₁-C₄ alkyl―(C₁-C₄ alkoxyl) and C₁-C₄ alkoxy-(C₁-C₄ alkoxy)―, wherein R^{A} and R^{B} are each independently selected from hydrogen, ―C₁-C₄ alkyl, ―CO(C₁-C₄ alkyl), ―OCO(C₁-C₄ alkyl), ―(C₁-C₄ alkyl)-NH2, ―(C₁-C₄ alkyl)-C₁-C₄ alkoxyl, or ―CO₂(C₁-C₄ alkyl);
   R² and R⁷ are each independently hydrogen, ―CON(R^{C})₂, ―COOH, or CO₂(R^{D}), or one of R² and R⁷ is ―CON(R^{C})(R^{D}) and the other is H, ―COOH, or CO₂(R^{E}), wherein R^{C} and R^{D} are each independently selected from hydrogen, ―C₁-C₄ alkyl, ―CO(C₁-C₄ alkyl), ― OCO(C₁-C₄ alkyl), ―(C₁-C₄ alkyl)-NH2, ―(C₁-C₄ alkyl)-C₁-C₄ alkoxyl, or ―CO₂(C₁-C₄ alkyl); R³ and R⁸ are each independently H, halo(C₁-C₆alkyl), halo(C₁-C₆alkoxy)-, hydroxy, -O-P(O)(OH)₂, -O-P(O)(R^{I}R^{II})₂, -NR^{c}R^{d}, -COR^{c}, -CO₂R^{c}, -N(R^{d})COR^{c}, -N(R^{d})S O₂R^{c}, -N(R^{g})SO₂(C₁-C₂alkyl)-N(R^{h})(R^{f}), -N(R^{g})CO(C₁-C₂alkyl)-N(R^{h})(R^{f});
   R^{e}, R^{f}, R^{g}, and R^{h} are each independently H or C₁-C₄ alkyl;
   R⁴, R⁵, R¹¹ and R¹² are each independently H or C₁-C₄ alkyl;
   R⁶ and R¹⁰ are each C₁-C₄ alkyl; and
   each occurrence of R^{I} and R^{II} is independently C₁-C₆ alkyloxy―; and
(ii) aluminium hydroxide, aluminium phosphate, aluminium oxyhydroxide, or aluminium hydroxyphosphate.

### DEFINITIONS

As used herein, the term "STING agonist" (also referred to as "STINGa" herein) refers to a compound of Formula (I) that is capable of binding to and activating the STING receptor and STING signaling. For example, the STING agonist, upon contact with the STING receptor causes one or more of the following: (1) stimulates or activates the STING receptor, (2) upregulates IRF3 and NFkB signaling pathways and/or (3) induces IFN-b and other cytokines. STING agonist activity can be measured *in vitro* by various assays known in the art such as, but not limited to, measurement of cell signaling, cell proliferation, immune cell activation markers, cytokine production. STING agonist activity can also be measured *in vivo* by various assays that measure surrogate end points such as, but not limited to the measurement of T-cell proliferation or innate immunity-related cytokine production, in particular type I interferon.

The alternative definitions for the various groups and substituent groups of Formula (I) provided throughout the specification are intended to particularly describe each compound species disclosed herein, individually, as well as groups of one or more compound species. The scope of the STING agonists in accordance with the invention includes any combination of these groups and substituent group definitions.

It will be appreciated by those skilled in the art that said compounds may exist in other tautomeric forms including zwitterionic forms, or isomeric forms. All tautomeric (including zwitterionic forms) and isomeric forms of Formula (I) and compounds described herein are intended to be encompassed within the scope of the present invention.

For example, it will be appreciated by those skilled in the art that the compounds for use in this invention may exist in tautomeric forms including, but not limited to, Formula (A), Formula (B) and/or Formula (C) or zwitterionic forms including, but not limited to, Formula (D) or Formula (E).

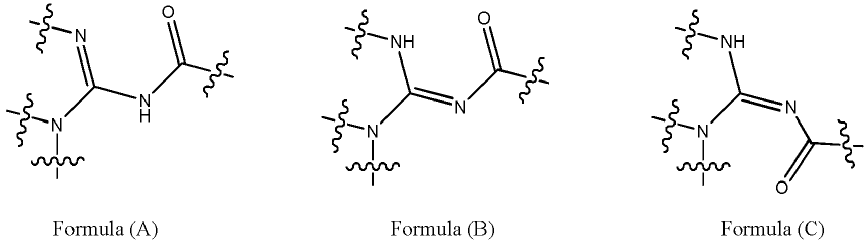

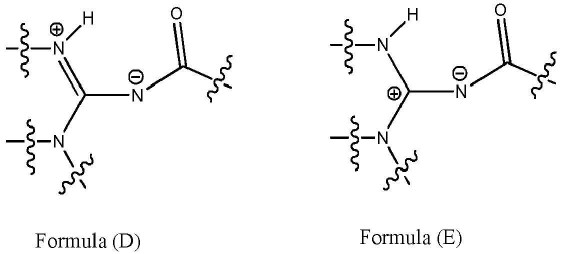

The chemical names provided for the intermediate compounds and/or the compounds for use in this invention described herein may refer to any one of the tautomeric representations of such compounds (in some instances, such alternate names are provided with the experimental). It is to be understood that any reference to a named compound (an intermediate compound or a compound of the invention) or a structurally depicted compound (an intermediate compound or a compound of the invention) is intended to encompass all tautomeric forms including zwitterionic forms of such compounds and any mixture thereof.

As used herein, the term "alkyl" represents a saturated, straight or branched hydrocarbon group having the specified number of carbon atoms. The term "C₁-C₄alkyl" refers to a straight or branched alkyl moiety containing from 1 to 4 carbon atoms. Exemplary alkyls include, but are not limited to methyl, ethyl, *n*-propyl, isopropyl, *n*-butyl, isobutyl, *s*-butyl, *t*-butyl, pentyl and hexyl.

When a substituent term such as "alkyl" is used in combination with another substituent term, for example as in "hydroxy(C₁-C₄alkyl)", the linking substituent term (e.g., alkyl) is intended to encompass a divalent moiety, wherein the point of attachment is through that linking substituent. Examples of "hydroxy(C₁-C₄alkyl)" groups include, but are not limited to, hydroxymethyl, hydroxyethyl, and hydroxyisopropyl.

As used herein, the term "halo(alkyl)" represents a saturated, straight or branched hydrocarbon group having the specified number (n) of carbon atoms and one or more (up to 2n+1) halogen atoms. For example, the term "halo(C₁-C₅alkyl)" represents a group having one or more halogen atoms, which may be the same or different, at one or more carbon atoms of an alkyl moiety containing from 1 to 5 carbon atoms. Examples of "halo(C₁-C₅alkyl)" groups include, but are not limited to, -CF₃ (trifluoromethyl), -CCl₃ (trichloromethyl), 1,1-difluoroethyl, 2,2,2-trifluoroethyl, and hexafluoroisopropyl.

"Alkenyl" refers to straight or branched hydrocarbon group having the specified number of carbon atoms and at least 1 and up to 3 carbon-carbon double bonds. Examples include ethenyl and propenyl.

"Alkoxy-" or "(alkyl)oxy-" refers to an "alkyl-oxy-" group, containing an alkyl moiety, having the specified number of carbon atoms, attached through an oxygen linking atom. For example, the term "C₁-C₄alkoxy-" represents a saturated, straight or branched hydrocarbon moiety having at least 1 and up to 4 carbon atoms attached through an oxygen linking atom. Exemplary "C₁-C₄alkoxy-" or "(C₁-C₄alkyl)oxy-" groups include, but are not limited to, methoxy, ethoxy, n-propoxy, isopropoxy, n-butoxy, s-butoxy, and t-butoxy. Exemplary "C₁-C₆alkoxy-" or "C₁-C₆alkyloxy-" groups include, but are not limited to, methoxy, ethoxy, n-propoxy, isopropoxy, n-butoxy, s-butoxy, and t-butoxy.

As used herein, the term "halo(alkoxy)-" represents a saturated, straight or branched hydrocarbon group having the specified number (n) of carbon atoms and one or more (up to 2n+1) halogen atoms, attached through an oxygen linking atom. For example, the term "halo(C₁-C₄alkoxy)-" refers to a "haloalkyl-oxy-" group, containing a "halo(C₁-C₄alkyl)" moiety attached through an oxygen linking atom. Exemplary "halo(C₁-C₄alkoxy)-" groups include, but are not limited to, -OCHF₂ (difluoromethoxy), -OCF₃ (trifluoromethoxy), -OCH₂CF₃ (trifluoroethoxy), and -OCH(CF₃)₂ (hexafluoroisopropoxy).

A heterocyclic group or moiety is a cyclic group or moiety having, as ring members, atoms of at least two different elements, which cyclic group or moiety may be saturated, partially unsaturated (non-aromatic) or fully unsaturated (aromatic).

"Heterocycloalkyl" refers to a non-aromatic, monocyclic or bicyclic group containing 3-10 ring atoms and containing one or more (generally one or two) heteroatom ring members independently selected from oxygen, sulfur, and nitrogen. The point of attachment of a heterocycloalkyl group may be by any suitable carbon or nitrogen atom.

The term "5-6 membered heterocycloalkyl" represents a saturated, monocyclic group, containing 5 or 6 ring atoms, which includes one or two heteroatoms selected independently from oxygen, sulfur, and nitrogen. Illustrative examples of 5-6 membered heterocycloalkyl groups include, but are not limited to pyrrolidinyl, tetrahydrofuranyl, tetrahydrothienyl, tetrahydropyranyl, tetrahydrothiopyranyl, piperidinyl, piperazinyl, morpholinyl, and thiomorpholinyl.

The terms "halogen" and "halo" refers to a halogen radical, for example, a fluoro, chloro, bromo, or iodo substituent.

"Oxo" represents a double-bonded oxygen moiety; for example, if attached directly to a carbon atom forms a carbonyl moiety (C = O).

"Hydroxy" or "hydroxyl" is intended to mean the radical -OH.

As used herein, the term "cyano" refers to a nitrile group, -C=N.

As used herein, the term "optionally substituted" indicates that a group (such as an alkyl, cycloalkyl, alkoxy, heterocycloalkyl, aryl, or heteroaryl group) or ring or moiety may be unsubstituted, or the group, ring or moiety may be substituted with one or more substituent(s) as defined in the substituent definitions (A, R³, etc,) provided herein. In the case where groups may be selected from a number of alternative groups, the selected groups may be the same or different.

The term "independently" means that where more than one substituent is selected from a number of possible substituents, those substituents may be the same or different.

The term "pharmaceutically acceptable" refers to those compounds, materials, compositions, and dosage forms which are, within the scope of sound medical judgment, suitable for use in contact with the tissues of human beings and animals without excessive toxicity, irritation, or other problem or complication, commensurate with a reasonable benefit/risk ratio.
As used herein, the term "vaccine" is optionally substitutable with the term "immunogenic composition".

As used herein, the term "reactogenicity" refers to a subset of adverse events that is associated with the inflammatory response to the vaccination. The adverse events can be divided into both local (e.g. pain, swelling, erythma and induration) and systemic (e.g. fever, nausea/vomiting, diarrhoea, headaches, fatigue and myalgia). Improving vaccines by reducing their reactogenicity may improve ease of access of vaccines to specific populations, for example by reducing pain in adolescents and fever in infants. Accordingly, reduced reactogenicity may improve vaccine uptake leading to greater population coverage and therefore reducing morbidity/mortality. Moreover, an excessive inflammation may also possibly negatively affect the quality of the immune response induced by a vaccine or an immunogenic composition. It is therefore an object of the invention to reduce the reactogenicity of vaccines.

Identity or homology with respect to a sequence is defined herein as the percentage of amino acid residues in the candidate sequence that are identical with the reference amino acid sequence after aligning the sequences and introducing gaps, if necessary, to achieve the maximum percent sequence identity, and not considering any conservative substitutions as part of the sequence identity.

Sequence identity can be determined by standard methods that are commonly used to compare the similarity in position of the amino acids of two polypeptides. Using a computer program such as BLAST or FASTA, two polypeptides are aligned for optimal matching of their respective amino acids (either along the full length of one or both sequences or along a pre-determined portion of one or both sequences). The programs provide a default opening penalty and a default gap penalty, and a scoring matrix such as PAM 250 [a standard scoring matrix; see Dayhoff et al., in Atlas of Protein Sequence and Structure, vol. 5, supp. 3 (1978)] can be used in conjunction with the computer program. For example, the percent identity can then be calculated as: the total number of identical matches multiplied by 100 and then divided by the sum of the length of the longer sequence within the matched span and the number of gaps introduced into the shorter sequences in order to align the two sequences.

### STING agonist

In one embodiment, the STING agonist is of Formula (II) or a pharmaceutically acceptable salt thereof 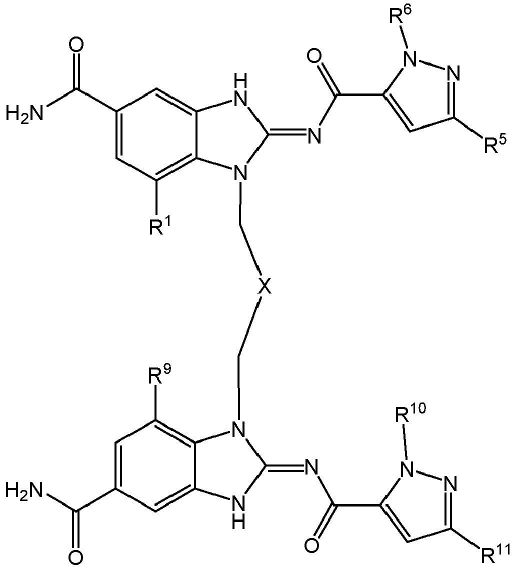 wherein X, R¹, R⁵, R⁶, R⁹, R¹⁰ and R¹¹ are as defined in relation to Formula (I).

In one embodiment, R¹ and R⁹ in Formula (I) or (II) are each independently H, halogen, optionally substituted (C₁-C₆alkyl), or optionally substituted (C₁-C₆alkyl)oxy-, and the C₁-C₆alkyl of said optionally substituted (C₁-C₆alkyl), optionally substituted (C₁-C₆alkyl)oxy- is optionally substituted with 1-4 substituents each independently selected from the group consisting of hydroxyl,―O-P(O)(OH)₂, ― O-P(O)(R^{I}R^{II})₂, -N(R^{e})(R^{f}), C₁-C₄alkoxyl, phenyl, optionally substituted 5-6 membered heterocycloalkyl containing at least one nitrogen or oxygen as a member of the ring, each R^{e} is independently selected from H, (C₁-C₄alkyl), -(C₁C₄alkyl)-NH₂, or -(C₁C₄alkyl) C₁-C₄alkoxy and each R^{f} is independently H or (C₁-C₄alkyl).

In one embodiment, the compound of Formula (I) or (II) includes at least one phosphate group. In one embodiment, one of R¹ and R⁹ in Formula (I) or (II) is ―O―P(O)(OH)₂, ―O―P(O)(R^{I}R^{II})₂, C₁-C₆ alkyl substituted with ―O―

P(O)(OH)₂ or ―O―P(O)(R^{I}R^{II})₂, or a C₁-C₆ alkyloxy group. In one embodiment, one of R¹ and R⁹ in Formula (I) or (II) is ―O―P(O)(OH)₂, ―O―P(O)(R^{I}R^{II})₂, C₁-C₆ alkyl substituted with ―O―P(O)(OH)₂ or ―O―P(O)(R^{I}R^{II})₂. In one embodiment, one of ―O―P(O)(OH)₂, ―O―P(O)(R^{I}R^{II})₂, C₁-C₆ alkyl substituted with ―O―P(O)(OH)₂ or ―O―P(O)(R^{I}R^{II})₂, and the other is H, halogen, hydroxyl, ―O― P(O)(OH)₂, ―O―P(O)(R^{I}R^{II})₂, optionally substituted C₁-C₆ alkyl or optionally substituted C₁-C₆ alkyloxy, wherein optionally substituted means substituted with 1-4 substituents each independently selected from the group consisting of hydroxyl, ―O―P(O)(OH)₂, ―O-P(O)(R^{I}R^{II})₂, C₁-C₄ alkoxyl, -N(R^{A})₂, - CO2(R^{b}). In one embodiment, one of R¹ and R⁹ in Formula (I) or (II) is ―O―P(O)(OH)₂, ―O―P(O)(R^{I}R^{II})₂, C₁-C₆ alkyl substituted with ―O―P(O)(OH)₂ or ―O―P(O)(R^{I}R^{II})₂, and the other is H, halogen, hydroxyl, ―O―P(O)(OH)₂, ―O―P(O)(R^{I}R^{II})₂, optionally substituted C₁-C₆ alkyl or optionally substituted C₁-C₆ alkyloxy, wherein optionally substituted means substituted with 1-4 substituents each independently selected from the group consisting of hydroxyl, ―O―P(O)(OH)2, ―O-P(O)(R^{I}R^{II})₂, -N(R^{A})₂, -CO₂(R^{B}). In one embodiment, one of R¹ and R⁹ in Formula (I) or (II) is ―O―P(O)(OH)₂, ―O―P(O)(R^{I}R^{II})₂, C₁-C₆ alkyl substituted with ―O―P(O)(OH)₂ or ―O―P(O)(R^{I}R^{II})₂, and the other is H, halogen, hydroxyl, ―O― P(O)(OH)₂, ―O―P(O)(R^{I}R^{II})₂, C₁-C₆ alkyl or C₁-C₆ alkyloxy.

In one embodiment, R³ in Formula (I) or (II) is H. In one embodiment, R⁸ in Formula (I) or (II) is H. In one embodiment, R³ and R⁸ in Formula (I) or (II) are H.

In one embodiment, R⁴ in Formula (I) or (II) is H. In one embodiment, R¹³ in Formula (I) or (II) is H. In one embodiment, R⁴ and R¹³ in Formula (I) or (II) are H.

In one embodiment, R⁶ in Formula (I) or (II) is ethyl. In one embodiment, R¹⁰ in Formula (I) or (II) is ethyl. In one embodiment, R⁶ and R¹⁰ in Formula (I) or (II) are ethyl.

In one embodiment, at least one of R¹ and R⁹ in Formula (I) and (II) is selected from the following groups: 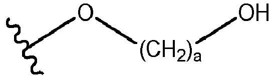 where a is a number from 1 to 6; 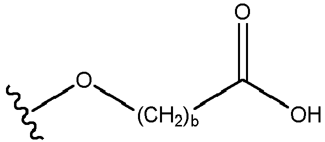 where b is a number from 1 to 6; 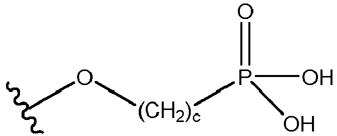 where c is a number from 1 to 6; 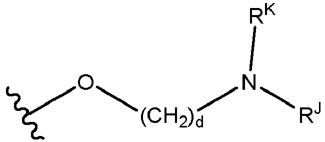 where d is a number from 1 to 6, and R^{J} and R^{K} are C1-C3 alkyl; or 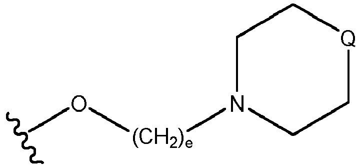 where e is a number from 1 to 6, and Q is selected from O and N(R^{X}) wherein R^{X} is a C₁-C₆ alkyl or C₁-C₆ alkoxy group.

In one embodiment, the STING agonist is selected from the group consisting of
(E)-1-(4-(5-carbamoyl-2-(1-ethyl-3-methyl-1H-pyrazole-5-carboxamido)-1H-benzo[d]imidazol-1-yl)but-2-en-1-yl)-2-(1-ethyl-3-methyl-1H-pyrazole-5-carboxamido)-7-(3-hydroxypropoxy)-1H-benzo[d]imidazole-5-carboxamide;
(E)-1-((E)-4-((E)-5-carbamoyl-2-((1-ethyl-3-methyl-1H-pyrazole-5-carbonyl)imino)-2,3-dihydro-1H-benzo[d]imidazol-1-yl)but-2-en-1-yl)-2-((1-ethyl-3-methyl-1H-pyrazole-5-carbonyl)imino)-7-(3-hydroxypropoxy)-2,3-dihydro-1H-benzo[d]imidazole-5-carboxamide;
(Z)-1-((E)-4-((Z)-5-carbamoyl-2-((1-ethyl-3-methyl-1H-pyrazole-5-carbonyl)imino)-2,3-dihydro-1H-benzo[d]imidazol-1-yl)but-2-en-1-yl)-2-((1-ethyl-3-methyl-1H-pyrazole-5-carbonyl)imino)-7-(3-hydroxypropoxy)-2,3-dihydro-1H-benzo[d]imidazole-5-carboxamide;
(E)-4-((5-carbamoyl-1-(4-(5-carbamoyl-2-(1-ethyl-3-methyl-1H-pyrazole-5-carboxamido)-1H-benzo[d]imidazol-1-yl)but-2-en-1-yl)-2-(1-ethyl-3-methyl-1H-pyrazole-5-carboxamido)-1H-benzo[d]imidazol-7-yl)oxy)butanoic acid;
(E)-1-(4-(5-carbamoyl-2-(1-ethyl-3-methyl-1H-pyrazole-5-carboxamido)-7-methoxy-1H-benzo[d]imidazol-1-yl)but-2-en-1-yl)-7-(3-(dimethylamino)propoxy)-2-(1-ethyl-3-methyl-1H-pyrazole-5-carboxamido)-1H-benzo[d]imidazole-5-carboxamide;
(E)-1-((E)-4-((E)-5-carbamoyl-2-((1-ethyl-3-methyl-1H-pyrazole-5-carbonyl)imino)-7-(3-(4-(2-hydroxyethyl)piperazin-1-yl)propoxy)-2,3-dihydro-1H-benzo[d]imidazol-1-yl)but-2-en-1-yl)-2-((1-ethyl-3-methyl-1H-pyrazole-5-carbonyl)imino)-7-methoxy-2,3-dihydro-1H-benzo[d]imidazole-5-carboxamide;
(E)-1-((E)-4-((E)-5-carbamoyl-2-((1-ethyl-3-methyl-1H-pyrazole-5-carbonyl)imino)-7-(3-hydroxypropoxy)-2,3-dihydro-1H-benzo[d]imidazol-1-yl)but-2-en-1-yl)-2-((1-ethyl-3-methyl-1H-pyrazole-5-carbonyl)imino)-7-methoxy-2,3-dihydro-1H-benzo[d]imidazole-5-carboxamide;
(E)-1-(4-(5-carbamoyl-2-(1-ethyl-3-methyl-1H-pyrazole-5-carboxamido)-1H-benzo[d]imidazol-1-yl)but-2-en-1-yl)-2-(1-ethyl-3-methyl-1H-pyrazole-5-carboxamido)-7-(3-morpholinopropoxy)-1H-benzo[d]imidazole-5-carboxamide;
(E)-1-((E)-4-((E)-5-carbamoyl-2-((1-ethyl-3-methyl-1H-pyrazole-5-carbonyl)imino)-2,3-dihydro-1H-benzo[d]imidazol-1-yl)but-2-en-1-yl)-2-((1-ethyl-3-methyl-1H-pyrazole-5-carbonyl)imino)-7-(3-morpholinopropoxy)-2,3-dihydro-1H-benzo[d]imidazole-5-carboxamide;
(Z)-1-((E)-4-((Z)-5-carbamoyl-2-((1-ethyl-3-methyl-1H-pyrazole-5-carbonyl)imino)-2,3-dihydro-1H-benzo[d]imidazol-1-yl)but-2-en-1-yl)-2-((1-ethyl-3-methyl-1H-pyrazole-5-carbonyl)imino)-7-(3-morpholinopropoxy)-2,3-dihydro-1H-benzo[d]imidazole-5-carboxamide;
(E)-1-(4-(5-carbamoyl-2-(1-ethyl-3-methyl-1H-pyrazole-5-carboxamido)-7-(3-morpholinopropoxy)-1H-benzo[d]imidazol-1-yl)but-2-en-1-yl)-2-(1-ethyl-3-methyl-1H-pyrazole-5-carboxamido)-7-methoxy-1H-benzo[d]imidazole-5-carboxamide;
(E)-1-((E)-4-((E)-5-carbamoyl-2-((1-ethyl-3-methyl-1H-pyrazole-5-carbonyl)imino)-7-(3-morpholinopropoxy)-2,3-dihydro-1H-benzo[d]imidazol-1-yl)but-2-en-1-yl)-2-((1-ethyl-3-methyl-1H-pyrazole-5-carbonyl)imino)-7-methoxy-2,3-dihydro-1H-benzo[d]imidazole-5-carboxamide;
(E)-3-((5-carbamoyl-1-(4-(5-carbamoyl-2-(1-ethyl-3-methyl-1H-pyrazole-5-carboxamido)-7-methoxy-1H-benzo[d]imidazol-1-yl)but-2-en-1-yl)-2-(1-ethyl-3-methyl-1H-pyrazole-5-carboxamido)-1H-benzo[d]imidazol-7-yl)oxy)propyl dihydrogen phosphate;
3-(((Z)-6-carbamoyl-3-((E)-4-((Z)-5-carbamoyl-2-((1-ethyl-3-methyl-1H-pyrazole-5-carbonyl)imino)-7-methoxy-2,3-dihydro-1H-benzo[d]imidazol-1-yl)but-2-en-1-yl)-2-((1-ethyl-3-methyl-1H-pyrazole-5-carbonyl)imino)-2,3-dihydro-1H-benzo[d]imidazol-4-yl)oxy)propyl dihydrogen phosphate;
3-(((E)-6-carbamoyl-3-((E)-4-((E)-5-carbamoyl-2-((1-ethyl-3-methyl-1H-pyrazole-5-carbonyl)imino)-7-methoxy-2,3-dihydro-1H-benzo[d]imidazol-1-yl)but-2-en-1-yl)-2-((1-ethyl-3-methyl-1H-pyrazole-5-carbonyl)imino)-2,3-dihydro-1H-benzo[d]imidazol-4-yl)oxy)propyl dihydrogen phosphate; or
a pharmaceutically acceptable salt thereof.

In one embodiment, the STING agonist is 4-(((E)-6-carbamoyl-3-((E)-4-((E)-5-carbamoyl-2-((1-ethyl-3-methyl-1H-pyrazole-5-carbonyl)imino)-2,3-dihydro-1H-benzo[d]imidazol-1-yl)but-2-en-1-yl)-2-((1-ethyl-3-methyl-1H-pyrazole-5-carbonyl)imino)-2,3-dihydro-1H-benzo[d]imidazol-4-yl)oxy)butanoic acid as shown below or a pharmaceutically acceptable salt thereof: 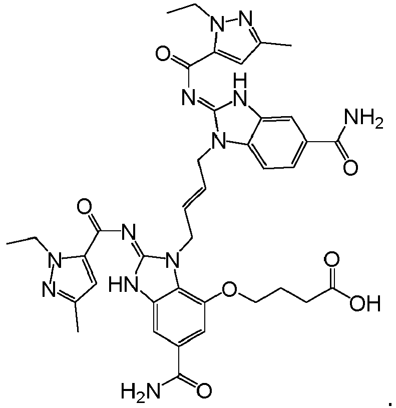 This compound may also be labelled as (E)-4-((5-carbamoyl-1-(4-(5-carbamoyl-2-(1-ethyl-3-methyl-1H-pyrazole-5-carboxamido)-1H-benzo[d]imidazol-1-yl)but-2-en-1-yl)-2-(1-ethyl-3-methyl-1H-pyrazole-5-carboxamido)-1H-benzo[d]imidazol-7-yl)oxy)butanoic acid or 4-(((Z)-6-carbamoyl-3-((E)-4-((E)-5-carbamoyl-2-((1-ethyl-3-methyl-1H-pyrazole-5-carbonyl)imino)-2,3-dihydro-1H-benzo[d]imidazol-1-yl)but-2-en-1-yl)-2-((1-ethyl-3-methyl-1H-pyrazole-5-carbonyl)imino)-2,3-dihydro-1H-benzo[d]imidazol-4-yl)oxy)butanoic acid depending on its isomeric/tautomeric form.

In one embodiment, the STING agonist is (E)-1-((E)-4-((E)-5-carbamoyl-2-((1-ethyl-3-methyl-1H-pyrazole-5-carbonyl)imino)-7-methoxy-2,3-dihydro-1H-benzo[d]imidazol-1-yl)but-2-en-1-yl)-7-(3-(dimethylamino)propoxy)-2-((1-ethyl-3-methyl-1H-pyrazole-5-carbonyl)imino)-2,3-dihydro-1H-benzo[d]imidazole-5-carboxamide as represented by the below structure: 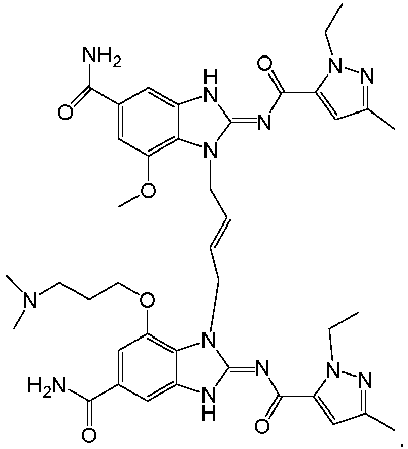 The compound may be labelled as (E)-1-(4-(5-carbamoyl-2-(1-ethyl-3-methyl-1H-pyrazole-5-carboxamido)-7-methoxy-1H-benzo[d]imidazol-1-yl)but-2-en-1-yl)-7-(3-(dimethylamino)propoxy)-2-(1-ethyl-3-methyl-1H-pyrazole-5-carboxamido)-1H-benzo[d]imidazole-5-carboxamide depending on its isomeric/tautomeric form.

In one embodiment, the STING agonist is (E)-1-((E)-4-((E)-5-carbamoyl-2-((1-ethyl-3-methyl-1H-pyrazole-5-carbonyl)imino)-7-(3-(4-(2-hydroxyethyl)piperazin-1-yl)propoxy)-2,3-dihydro-1H-benzo[d]imidazol-1-yl)but-2-en-1-yl)-2-((1-ethyl-3-methyl-1H-pyrazole-5-carbonyl)imino)-7-methoxy-2,3-dihydro-1H-benzo[d]imidazole-5-carboxamide as shown by the structure below:

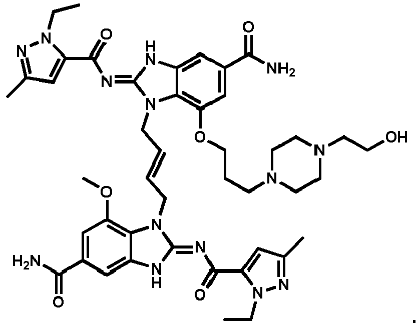

In one embodiment, the STING agonist is (E)-1-((E)-4-((E)-5-carbamoyl-2-((1-ethyl-3-methyl-1H-pyrazole-5-carbonyl)imino)-2,3-dihydro-1H-benzo[d]imidazol-1-yl)but-2-en-1-yl)-2-((1-ethyl-3-methyl-1H-pyrazole-5-carbonyl)imino)-7-(3-morpholinopropoxy)-2,3-dihydro-1H-benzo[d]imidazole-5-carboxamide (as shown below) or a pharmaceutically acceptable salt thereof: 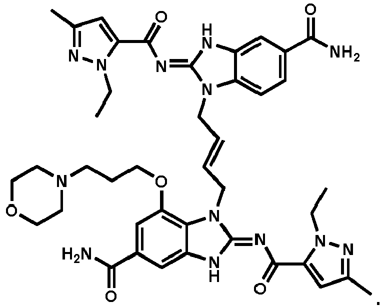 The compound may also be labelled as (*E*)-1-(4-(5-Carbamoyl-2-(1-ethyl-3-methyl-1H-pyrazole-5-carboxamido)-1H-benzo[d]imidazol-1-yl)but-2-en-1-yl)-2-(1-ethyl-3-methyl-1H-pyrazole-5-carboxamido)-7-(3-morpholinopropoxy)-1H-benzo[d]imidazole-5-carboxamide or (Z)-1-((E)-4-((Z)-5-carbamoyl-2-((1-ethyl-3-methyl-1H-pyrazole-5-carbonyl)imino)-2,3-dihydro-1H-benzo[d]imidazol-1-yl)but-2-en-1-yl)-2-((1-ethyl-3-methyl-1H-pyrazole-5-carbonyl)imino)-7-(3-morpholinopropoxy)-2,3-dihydro-1H-benzo[d]imidazole-5-carboxamide depending on its isomeric/tautomeric form.

In one embodiment, the STING agonist is (E)-1-((E)-4-((E)-5-carbamoyl-2-((1-ethyl-3-methyl-1H-pyrazole-5-carbonyl)imino)-2,3-dihydro-1H-benzo[d]imidazol-1-yl)but-2-en-1-yl)-2-((1-ethyl-3-methyl-1H-pyrazole-5-carbonyl)imino)-7-(3-hydroxypropoxy)-2,3-dihydro-1H-benzo[d]imidazole-5-carboxamide (as shown by the structure below) or a pharmaceutically acceptable salt thereof: 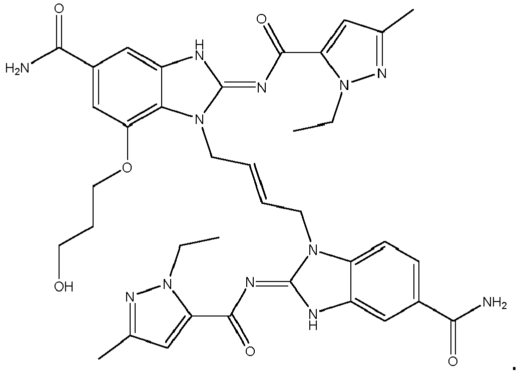 This compound may also be labelled (E)-1-(4-(5-carbamoyl-2-(1-ethyl-3-methyl-1H-pyrazole-5-carboxamido)-1H-benzo[d]imidazol-1-yl)but-2-en-1-yl)-2-(1-ethyl-3-methyl-1H-pyrazole-5-carboxamido)-7-(3-hydroxypropoxy)-1H-benzo[d]imidazole-5-carboxamide or (E)-1-((E)-4-((E)-5-carbamoyl-2-((1-ethyl-3-methyl-1H-pyrazole-5-carbonyl)imino)-2,3-dihydro-1H-benzo[d]imidazol-1-yl)but-2-en-1-yl)-2-((1-ethyl-3-methyl-1H-pyrazole-5-carbonyl)imino)-7-(3-hydroxypropoxy)-2,3-dihydro-1H-benzo[d]imidazole-5-carboxamide depending on its isomeric/tautomeric form.

In one embodiment, the STING agonist is (E)-1-((E)-4-((E)-5-carbamoyl-2-((1-ethyl-3-methyl-1H-pyrazole-5-carbonyl)imino)-7-(3-hydroxypropoxy)-2,3-dihydro-1H-benzo[d]imidazol-1-yl)but-2-en-1-yl)-2-((1-ethyl-3-methyl-1H-pyrazole-5-carbonyl)imino)-7-methoxy-2,3-dihydro-1H-benzo[d]imidazole-5-carboxamide as represented by the below formula or a pharmaceutically acceptable salt thereof: 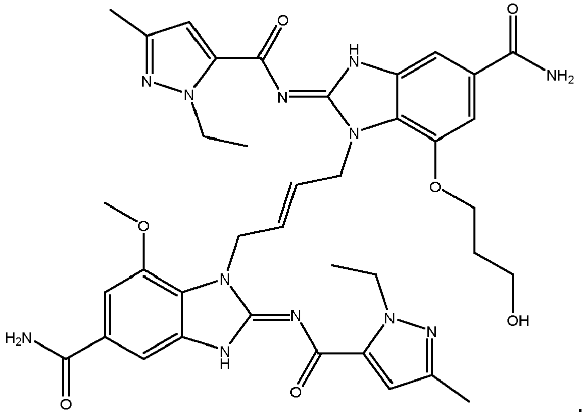 This compound may also be labelled (E)-1-(4-(5-Carbamoyl-2-(1-ethyl-3-methyl-1H-pyrazole-5-carboxamido)-7-(3-hydroxypropoxy)-1H-benzo[d]imidazol-1-yl) but-2-en-1-yl)-2-(1-ethyl-3-methyl-1H-pyrazole-5-carboxamido)-7-methoxy-1H-benzo[d]imidazole-5-carboxamide or (Z)-1-((E)-4-((Z)-5-carbamoyl-2-((1-ethyl-3-methyl-1H-pyrazole-5-carbonyl)imino)-7-(3-hydroxypropoxy)-2,3-dihydro-1H-benzo[d]imidazol-l-yl)but-2-en-l-yl)-2-((1-ethyl-3-methyl-1H-pyrazole-5-carbonyl)imino)-7-methoxy-2,3-dihydro-1H-benzo[d]imidazole-5-carboxamide depending on the isomeric/tautomeric form.

In one embodiment, the STING agonist is 3-(((E)-6-carbamoyl-3-((E)-4-((E)-5-carbamoyl-2-((1-ethyl-3-methyl-1H-pyrazole-5-carbonyl)imino)-7-methoxy-2,3-dihydro-1H-benzo[d]imidazol-1-yl)but-2-en-1-yl)-2-((1-ethyl-3-methyl-1H-pyrazole-5-carbonyl)imino)-2,3-dihydro-1H-benzo[d]imidazol-4-yl)oxy)propyl dihydrogen phosphate as represented by the below formula or a pharmaceutically acceptable salt thereof:

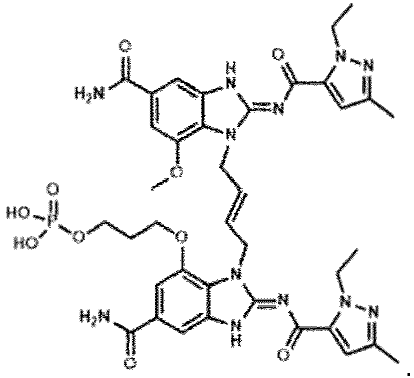

The compound may also be labelled as(E)-3-((5-carbamoyl-1-(4-(5-carbamoyl-2-(1-ethyl-3-methyl-1H-pyrazole-5-carboxamido)-7-methoxy-1H-benzo[d]imidazol-1-yl)but-2-en-1-yl)-2-(1-ethyl-3-methyl-1H-pyrazole-5-carboxamido)-1H-benzo[d]imidazol-7-yl)oxy)propyl dihydrogen phosphate or 3-(((E)-6-carbamoyl-3-((E)-4-((E)-5-carbamoyl-2-((1-ethyl-3-methyl-1H-pyrazole-5-carbonyl)imino)-7-methoxy-2,3-dihydro-1H-benzo[d]imidazol-1-yl)but-2-en-1-yl)-2-((1-ethyl-3-methyl-1H-pyrazole-5-carbonyl)imino)-2,3-dihydro-1H-benzo[d]imidazol-4-yl)oxy)propyl dihydrogen phosphate depending on the isomeric/tautomeric form.

In one embodiment, the STING agonist of Formula (I) or(II) in free base or free acid form.

As used herein, the STING agonist of Formula (I) or Formula (II) as defined herein, may be in any form, i.e., any tautomeric form, any isomeric form, any salt or no salt form (e.g., as a free acid or base form, or as a salt, for example a pharmaceutically acceptable salt thereof) and any physical form thereof (e.g., including non-solid forms such as liquid or semi-solid forms, and solid forms such as amorphous or crystalline forms, specific polymorphic forms, solvate forms, including hydrate forms such as mono-, di- and hemi- hydrates, and mixtures of various forms).

Accordingly, included for use within the present invention are the compounds of Formula (I) or Formula (II), as defined herein, in any salt or non-salt form and any physical form thereof, and mixtures of various forms. While such are included for use within the present invention, it will be understood that the compounds of Formula (I) or (II), as defined herein, in any salt or no salt form, and in any physical form thereof, may have varying levels of activity, different bioavailabilities and different handling properties for formulation purposes.

Since the STING agonist of Formula (I), or a pharmaceutically acceptable salt thereof, is intended for use in immunogenic compositions it will readily be understood that it is preferably provided in substantially pure form, for example at least 60% pure, more suitably at least 75% pure and preferably at least 85%, especially at least 98% pure (% are on a weight for weight basis). Impure preparations of a compound may be used for preparing the more pure form used in the immunogenic compositions.

The STING agonists for use in the invention may contain one or more asymmetric centers (also referred to as a chiral center), such as a chiral carbon, or a chiral -SO- moiety. Said STING agonists may contain one or more chiral centers may be present as racemic mixtures, diastereomeric mixtures, enantiomerically enriched mixtures, diastereomerically enriched mixtures, or as enantiomerically or diastereomerically pure individual stereoisomers.

The stereochemistry of the chiral center present in compounds of this invention is generally represented in the compound names and/or in the chemical structures illustrated herein. Where the stereochemistry of a chiral center present in a compound of this invention, or in any chemical structure illustrated herein, is not specified, the structure is intended to encompass any stereoisomer and all mixtures thereof. Accordingly, all STING agonists of Formula (I) or (II) and salts thereof, whether as individual isomers isolated such as to be substantially free of the other isomer (i.e. pure) or as mixtures (i.e. racemates and racemic mixtures) are encompassed for use in the invention. An individual isomer isolated such as to be substantially free of the other isomer (i.e. pure) may be isolated such that less than 10%, particularly less than about 1%, for example less than about 0.1% of the other isomer is present.

Individual stereoisomers of the STING agonists may be resolved (or mixtures of stereoisomers may be enriched) using methods known to those skilled in the art. For example, such resolution may be carried out (1) by formation of diastereoisomeric salts, complexes or other derivatives; (2) by selective reaction with a stereoisomer-specific reagent, for example by enzymatic oxidation or reduction; or (3) by gas-liquid or liquid chromatography in a chiral environment, for example, on a chiral support such as silica with a bound chiral ligand or in the presence of a chiral solvent. It will be appreciated that where the desired stereoisomer is converted into another chemical entity by one of the separation procedures described above, a further step is required to liberate the desired form. Alternatively, specific stereoisomers may be synthesized by asymmetric synthesis using optically active reagents, substrates, catalysts or solvents, or by converting one enantiomer to the other by asymmetric transformation.

The definition of the STING agonist used herein also includes various deuterated forms of the compounds. Each available hydrogen atom attached to a carbon atom may be independently replaced with a deuterium atom. A person of ordinary skill in the art will know how to synthesize deuterated forms of the compounds of this invention. For example, α-deuterated α-amino acids are commercially available or may be prepared by conventional techniques (see for example: Elemes, Y. and Ragnarsson, U. J. Chem. Soc., Perkin Trans. 1, 1996, 6, 537-40). Employing such compounds may allow for the preparation of compounds in which the hydrogen atom at a chiral center is replaced with a deuterium atom. Other commercially available deuterated starting materials may be employed in the preparation of deuterated analogs of the compounds of this invention (see for example: methyl-d₃-amine available from Aldrich Chemical Co., Milwaukee, WI), or they may be synthesized using conventional techniques employing deuterated reagents (e.g. by reduction using lithium aluminum deuteride or sodium borodeuteride or by metal-halogen exchange followed by quenching with D₂O or methanol-d₃).

In one embodiment, the STING agonist of Formula (I) or (II) is used in the form of a pharmaceutically acceptable salt.

Suitable pharmaceutically acceptable salts of the STING agonist of Formula (I) or (II) can include acid addition salts or base addition salts. For reviews of suitable pharmaceutically acceptable salts see Berge et al., J. Pharm. Sci., 66:1-19, (1977) and P. H. Stahl and C. G. Wermuth, Eds., Handbook of Pharmaceutical Salts: Properties, Selection and Use, Weinheim/Zürich:Wiley-VCH/VHCA (2002).

Salts of the STING agonist of Formula (I) or (II) containing a basic amine or other basic functional group may be prepared by any suitable method known in the art, such as treatment of the free base with a suitable inorganic or organic acid. Examples of pharmaceutically acceptable salts so formed include acetate, adipate, ascorbate, aspartate, benzenesulfonate, benzoate, camphorate, camphor-sulfonate (camsylate), caprate (decanoate), caproate (hexanoate), caprylate (octanoate), carbonate, bicarbonate, cinnamate, citrate, cyclamate, dodecylsulfate (estolate), ethane-1,2-disulfonate (edisylate), ethanesulfonate (esylate), formate, fumarate (hemi-fumarate, etc.), galactarate (mucate), gentisate (2,5-dihydroxybenzoate), glucoheptonate (gluceptate), gluconate, glucuronate, glutamate, glutarate, glycerophosphorate, glycolate, hippurate, hydrobromide, hydrochloride (dihydrochloride, etc.), hydroiodide, isobutyrate, lactate, lactobionate, laurate, maleate, malate, malonate, mandelate, methanesulfonate (mesylate), naphthalene-1,5-disulfonate (napadisylate), naphthalene-sulfonate (napsylate), nicotinate, nitrate, oleate, oxalate, palmitate, pamoate, phosphate (diphosphate, etc.), proprionate, pyroglutamate, salicylate, sebacate, stearate, succinate, sulfate, tartrate, thiocyanate, p-toluenesulfonate (tosylate), undecylenate, 1-hydroxy-2-naphthoate, 2,2-dichloroacetate, 2-hydroxyethanesulfonate (isethionate), 2-oxoglutarate, 4-acetamidobenzoate, and 4-aminosalicylate.

Salts of the disclosed compounds containing a carboxylic acid or other acidic functional group can be prepared by reacting with a suitable base. Such a pharmaceutically acceptable salt may be made with a base which affords a pharmaceutically acceptable cation, which includes alkali metal salts (especially sodium and potassium), alkaline earth metal salts (especially calcium and magnesium), aluminum salts and ammonium salts, as well as salts made from physiologically acceptable organic bases such as trimethylamine, triethylamine, morpholine, pyridine, piperidine, picoline, dicyclohexylamine, *N*,*N*'-dibenzylethylenediamine, 2-hydroxyethylamine, *bis*-(2-hydroxyethyl)amine, tri-(2-hydroxyethyl)amine, procaine, dibenzylpiperidine, dehydroabietylamine, *N*,*N*'-bisdehydroabietylamine, glucamine, *N*-methylglucamine, collidine, choline, quinine, quinoline, and basic amino acids such as lysine and arginine.

The invention includes within its scope all possible stoichiometric and non-stoichiometric forms of the salts (e.g., hydrobromide, dihydrobromide, fumarate, hemi-fumarate, etc.) of the STING agonist of Formula (I) or (II).

When a disclosed compound or its salt is named or depicted by structure, it is to be understood that the compound or salt, including solvates (particularly, hydrates) thereof, may exist in crystalline forms, non-crystalline forms or a mixture thereof. The compound or salt, or solvates (particularly, hydrates) thereof, may also exhibit polymorphism (i.e. the capacity to occur in different crystalline forms). These different crystalline forms are typically known as "polymorphs." It is to be understood that the invention includes all polymorphs of any compound of this invention, e.g., all polymorphic forms of any compound named or depicted by structure herein, including any salts and/or solvates (particularly, hydrates) thereof.

Polymorphs have the same chemical composition but differ in packing, geometrical arrangement, and other descriptive properties of the crystalline solid state. Polymorphs, therefore, may have different physical properties such as shape, density, hardness, deformability, stability, and dissolution properties. Polymorphs typically exhibit different melting points, IR spectra, and X-ray powder diffraction patterns, which may be used for identification. It will be appreciated that different polymorphs may be produced, for example, by changing or adjusting the conditions used in crystallizing/recrystallizing the compound. Polymorphic forms may be characterized and differentiated using a number of conventional analytical techniques, including, but not limited to, X-ray powder diffraction (XRPD) patterns, infrared (IR) spectra, Raman spectra, differential scanning calorimetry (DSC), thermogravimetric analysis (TGA) and solid state nuclear magnetic resonance (SSNMR).

The skilled artisan will appreciate that pharmaceutically acceptable solvates (particularly, hydrates) of the STING agonist, including pharmaceutically acceptable solvates of a pharmaceutically acceptable salt of the STING agonist, may be formed when solvent molecules are incorporated into the crystalline lattice during crystallization. Solvates may involve non-aqueous solvents such as ethanol, or they may involve water as the solvent that is incorporated into the crystalline lattice. Solvates wherein water is the solvent that is incorporated into the crystalline lattice are typically referred to as "hydrates."

The STING agonist defined herein includes within its scope all possible stoichiometric and non-stoichiometric salt and/or hydrate forms.

Salts and solvates (e.g. hydrates and hydrates of salts) of the STING agonist used in the present invention includes all which are suitable for use in medicine are those wherein the counterion or associated solvent is pharmaceutically acceptable. Salts having non-pharmaceutically acceptable counterions are within the scope of the present invention, for example, for use as intermediates in the preparation of other compounds of the invention.

Typically, a pharmaceutically acceptable salt may be readily prepared by using a desired acid or base as appropriate. The resultant salt may crystallize or precipitate from solution, or form by trituration, and may be recovered by filtration, or by evaporation of the solvent.

The invention includes all prodrugs of the STING agonists of Formula (I), which upon administration to the recipient are capable of providing (directly or indirectly) a compound of Formula (I), or an active metabolite or residue thereof. Such derivatives are recognisable to those skilled in the art, without undue experimentation. Nevertheless, reference is made to the teaching of Burger's Medicinal Chemistry and Drug Discovery, 5th Edition, Vol 1: Principles and Practice, which is incorporated herein by reference to the extent of teaching such derivatives.

It is to be further understood that the present invention includes within its scope all tautomeric or isomer forms of any free base form of the compounds of this invention as well as all possible stoichiometric and non-stoichiometric salt forms.

### ALUMINIUM salt

The adjuvant composition of the present invention includes aluminium hydroxide, aluminium phosphate, aluminium oxyhydroxide, or aluminium hydroxyphosphate.

Suitable forms of aluminium hydroxide, aluminium phosphate, aluminium oxyhydroxide, or aluminium hydroxyphosphate for adjuvant use are well known to the skilled person. In one embodiment, the adjuvant composition includes aluminium phosphate, aluminium hydroxide or a combination thereof. Suitable forms of aluminium hydroxide, aluminium phosphate, aluminium oxyhydroxide, or aluminium hydroxyphosphate include but are not limited to Rehydragel^{™} HS, Alhydrogel^{™} 85, Rehydragel^{™} PM, Rehydragel^{™} AB, Rehydragel^{™} HPA, Rehydragel^{™} LV, Alhydrogel^{™} or a combination thereof.

In one embodiment, the adjuvant composition of the present invention includes aluminium phosphate.

In one embodiment, the adjuvant composition of the present invention includes aluminium hydroxide, aluminium oxyhydroxide, or aluminium hydroxyphosphate.

In one embodiment, the adjuvant composition of the present invention includes aluminium hydroxide. In one embodiment, the adjuvant composition includes aluminium hydroxide and does not include (i.e. is substantially free from) aluminium oxyhydroxide, or aluminium hydroxyphosphate.

In one embodiment, the adjuvant composition of the present invention includes aluminium phosphate.

In one embodiment, the adjuvant composition of the present invention includes aluminium oxyhydroxide.

In one embodiment, the adjuvant composition of the present invention includes aluminium hydroxyphosphate.

In one embodiment, the adjuvant composition of the present invention includes aluminium hydroxide. In one embodiment, the adjuvant composition of the present invention includes aluminium hydroxide, and a STING agonist of Formula (I) selected from the group consisting of
(E)-1-(4-(5-carbamoyl-2-(1-ethyl-3-methyl-1H-pyrazole-5-carboxamido)-1H-benzo[d]imidazol-1-yl)but-2-en-1-yl)-2-(1-ethyl-3-methyl-1H-pyrazole-5-carboxamido)-7-(3-hydroxypropoxy)-1H-benzo[d]imidazole-5-carboxamide;
(E)-1-((E)-4-((E)-5-carbamoyl-2-((1-ethyl-3-methyl-1H-pyrazole-5-carbonyl)imino)-2,3-dihydro-1H-benzo[d]imidazol-1-yl)but-2-en-1-yl)-2-((1-ethyl-3-methyl-1H-pyrazole-5-carbonyl)imino)-7-(3-hydroxypropoxy)-2,3-dihydro-1H-benzo[d]imidazole-5-carboxamide;
(Z)-1-((E)-4-((Z)-5-carbamoyl-2-((1-ethyl-3-methyl-1H-pyrazole-5-carbonyl)imino)-2,3-dihydro-1H-benzo[d]imidazol-1-yl)but-2-en-1-yl)-2-((1-ethyl-3-methyl-1H-pyrazole-5-carbonyl)imino)-7-(3-hydroxypropoxy)-2,3-dihydro-1H-benzo[d]imidazole-5-carboxamide;
(E)-4-((5-carbamoyl-1-(4-(5-carbamoyl-2-(1-ethyl-3-methyl-1H-pyrazole-5-carboxamido)-1H-benzo[d]imidazol-1-yl)but-2-en-1-yl)-2-(1-ethyl-3-methyl-1H-pyrazole-5-carboxamido)-1H-benzo[d]imidazol-7-yl)oxy)butanoic acid;
(E)-1-(4-(5-carbamoyl-2-(1-ethyl-3-methyl-1H-pyrazole-5-carboxamido)-7-methoxy-1H-benzo[d]imidazol-1-yl)but-2-en-1-yl)-7-(3-(dimethylamino)propoxy)-2-(1-ethyl-3-methyl-1H-pyrazole-5-carboxamido)-1H-benzo[d]imidazole-5-carboxamide;
(E)-1-((E)-4-((E)-5-carbamoyl-2-((1-ethyl-3-methyl-1H-pyrazole-5-carbonyl)imino)-7-(3-(4-(2-hydroxyethyl)piperazin-1-yl)propoxy)-2,3-dihydro-1H-benzo[d]imidazol-1-yl)but-2-en-1-yl)-2-((1-ethyl-3-methyl-1H-pyrazole-5-carbonyl)imino)-7-methoxy-2,3-dihydro-1H-benzo[d]imidazole-5-carboxamide;
(E)-1-((E)-4-((E)-5-carbamoyl-2-((1-ethyl-3-methyl-1H-pyrazole-5-carbonyl)imino)-7-(3-hydroxypropoxy)-2,3-dihydro-1H-benzo[d]imidazol-1-yl)but-2-en-1-yl)-2-((1-ethyl-3-methyl-1H-pyrazole-5-carbonyl)imino)-7-methoxy-2,3-dihydro-1H-benzo[d]imidazole-5-carboxamide;
(E)-1-(4-(5-carbamoyl-2-(1-ethyl-3-methyl-1H-pyrazole-5-carboxamido)-1H-benzo[d]imidazol-1-yl)but-2-en-1-yl)-2-(1-ethyl-3-methyl-1H-pyrazole-5-carboxamido)-7-(3-morpholinopropoxy)-1H-benzo[d]imidazole-5-carboxamide;
(E)-1-((E)-4-((E)-5-carbamoyl-2-((1-ethyl-3-methyl-1H-pyrazole-5-carbonyl)imino)-2,3-dihydro-1H-benzo[d]imidazol-1-yl)but-2-en-1-yl)-2-((1-ethyl-3-methyl-1H-pyrazole-5-carbonyl)imino)-7-(3-morpholinopropoxy)-2,3-dihydro-1H-benzo[d]imidazole-5-carboxamide;
(Z)-1-((E)-4-((Z)-5-carbamoyl-2-((1-ethyl-3-methyl-1H-pyrazole-5-carbonyl)imino)-2,3-dihydro-1H-benzo[d]imidazol-1-yl)but-2-en-1-yl)-2-((1-ethyl-3-methyl-1H-pyrazole-5-carbonyl)imino)-7-(3-morpholinopropoxy)-2,3-dihydro-1H-benzo[d]imidazole-5-carboxamide;
(E)-1-(4-(5-carbamoyl-2-(1-ethyl-3-methyl-1H-pyrazole-5-carboxamido)-7-(3-morpholinopropoxy)-1H-benzo[d]imidazol-1-yl)but-2-en-1-yl)-2-(1-ethyl-3-methyl-1H-pyrazole-5-carboxamido)-7-methoxy-1H-benzo[d]imidazole-5-carboxamide;
(E)-1-((E)-4-((E)-5-carbamoyl-2-((1-ethyl-3-methyl-1H-pyrazole-5-carbonyl)imino)-7-(3-morpholinopropoxy)-2,3-dihydro-1H-benzo[d]imidazol-1-yl)but-2-en-1-yl)-2-((1-ethyl-3-methyl-1H-pyrazole-5-carbonyl)imino)-7-methoxy-2,3-dihydro-1H-benzo[d]imidazole-5-carboxamide;
(E)-3-((5-carbamoyl-1-(4-(5-carbamoyl-2-(1-ethyl-3-methyl-1H-pyrazole-5-carboxamido)-7-methoxy-1H-benzo[d]imidazol-1-yl)but-2-en-1-yl)-2-(1-ethyl-3-methyl-1H-pyrazole-5-carboxamido)-1H-benzo[d]imidazol-7-yl)oxy)propyl dihydrogen phosphate;
3-(((Z)-6-carbamoyl-3-((E)-4-((Z)-5-carbamoyl-2-((1-ethyl-3-methyl-1H-pyrazole-5-carbonyl)imino)-7-methoxy-2,3-dihydro-1H-benzo[d]imidazol-1-yl)but-2-en-1-yl)-2-((1-ethyl-3-methyl-1H-pyrazole-5-carbonyl)imino)-2,3-dihydro-1H-benzo[d]imidazol-4-yl)oxy)propyl dihydrogen phosphate;
3-(((E)-6-carbamoyl-3-((E)-4-((E)-5-carbamoyl-2-((1-ethyl-3-methyl-1H-pyrazole-5-carbonyl)imino)-7-methoxy-2,3-dihydro-1H-benzo[d]imidazol-1-yl)but-2-en-1-yl)-2-((1-ethyl-3-methyl-1H-pyrazole-5-carbonyl)imino)-2,3-dihydro-1H-benzo[d]imidazol-4-yl)oxy)propyl dihydrogen phosphate; or
a pharmaceutically acceptable salt thereof.

In one embodiment, the adjuvant composition of the present invention comprises aluminium hydroxide and a STING agonist which is 3-(((E)-6-carbamoyl-3-((E)-4-((E)-5-carbamoyl-2-((1-ethyl-3-methyl-1H-pyrazole-5-carbonyl)imino)-7-methoxy-2,3-dihydro-1H-benzo[d]imidazol-1-yl)but-2-en-1-yl)-2-((1-ethyl-3-methyl-1H-pyrazole-5-carbonyl)imino)-2,3-dihydro-1H-benzo[d]imidazol-4-yl)oxy)propyl dihydrogen phosphate, or a pharmaceutically acceptable salt thereof.

In one embodiment, the aluminium hydroxide, aluminium phosphate, aluminium oxyhydroxide, or aluminium hydroxyphosphate (e.g., the aluminium hydroxide) may have a protein adsorption capacity of between 2.5 and 3.5, 2.6 and 3.4, 2.7 and 3.3 or 2.9 and 3.2, 2.5 and 3.7, 2.6 and 3.6, 2.7 and 3.5, or 2.8 and 3.4 protein (BSA)/mL Al³⁺. In one embodiment of the present invention, the aluminium hydroxide has a protein adsorption capacity of between 2.9 and 3.2 mg BSA/mg Al³⁺. Protein adsorption capacity of the aluminium salt can be measured by any means known to the skilled person. The protein adsorption capacity of the aluminium salt may be measured using the method as described in Example 1 of WO 12/136823 (which utilises BSA) or variations thereof. Aluminium hydroxide described herein (i.e. having the protein adsorption capacity described herein) may have a crystal size of between 2.8 and 5.7nm as measured by X-ray diffraction, for example 2.9 to 5.6nm, 2.8 to 3.5nm, 2.9 to 3.4nm or 3.4 to 5.6nm or 3.3 and 5.7nm as measured by X-ray diffraction. X-ray diffraction is well known to the skilled person. In a particular embodiment of the invention the crystal size is measured using the method described in Example 1 of WO 12/136823 or variations thereof.

In embodiments wherein the adjuvant composition of the invention further comprises an antigen, the antigen may be adsorbed onto the aluminium salt. This can be done prior to mixing.

The meaning of "adsorbed antigen" is for example taken to mean greater than 20%, 30%, 40%, 50%, 60%, 70%, 80%, or 90% adsorbed. The amount of the adsorbed antigen can be measured using HPLC (e.g., by centrifuging the composition and conducting HPLC on the supernatant). In one embodiment, the antigen is adsorbed in an amount of greater than 20% (e.g., greater than 50%) onto the aluminium hydroxide, aluminium phosphate, aluminium oxyhydroxide, or aluminium hydroxyphosphate. In one embodiment, the antigen is adsorbed onto aluminium hydroxide in an amount of greater than 50%. In some embodiments, the antigen is adsorbed onto aluminium hydroxide in an amount of greater than 60%. In some embodiments, the antigen is adsorbed onto aluminium hydroxide in an amount of greater than 70%. In some embodiments, the antigen is adsorbed onto aluminium hydroxide in an amount of greater than 80%. In one embodiment, the antigen is adsorbed onto aluminium hydroxide in an amount of greater than 90%.

The meaning of "adsorbed STING agonist" is for example taken to mean greater than 20%, 30%, 40%, 50%, 60%, 70%, 80%, or 90% adsorbed. The amount of the adsorbed STING agonist can be measured using HPLC (e.g., by centrifuging the composition and conducting HPLC on the supernatant).

In one embodiment, the adjuvant composition of the present invention includes a ratio of STING agonist toaluminium hydroxide, aluminium phosphate, aluminium oxyhydroxide, or aluminium hydroxyphosphate of 1:2.5-250. In one embodiment, the adjuvant composition of the present invention contains the STING agonist defined herein and aluminium hydroxide in an amount of 1:1-250. In one embodiment, the ratio of STING agonist to aluminium hydroxide is 1:2.5-100.

In one embodiment, the ratio of STING agonist to aluminium hydroxide is 1:1-10. In one embodiment, the ratio of STING agonist to aluminium hydroxide is 1:1. In one embodiment, the ratio of STING agonist to aluminium hydroxide is 1:5.

In one embodiment, the ratio of STING agonist to aluminium hydroxide is 1:10-100. In one embodiment, the ratio of STING agonist to aluminium hydroxide is 1:10. In one embodiment, the ratio of STING agonist to aluminium hydroxide is 1:25. In one embodiment, the ratio of STING agonist to aluminium hydroxide is 1:50. In one embodiment, the ratio of STING agonist to aluminium hydroxide is 1:75. In one embodiment, the ratio of STING agonist to aluminium hydroxide is 1:50-100. In one embodiment, the ratio of STING agonist to aluminium hydroxide is 1:50-125. In one embodiment, the ratio of STING agonist to aluminium hydroxide is 1:100.

In one embodiment, the ratio of STING agonist to aluminium hydroxide is 1: 100-200. In one embodiment, the ratio of STING agonist to aluminium hydroxide is 1:150. In one embodiment, the ratio of STING agonist to aluminium hydroxide is 1:175. In one embodiment, the ratio of STING agonist to aluminium hydroxide is 1:200. In one embodiment, the ratio of STING agonist to aluminium hydroxide is 1:250.

In one embodiment, the immunogenic composition of the present invention includes aluminium hydroxide, and the ratio of antigen to aluminium hydroxide is 1:1 to 50. In one embodiment, the immunogenic composition of the present invention includes aluminium hydroxide, and the ratio of antigen to aluminium hydroxide is 1:1 to 25. In one embodiment, the immunogenic composition of the present invention includes aluminium hydroxide, and the ratio of antigen to aluminium hydroxide is 1:1 to 10. In one embodiment, the immunogenic composition of the present invention includes aluminium hydroxide, and the ratio of antigen to aluminium hydroxide is 1:1 to 5. In one embodiment, the ratio of antigen to aluminium hydroxide is 1:3.125.

### FORMULATIONS

The adjuvant compositions or immunogenic compositions of the present invention may be administered via various suitable routes, including parenteral, such as intramuscular or subcutaneous administration. In one embodiment, the adjuvant composition is adapted for intramuscular administration. In one embodiment, the adjuvant composition of the present invention is adapted for subcutaneous administration. In one embodiment, the adjuvant composition of the present invention is adapted for intranasal administration.

The adjuvant composition of the present invention may be administered with an antigen. In embodiments where the adjuvant composition of the present invention is administered in combination with an antigen, the antigen may be administered separately from the adjuvant composition of the present invention or may be administered within the same composition as the adjuvant composition of the present invention. The antigen may also be administered via a different route to the adjuvant composition of the present invention, when co-administered separately.

In one embodiment, the adjuvant composition is in aqueous form.

In one embodiment, the adjuvant composition is in non-aqueous form.

The pH of the adjuvant composition can be adjusted in view of the components and necessary suitability for administration to the subject. In one embodiment, the pH of the adjuvant composition is at least 4, at least 5, at least 5.5, at least 5.8, at least 6. The pH of the adjuvant composition may be less than 9, less than 8, less than 7.5 or less than 7.

In one embodiment, the pH of the adjuvant composition is between 4 and 9, between 5 and 8, such as between 5.5 and 8. In a further embodiment, a buffer is added to the formulation. It is well known that for parenteral administration solutions should have a pharmaceutically acceptable osmolality to avoid cell distortion or lysis. A pharmaceutically acceptable osmolality will generally mean that solutions will have an osmolality which is approximately isotonic or mildly hypertonic. Suitably the adjuvant compositions of the present invention will have an osmolality in the range of 250 to 750 mOsm/kg, for example, the osmolality may be in the range of 250 to 550 mOsm/kg, such as in the range of 280 to 500 mOsm/kg.

Osmolality may be measured according to techniques known in the art, such as by the use of a commercially available osmometer, for example the Advanced Model 2020 available from Advanced Instruments Inc. (USA). A desired osmolality may be achieved by the inclusion of salts or through the use of non-ionic isotonicity agents. In one embodiment of the present invention, suitable non-ionic isotonicity agents are polyols, sugars (in particular sucrose, fructose, dextrose or glucose) or amino acids such as glycine. In one embodiment the polyol is a sugar alcohol especially a C₃₋₆ sugar alcohol. Exemplary sugar alcohols include glycerol, erythritol, threitol, arabitol, xylitol, ribitol, sorbitol, mannitol, dulcitol and iditol. In a specific example of this embodiment, a suitable non-ionic isotonicity agent is sorbitol. In a particular embodiment of the invention the non-ionic isotonicity agent in the formulations of the invention is sucrose and/or sorbitol. Parenteral compositions are suitably sterile.

In one embodiment, the adjuvant composition is in the form of a parenteral dosage forms suitable to be administered in the form of sterile or sterilizable injectable solutions, suspensions, dry and/or lyophilized products ready to be dissolved or suspended in a pharmaceutically acceptable vehicle for injection (reconstitutable powders) and emulsions. Vehicles used in such dosage forms include, but are not limited to, Water for Injection USP; aqueous vehicles such as, but not limited to, Sodium Chloride Injection, Ringer's Injection, Dextrose Injection, Dextrose and Sodium Chloride Injection, and Lactated Ringer's Injection; water-miscible vehicles such as, but not limited to, ethyl alcohol, polyethylene glycol, and polypropylene glycol; and non-aqueous vehicles such as, but not limited to, corn oil, cottonseed oil, peanut oil, sesame oil, ethyl oleate, isopropyl myristate, and benzyl benzoate.

### ANTIGEN

The adjuvant composition of the present invention may be administered in combination with an antigen. The antigen may be formulated separately or in the same formulation (i.e., the antigen may be administered as part of the same formulation or a separate formulation).

The term "antigen" refers to any molecule capable of raising an immune response in a human or animal. The immune response is a protective immune response, e.g. reducing partially or completely the severity of one or more symptoms and/or time over which one or more symptoms are experienced by a subject, reducing the likelihood of developing an established infection after challenge and/or slowing progression of an associated illness (e.g. extending survival).

The antigen may be a whole-organism, a protein/polypeptide, a polysaccharide, a peptide, a protein-polysaccharide conjugate, or a hapten capable of raising an immune response in a human or an animal, each of these types of antigen, or any combination of two or more thereof, being specifically contemplated as a possible antigen in specific embodiments of the adjuvant composition of the present invention. In the sense of the present invention, the terms "protein" and "polypeptide" are synonymous and interchangeable.

The immune response may be raised against a pathogen, such as for example, viruses, bacteria, parasites or fungus. That is, in one embodiment, the antigen is derived from a human pathogen. In one embodiment, the antigen is derived from a human pathogen selected from the group consisting of bacteria, virus, fungi, parasitic microorganisms and multicellular parasites. In one embodiment, the antigen is derived from a combination of two or more bacteria, virus, fungi, parasitic microorganisms and multicellular parasites.

Alternatively, the antigen may be derived from a tumor cell (i.e., the antigen may be a tumor-associated antigen), and the adjuvant composition of the present invention may be useful for the immunotherapeutic treatment of cancers.

In the sense of the present invention, "an antigen derived from an organism" encompasses, in particular, the organism as a whole (whole-organisms, such as for example a whole-virus or a whole-bacterium), or one or more molecules only from the organism. The antigen may be the naturally occurring whole-organism, and the one or more molecules for instance one or more polypeptides, from the organism may be isolated and purified from such naturally occurring whole-organism.

Alternatively, the antigen may be artificially produced, for example, using recombinant technology or using chemical synthesis. Such recombinant antigens may be in a wild-type form, i.e. their nucleotide sequence, or amino acid sequence, is identical to the sequence of the corresponding antigens derived from the naturally occurring whole-organism. Alternatively, said recombinant antigens may advantageously comprise one or more mutations, i.e. their nucleotide sequence, or amino acid sequence, comprises one or more mutations, as compared with the sequence of the corresponding wild type antigens. Whole-organisms may be live attenuated or killed/inactivated. Inactivation processes using physical and/or chemical means are known to the skilled person.

In some embodiments, the antigen comprises at least one B or T cell epitope, an antigen comprises B and T cell epitopes. The elicited immune response may be an antigen specific B cell response which produces neutralizing antibodies. The elicited immune response may be an antigen specific T cell response, which may be a systemic and/or a local response. The antigen specific T cell response may comprise a CD4+ T cell response, such as a response involving CD4+ T cells expressing a plurality of cytokines, e.g. IFN gamma, TNF alpha and/or IL2. Alternatively, or additionally, the antigen specific T cell response comprises a CD8+ T cell response, such as a response involving CD8+ T cells expressing a plurality of cytokines, e.g., IFN gamma, TNF alpha and/or IL2.

Suitably the encoded antigen contains 3000 residues or fewer, especially 2000 residues or fewer, in particular 1500 residues or fewer. The encoded antigen may contain 1000 residues or fewer, 800 residues or fewer, 600 residues or fewer, 400 residues or fewer or 200 residues or fewer.

Suitably the antigen contains 50 residues or more, especially 100 residues or more, in particular 150 residues or more.

Suitably the antigen contains 50 to 3000 residues, especially 100 to 1500 residues, in particular 200 to 1000 residues.

### VIRAL ANTIGENS

The antigen used either in or with the adjuvant composition of the present invention may derive from a virus. Accordingly, in particular embodiments, the antigen derives from a virus. In particular, the antigen may be a whole-virus. The whole virus may be live attenuated or killed/inactivated. Alternatively, the antigen may be a polypeptide derived from a virus.

Suitable viruses are from the families Orthomyxoviridae, such as for instance influenza viruses, Paramyxoviridae, such as for instance respiratory syncytial viruses (RSV), mumps virus or measles, Togaviridae, such as for instance rubella virus, Papovaviridae, such as for instance human papillomaviruses (HPV), Herpesviridae, such as for instance herpes simplex viruses (HSV), human cytomegaloviruses (HCMV), Epstein-Barr viruses (EBV), or varicella-zoster viruses (VZV), Picornaviridae, such as for instance enteroviruses, rhinoviruses, polioviruses, Fiaviviridae, such as for instance Dengue viruses or hepatitis C virus (HCV), Hepadnaviridae, such as for instance hepatitis B virus (HBV), Retroviridae, such as for instance human immunodeficiency viruses (HIV), Reoviridae, such as for example rotaviruses, Rhabdoviridae, such as for instance rabies viruses, or Flloviridae, such as for example Ebola virus. In one embodiment, the antigen used either in or with the adjuvant composition of the present invention derives from a virus selected from the group consisting of influenza virus, RSV, HPV, measles, rubella virus, mumps virus, HCMV, VZV, Dengue virus, poliovirus, HIV, HBV, Ebola virus and rotavirus, or any combination of two or more thereof.

In a particular embodiment, the antigen derives from HCMV. Suitably, the HCMV antigen is the glycoprotein gB, which may lack the transmembrane domain (as disclosed in EP0802979), optionally in combination with one or more of the HCMV proteins pp65, IE1, pUL131, gL, gH, pUL128, and pUL130. Suitably, the HCMV antigen is a combination of gB, gL, gH, pUL131, pUL128 and pUL130. Alternatively, the HCMV antigen is a combination of gL, gH, pUL131, pUL128 and pUL130.

In one embodiment, the antigen is a varicella-zoster viruses (VZV) antigen. In one embodiment, the VZV antigen is a gE antigen. Suitably, the VZV antigen is the glycoprotein gE, which may be deleted from its transmembrane domain, as disclosed in EP0405867 B1. In one embodiment, the VZV antigen is a protein derived Varicella Zoster Virus which is one of gpI, gpII or gpIII as defined on pages 5-7 of EP 0 405 867 B1 and is missing from 4-20 percent of the total amino acid residues of the full length glycoprotein at the carboxy terminal end.

In a further embodiment, the antigen derives from RSV. Suitably, the RSV antigen is a polypeptide selected from the group consisting of the fusion protein (F), the attachment protein (G), the matrix protein (M2) and the nucleoprotein (N). Particularly suitable as an RSV polypeptide antigen to be included in or administered with the adjuvant composition of the present invention are conformationally constrained F polypeptides. Conformationally constrained F polypeptides have previously been described in both the prefusion (PreF) and postfusion (PostF) conformations. Exemplary F protein antigens conformationally constrained in the prefusion conformation have been described in the art and are disclosed in detail in e.g. WO 09/079796, WO 10/149745, WO 11/008974 and WO 12/158613. Likewise, F protein antigens conformationally constrained in the postfusion conformation are also well known in the art and can be used in or administered with the adjuvant composition of the present invention. Examples of postfusion conformationally constrained F protein polypeptides are disclosed in details in e.g. WO 11/008974, and Swanson et al. (PNAS, 2011, Vol. 108: 9619-9624). In particular embodiments, the adjuvant composition of the present invention comprises an antigen polypeptide derived from RSV selected from the group consisting of: F protein, preF protein, N protein and M2 protein.

In a further embodiment, the antigen derives from HBV. Suitably, the antigen is the Hepatitis B surface antigen (HBS)

In one embodiment, the antigen is derived from a coronavirus, particularly from SARS-CoV-2.

A plurality of antigens maybe encoded. Consequently, in some embodiments the antigen is derived from at least one coronavirus, for example from SARS-CoV-2. In some embodiments the antigen is derived from more than one coronavirus (such as 2, 3, 4 or 5), for example from SARS-CoV-2 (such as a plurality of SARS-CoV-2 variant antigens).

SARS-CoV-2 makes use of a densely glycosylated spike (S) protein to gain entry into host cells. In coronaviruses, the S protein is a trimeric class I fusion protein which exists in a metastable pre-fusion conformation that undergoes a substantial structural rearrangement to fuse the viral membrane with the host cell membrane (Li, 2016; Bosch, 2003).

A coronavirus protein of use in the present invention is a fragment or variant of a native coronavirus protein which is capable of eliciting neutralising antibodies and/or a T cell response (such as a CD4 or CD8 T cell response) to a coronavirus, suitably a protective immune response.

A SARS-CoV-2 S protein of use in the present invention comprises, such as consists of, a fragment or variant of a native SARS-CoV-2 S protein which is capable of eliciting neutralising antibodies and/or a T cell response (such as a CD4 or CD8 T cell response) to SARS-CoV-2, suitably a protective immune response.

The encoded SARS-CoV-2 S protein may comprise, such as consist of, a full length S protein (such as SEQ ID NO:1). Alternatively, the encoded SARS-CoV-2 S protein may comprise, such as consist of, an amino acid sequence having at least 90% identity to the amino acid sequence set forth in SEQ ID NO:1. The encoded SARS-CoV-2 S protein may comprise, such as consist of, an amino acid sequence having at least 95% identity to the amino acid sequence set forth in SEQ ID NO:1, especially at least 98% identity to the amino acid sequence set forth in SEQ ID NO:1, in particular at least 99% identity to the amino acid sequence set forth in SEQ ID NO:1, such as 100% identity to the amino acid sequence set forth in SEQ ID NO:1.

The encoded SARS-CoV-2 S protein may comprise, or consist of, one or more domains of a full length SARS-CoV-2 S protein, such as the ectodomain (SEQ ID NO:2) or receptor binding domain (RBD, SEQ ID NO:3), or variants thereof.

The encoded SARS-CoV-2 S protein may comprise, such as consist of, an amino acid sequence having at least 90% identity to the amino acid sequence set forth in SEQ ID NO:2. The encoded SARS-CoV-2 S protein may comprise, such as consist of, an amino acid sequence having at least 95% identity to the amino acid sequence set forth in SEQ ID NO:2, especially at least 98% identity to the amino acid sequence set forth in SEQ ID NO:2, in particular at least 99% identity to the amino acid sequence set forth in SEQ ID NO:2, such as 100% identity to the amino acid sequence set forth in SEQ ID NO:2.

The encoded SARS-CoV-2 S protein may comprise, such as consist of, an amino acid sequence having at least 90% identity to the amino acid sequence set forth in SEQ ID NO:3. The encoded SARS-CoV-2 S protein may comprise, such as consist of, an amino acid sequence having at least 95% identity to the amino acid sequence set forth in SEQ ID NO:3, especially at least 98% identity to the amino acid sequence set forth in SEQ ID NO:3, in particular at least 99% identity to the amino acid sequence set forth in SEQ ID NO:3, such as 100% identity to the amino acid sequence set forth in SEQ ID NO:3.

Suitably the encoded SARS-CoV-2 S protein is pre-fusion stabilised to facilitate appropriate presentation to the immune system. For example, Wrapp and colleagues (Wrapp *et al.*, 2020) produced a recombinant prefusion S ectodomain using a stabilization strategy that proved effective for other beta coronavirus S proteins (Pallesen *et al,* 2017; Kirchdoerfer *et al,* 2018). To this end, starting with the SARS-CoV-2 polynucleotide sequence (GenBank accession number MN908947.3), a gene encoding residues 1 to 1208 of SARS-CoV-2 S protein (UniProt accession number P0DTC2 version 1 dated 22 April 2020) with proline substitutions at residues 986 and 987, a "GSAS" substitution at the furin cleavage site (residues 682-685) a C-terminal T4 fibritin trimerization motif, an HRV3C protease cleavage site, a TwinStrepTag and an 8XHisTag was synthesized and cloned into the mammalian expression vector paH.

Residues 1 to 1208 of SARS-CoV-2 S protein with proline substitutions at residues 986 and 987, a "GSAS" substitution at the furin cleavage site are provided in SEQ ID NO:4, which is an example of a pre-fusion stabilized ectodomain of SARS-CoV-2 S protein.

The encoded SARS-CoV-2 S protein may comprise, such as consist of, an amino acid sequence having at least 90% identity to the amino acid sequence set forth in SEQ ID NO:4. The encoded SARS-CoV-2 S protein may comprise, such as consist of, an amino acid sequence having at least 95% identity to the amino acid sequence set forth in SEQ ID NO:4, especially at least 98% identity to the amino acid sequence set forth in SEQ ID NO:4, in particular at least 99% identity to the amino acid sequence set forth in SEQ ID NO:4, such as 100% identity to the amino acid sequence set forth in SEQ ID NO:4.

Suitably the SARS-CoV-2 S protein is a pre-fusion stabilised protein.

In one embodiment, the SARS-CoV-2 S protein is the stabilized recombinant prefusion S ectodomain disclosed by Wrapp *et al.*, 2020.

The SARS-CoV-2 S protein (such as a pre-fusion stabilized SARS-CoV-2 S protein) may desirably be in the form of a trimer and consequently may comprise a trimerization motif, such as a T4 fibritin trimerization motif, more suitably a C-terminal T4 fibritin trimerization motif. Alternative trimerization motifs include, for example, a domain derived from collagen called 'Trimer-Tag' such as disclosed in Liu *et al.*, 2017, or a molecular clamp, such as that disclosed in WO2018/176103.

An encoded SARS-CoV-2 S protein is desirably 1800 residues or fewer in length, especially 1500 residues or fewer, in particular 1400 residues or fewer, such as 1300 residues or fewer.

An encoded SARS-CoV-2 S protein is desirably 150 residues or more in length, especially 200 residues or more, in particular 400 residues or more, such as 600 residues or more.

In one embodiment, the antigen is a human cytomegalovirus (CMV) antigen.

In one embodiment, the antigen is a Zika virus antigen.

In one embodiment, the antigen is a human parainfluenza virus (PIV) antigen, such as a human PIV type 3 antigen.

In one embodiment the antigen is a human metapneumovirus (hMPV) antigen.

In one embodiment the antigen is a respiratory syncytial virus (RSV) antigen.

In one embodiment the antigen is an influenza virus antigen, such as a hemagglutinin or a neuraminidase.

In one embodiment the antigen is an Epstein-Barr virus (EBV) antigen.

In one embodiment, the antigen is an Herpes simplex virus (HSV) antigen, such as a gE and/or a gI antigen. Suitable antigens are disclosed in WO 2021/013798. In one embodiment, the antigen comprises i) a HSV2 gE antigen or HSV1 gE antigen, for example as defined in Figure 1 of WO 2021/013798, and/or ii) an HSV2 gI or HSV2 gI antigen, for example as defined in Figure 2 of WO 2021/013798. In a particular embodiment, the antigen comprises a HSV2 gE and gI heterodimer or an immunogenic fragment thereof.

### BACTERIA

The antigen used either in or with the adjuvant composition of the present invention may derive from a bacterium. Accordingly, in particular embodiments, the antigen derives from a bacterium. In one embodiment, the antigen is from a bacterium selected from the group consisting of: B. pertussis, S. Pneumoniae, and N. meningitidis, or any combination of two or more thereof.

The antigen may be a whole-bacterium and may be killed/inactivated or live attenuated. Particular whole-bacterium antigens for use in the present invention are Bordetella pertussis. In one embodiment, the B. pertussis antigen is the whole-bacterium (Pw antigen), optionally in combination with tetanus toxoid (T) and/or diphtheria toxoid (D). In some embodiments, the adjuvant composition of the present invention comprises or is administered in combination with Pw, tetanus toxoid and diphtheria toxoid (DTPw). Pw antigen may be inactivated by several known methods, including mercury-free methods. Such methods may include heat, formaldehyde, glutaraldehyde, acetone-I, or acetone-IO inactivation (see for example Gupta et al. , 1987, j. Biol. Stand. 15:87; Gupta et al. , 1986, Vaccine, 4:185). Methods of preparing inactivated Pw antigen suitable for use in the formulations of the invention are disclosed in WO 93/24148. In one embodiment of a Pw antigen-comprising adjuvant composition of the invention, the Pw component of the formulation elicits reduced reactogenicity. Reactogenicity of Pw vaccines is primarily caused by lipo-oligosaccharide ('LOS'), which is the endotoxin from the bacterial outer membrane. The lipid A part of the LOS is mainly responsible for the reactogenicity. In order to produce a less reactogenic Pw antigen-containing vaccine (relative to 'traditional' Pw vaccines such as produced by the above-discussed inactivation procedures), the endotoxin can be genetically or chemically detoxified and/or extracted from the outer membrane. In one embodiment, the B. pertussis antigen used in or with the adjuvant composition of the present invention comprises a "low reactogenicity" Pw antigen in which the LOS has been genetically or chemically detoxified and/or extracted. For example, the Pw antigen may be subjected to treatment with a mixture of an organic solvent, such as butanol, and water, as described in WO 06/002502 and Dias et al. (Human Vaccines & Immunotherapeutics, 2012, 9(2):339-348).

In alternative embodiments, 'low reactogenicity' is achieved by deriving the Pw antigen from a B. pertussis strain genetically engineered to produce a less toxic LOS. WO 06/065139 discloses genetic 3-O-deacylation and detoxification of B. pertussis LOS, resulting in strains comprising at least partially 3-O-deacylated LOS. The B. pertussis antigen used in or with the adjuvant composition of the present invention may therefore be a Pw antigen derived from a strain of B. pertussis which has been engineered to express a lipid A-modifying enzyme, such as a de-O-acylase. In particular, such a strain may express the 3-O-deacylase PagL as described in WO 06/065139, as well as in Geurtsen et al. (Infection and Immunity, 2006, 74(10):5574-5585) and Geurtsen et al. (Microbes and Infection, 2007, 9:1096-1103). Alternatively or additionally, the strain from which the Pw antigen is derived may naturally, or as a result of engineering, lacks the ability to modify its lipid A phosphate groups with glucosamine, has a lipid A diglucosamine backbone substituted at the C-3' position with C10-OH or C12-OH and/or express molecular LOS species that lack a terminal heptose. Such a strain, 18-323, is disclosed in Marr et al. (The Journal of Infectious Diseases, 2010, 202(12): 1897-1906).

Further particular bacterial antigens for use in or with the adjuvant composition of the present invention may derive from Streptococcus pneumoniae. At least one streptococcal protein and/or at least one streptococcal capsular saccharide, optionally conjugated to a carrier protein, can be suitably included as antigens in the adjuvant composition of the present invention or can be administered with such formulation. Suitable protein and saccharide antigens derived from Streptococcus pneumoniae are described in WO 14/060385. In some embodiments, the at least one Streptococcus pneumoniae protein is selected from the group consisting of Poly Histidine Triad family (PhtX), Choline Binding Protein Family (CbpX), CbpX truncates, LytX (autolytic enzyme) family, LytX truncates, CbpX truncate-LytX truncate chimeric proteins, PcpA (pneumococcal choline binding protein A), PspA (Pneumococcal Surface Protein A), PsaA (pneumococcal surface adhesion protein A, Sp128 (Streptococcus pneumoniae 128), Sp101(Streptococcus pneumoniae 101), Sp130 (Streptococcus pneumoniae 130), SP125 (Streptococcus pneumoniae 125) and SP133 (Streptococcus pneumoniae 133).

In one embodiments, the adjuvant composition of the present invention comprises or is administered with 1 or more (e.g. 7, 8, 9, 10, 11, 12, 13, 14, 15, 16, 17, 18, 19, 20, 21, 22 or 23) Streptococcus pneumoniae capsular saccharide, optionally conjugated to a carrier protein. In particular embodiments, the 1 or more Streptococcus pneumoniae capsular saccharide, optionally conjugated to a carrier protein, included in or administered with the adjuvant composition of the present invention comprises saccharides derived from serotypes selected from the following serotypes 1, 2, 3, 4, 5, 6A, 6B, 7F, 8, 9N, 9V, 10A, 11A, 12F, 14, 15B, 17F, 18C, 19A, 19F, 20, 22F, 23F and 33F.

The term "saccharide" may indicate a polysaccharide or oligosaccharide and includes both. Polysaccharides are isolated from bacteria and may be sized to some degree by known methods (see, for example, EP0497524 and EP0497525) and optionally by microfluidisation. Polysaccharides can be sized in order to reduce viscosity in polysaccharide samples and/or to improve filterability for conjugated products. The terms "conjugate" relate to a capsular saccharide covalently bonded to a carrier protein. The carrier protein may be any peptide or protein. Suitable carrier proteins are described in WO 14/060385. The carrier protein may be tetanus toxoid (TT), tetanus toxoid fragment C, non-toxic mutants of tetanus toxin, diphtheria toxoid (DT), CRM197, other non-toxic mutants of diphtheria toxin, such as CRM176, CRM228, CRM 45; CRM 9, CRM 45, CRM102, CRM103 and CRM107 (where CRM stands for Cross Reacting Material), pneumococcal pneumolysin, OMPC (outer membrane protein C), heat shock proteins, pertussis proteins, cytokines, lymphokines, growth factors or hormones, artificial proteins comprising multiple human CD4+ T cell epitopes from various pathogen derived antigens, such as N19 protein, pneumococcal surface protein PspA, iron uptake proteins, toxin A or B of C. difficile, H. influenzae Protein, pneumococcal PhtA (poly histidine triad protein A), pneumococcal PhtD (poly histidine triad protein D, pneumococcal PhtB (poly histidine triad protein B), or PhtE (poly histidine triad protein E). In one embodiment the at least one Streptococcus pneumoniae capsular saccharide conjugate is conjugated to a carrier protein selected from the group consisting of tetanus toxoid (TT), fragment C of TT, diphtheria toxoid, CRM197 (cross reacting material 197), detoxified pneumolysin, protein D (from H. influenzae), PhtD, PhtDE and N19. The saccharide may be linked to the carrier protein by any known method. Further particular bacterial antigens for use in the present invention are derived from Neisseria meningitidis. In some embodiments, the antigen of the is a N. meningitidis capsular saccharide from a serogroup selected from the group consisting of: serogroup A (MenA), serogroup C (MenC), serogroup Y (MenY), and serogroup W-135 (MenW), or any combination of two or more thereof, optionally conjugated to a carrier protein. Indeed, these saccharides may suitably be conjugated to any of the carrier protein described above in relation to streptococcal saccharides. In some embodiments, the antigen is a N. meningitidis serogroup A capsular saccharide (MenA), N. meningitidis serogroup C capsular saccharide (MenC), N. meningitidis serogroup Y capsular saccharide (MenY), and N. meningitidis serogroup W-135 capsular saccharide (MenW), optionally conjugated to the carrier protein CRM197 or the carrier protein TT.

Further particular bacterial antigens derived from Neisseria meningitidis for use in the present invention are derived from N. meningitidis serogroup B ("MenB"). Suitable antigens for eliciting anti-MenB responses include polypeptides, lipo-oligosaccharide and/or membrane vesicles. The adjuvant composition of the present invention may include or be administered with one or more serogroup B meningococcal polypeptide antigen(s). In some embodiments, the antigen is a N. Meningitidis serogroup B polypeptide selected from the group consisting of: NadA protein (also known as protein '961'), NHBA protein (also known as protein '287'), fHBP protein (also known as protein '741'), GNA1030 protein (also known as protein '953'), and GNA2091 protein (also known as protein '936'), or any combination of two or more thereof, optionally in combination with a N. meningitidis serogroup B-derived OMV. These antigens will usefully be present as purified polypeptides, e.g. recombinant polypeptides. Suitable forms of these antigens are disclosed in WO 04/032958. The five antigens may be present in the formulation as five separate proteins, or suitably at least two of the antigens are expressed as a single polypeptide chain (a 'hybrid' protein) e.g. such that the five antigens form fewer than five polypeptides, as described in WO 04/032958. In some embodiments, the adjuvant composition of the present invention comprises or is administered with at least NadA protein, NHBA protein, fHBP protein, GNA1030 protein and GNA2091 protein. In particular embodiments, the adjuvant composition of the present invention comprises or is administered with comprise SEQ ID NO:2, and SEQ ID NO:6 as disclosed in WO 04/032958. In further embodiments, the adjuvant composition of the present invention comprises or is administered with an N. meningitidis serogroup B-derived OMV, as described below.

Further particular bacterial antigens are outer membrane vesicles (OMV). These include any proteo-liposomic vesicle obtained by disruption of or blebbling from an outer membrane to form vesicles therefrom that include protein components of the outer membrane. Gram-negative bacteria, such as Neisseria secrete OMV during active growth. The primary immunogenic components of the OMV are the outer membrane proteins (OMPs) and the membrane-bound lipo-polysaccharides (LPS). OMVs may be prepared from any Gram-negative bacterium, including pathogenic Neisserial bacteria such as Neisseria gonorrhoea and Neisseria meningitidis. The OMV approach is particularly useful for Neisseria meningitidis serogroup B, as its polysaccharide capsule is poorly immunogenic. Accordingly, in some embodiments, the adjuvant composition of the present invention comprises or is administered with an OMV derived from a N. meningitidis serogroup B strain, optionally in combination with any of the above-described serogroup B meningococcal polypeptide antigens. OMVs are prepared artificially from bacteria, and may be prepared using detergent treatment (e.g. with deoxycholate), or by non-detergent means, as described in WO 12/020326, for example.

### PARASITES

The antigen used in or administered with the adjuvant composition of the present invention may derive from a parasite. In some embodiments, the antigen may derive from parasites causing Malaria. Accordingly, in some embodiments, the antigen in or administered with the adjuvant composition of the present invention is derived from parasites that cause Malaria, such as for example, Plasmodium falciparum or Plasmodium vivax. Suitably, the Plasmodium falciparum-derived antigen is RTS,S. As disclosed in WO 93/10152, RTS, S is a hybrid protein consisting of the C-terminal portion of the circumsporozoite (CS) protein of Plasmodium falciparum linked via four amino acids of the preS2 portion of Hepatitis B surface antigen to the surface (S) antigen of Hepatitis B virus.

### TUMOR-ASSOCIATED ANTIGENS

The antigen in the adjuvant composition or administered with the adjuvant composition of the present invention may be a tumor-associated antigen. Suitably, the antigen may be a tumor rejection antigen, such as those for prostate, breast, colorectal, lung, pancreatic, renal or melanoma cancers. Exemplary, non-limiting, antigens include MAGE 1, 3 and MAGE 4 or other MAGE antigens, such as disclosed in WO 99/40188.

### ADDITIONAL ANTIGENS

The present invention may involve a plurality of antigenic components, for example with the objective to elicit a broad immune response e.g. to a pathogen or to elicit responses to multiple pathogens. Consequently, more than one antigen may be present. Polysaccharides such as polysaccharide conjugates, may also be present.

### DOSAGES

The therapeutically effective amount will vary depending on, among others, the disease indicated, the severity of the disease, the age and relative health of the subject, the potency of the compound administered, the mode of administration and the treatment desired. In certain embodiments, the daily dosage of a STING agonist of Formula (I), satisfactory results are indicated to be obtained systemically at daily dosages of from about 0.03 to 2.5mg/kg per body weight.

In certain embodiments, the adjuvant composition of the present invention will include a STING agonist of Formula (I) in an amount of between 0.5 to 250 µg per dose. In some embodiment, the adjuvant composition of the present invention will include a STING agonist of Formula (I) in an amount of between 1.0 to 100 µg per dose. In some embodiment, the adjuvant composition of the present invention will include a STING agonist of Formula (I) in an amount of 1.0 µg per dose. In some embodiment, the adjuvant composition of the present invention will include a STING agonist of Formula (I) in an amount of 10.0 µg per dose. In some embodiment, the adjuvant composition of the present invention will include a STING agonist of Formula (I) in an amount of 25.0 µg per dose. In some embodiment, the adjuvant composition of the present invention will include a STING agonist of Formula (I) in an amount of 50.0 µg per dose. In some embodiment, the adjuvant composition of the present invention will include a STING agonist of Formula (I) in an amount of 75.0 µg per dose. In some embodiment, the adjuvant composition of the present invention will include a STING agonist of Formula (I) in an amount of 100.0 µg per dose.

In certain embodiments, the adjuvant composition of the present invention includes aluminium hydroxide in an amount of between 50 to 500 µg per dose. In some embodiment, the adjuvant composition of the present invention includes aluminium hydroxide in an amount of between 100 to 400 µg per dose. In some embodiment, the adjuvant composition of the present invention includes aluminium hydroxide in an amount of 50 µg per dose. In some embodiment, the adjuvant composition of the present invention includes aluminium hydroxide in an amount of 75 µg per dose. In some embodiment, the adjuvant composition of the present invention includes aluminium hydroxide in an amount of 100 µg per dose. In some embodiment, the adjuvant composition of the present invention includes aluminium hydroxide in an amount of 125 µg per dose. In some embodiment, the adjuvant composition of the present invention includes aluminium hydroxide in an amount of 150 µg per dose. In some embodiment, the adjuvant composition of the present invention includes aluminium hydroxide in an amount of 175 µg per dose. In some embodiment, the adjuvant composition of the present invention includes aluminium hydroxide in an amount of 200 µg per dose. In some embodiment, the adjuvant composition of the present invention includes aluminium hydroxide in an amount of 225 µg per dose. In some embodiment, the adjuvant composition of the present invention includes aluminium hydroxide in an amount of 250 µg per dose. In some embodiment, the adjuvant composition of the present invention includes aluminium hydroxide in an amount of 275 µg per dose. In some embodiment, the adjuvant composition of the present invention includes aluminium hydroxide in an amount of 300 µg per dose. In some embodiment, the adjuvant composition of the present invention includes aluminium hydroxide in an amount of 325 µg per dose. In some embodiment, the adjuvant composition of the present invention includes aluminium hydroxide in an amount of 350 µg per dose. In some embodiment, the adjuvant composition of the present invention includes aluminium hydroxide in an amount of 375 µg per dose. In some embodiment, the adjuvant composition of the present invention includes aluminium hydroxide in an amount of 400 µg per dose. In some embodiment, the adjuvant composition of the present invention includes aluminium hydroxide in an amount of 425 µg per dose. In some embodiment, the adjuvant composition of the present invention includes aluminium hydroxide in an amount of 450 µg per dose. In some embodiment, the adjuvant composition of the present invention includes aluminium hydroxide in an amount of 475 µg per dose. In some embodiment, the adjuvant composition of the present invention includes aluminium hydroxide in an amount of 500 µg per dose.

In one embodiment, the adjuvant composition of the present invention includes a STING agonist of Formula (I) (e.g., 3-(((E)-6-carbamoyl-3-((E)-4-((E)-5-carbamoyl-2-((1-ethyl-3-methyl-1H-pyrazole-5-carbonyl)imino)-7-methoxy-2,3-dihydro-1H-benzo[d]imidazol-1-yl)but-2-en-1-yl)-2-((1-ethyl-3-methyl-1H-pyrazole-5-carbonyl)imino)-2,3-dihydro-1H-benzo[d]imidazol-4-yl)oxy)propyl dihydrogen phosphate ) in an amount of between 0.5 to 250 µg per dose and aluminium hydroxide in an amount of 50 to 500 µg per dose. In some embodiment, the adjuvant composition of the present invention will include a STING agonist of Formula (I) 3-(((E)-6-carbamoyl-3-((E)-4-((E)-5-carbamoyl-2-((1-ethyl-3-methyl-1H-pyrazole-5-carbonyl)imino)-7-methoxy-2,3-dihydro-1H-benzo[d]imidazol-1-yl)but-2-en-1-yl)-2-((1-ethyl-3-methyl-1H-pyrazole-5-carbonyl)imino)-2,3-dihydro-1H-benzo[d]imidazol-4-yl)oxy)propyl dihydrogen phosphate in an amount of between 1.0 to 100 µg per dose and aluminium hydroxide in an amount of 50 to 500 µg per dose (e.g. 100-400 µg per dose).

In one embodiment, the adjuvant composition of the present invention will include a STING agonist of Formula (I) 3-(((E)-6-carbamoyl-3-((E)-4-((E)-5-carbamoyl-2-((1-ethyl-3-methyl-1H-pyrazole-5-carbonyl)imino)-7-methoxy-2,3-dihydro-1H-benzo[d]imidazol-1-yl)but-2-en-1-yl)-2-((1-ethyl-3-methyl-1H-pyrazole-5-carbonyl)imino)-2,3-dihydro-1H-benzo[d]imidazol-4-yl)oxy)propyl dihydrogen phosphate in an amount of between 1.0 µg per dose and aluminium hydroxide in an amount of 250 µg per dose. In one embodiment, the adjuvant composition of the present invention will include a STING agonist of Formula (I) 3-(((E)-6-carbamoyl-3-((E)-4-((E)-5-carbamoyl-2-((1-ethyl-3-methyl-1H-pyrazole-5-carbonyl)imino)-7-methoxy-2,3-dihydro-1H-benzo[d]imidazol-1-yl)but-2-en-1-yl)-2-((1-ethyl-3-methyl-1H-pyrazole-5-carbonyl)imino)-2,3-dihydro-1H-benzo[d]imidazol-4-yl)oxy)propyl dihydrogen phosphate in an amount of between 25 µg per dose and aluminium hydroxide in an amount of 250 µg per dose. In one embodiment, the adjuvant composition of the present invention will include a STING agonist of Formula (I) 3-(((E)-6-carbamoyl-3-((E)-4-((E)-5-carbamoyl-2-((1-ethyl-3-methyl-1H-pyrazole-5-carbonyl)imino)-7-methoxy-2,3-dihydro-1H-benzo[d]imidazol-1-yl)but-2-en-1-yl)-2-((1-ethyl-3-methyl-1H-pyrazole-5-carbonyl)imino)-2,3-dihydro-1H-benzo[d]imidazol-4-yl)oxy)propyl dihydrogen phosphate in an amount of between 50 µg per dose and aluminium hydroxide in an amount of 250 µg per dose. In one embodiment, the adjuvant composition of the present invention will include a STING agonist of Formula (I) 3-(((E)-6-carbamoyl-3-((E)-4-((E)-5-carbamoyl-2-((1-ethyl-3-methyl-1H-pyrazole-5-carbonyl)imino)-7-methoxy-2,3-dihydro-1H-benzo[d]imidazol-1-yl)but-2-en-1-yl)-2-((1-ethyl-3-methyl-1H-pyrazole-5-carbonyl)imino)-2,3-dihydro-1H-benzo[d]imidazol-4-yl)oxy)propyl dihydrogen phosphate in an amount of between 75 µg per dose and aluminium hydroxide in an amount of 250 µg per dose. In one embodiment, the adjuvant composition of the present invention will include a STING agonist of Formula (I) 3-(((E)-6-carbamoyl-3-((E)-4-((E)-5-carbamoyl-2-((1-ethyl-3-methyl-1H-pyrazole-5-carbonyl)imino)-7-methoxy-2,3-dihydro-1H-benzo[d]imidazol-1-yl)but-2-en-1-yl)-2-((1-ethyl-3-methyl-1H-pyrazole-5-carbonyl)imino)-2,3-dihydro-1H-benzo[d]imidazol-4-yl)oxy)propyl dihydrogen phosphate in an amount of between 100 µg per dose and aluminium hydroxide in an amount of 250 µg per dose.

### METHODS OF USE

The adjuvant composition of the present invention may be used in therapy (e.g., as a medicament). In one embodiment, the adjuvant composition of the present invention is for use in a method of immunizing a host comprising administering to the host the adjuvant composition and a vaccine composition.

The adjuvant composition of the present invention may be used in conjunction with a vaccine to improve the immunogenicity of the vaccine, or may be used as a vaccine when including an antigen in the adjuvant composition.

In one aspect, the present inventions provides a method of adjuvanting (i.e., improving/enhancing) an immune response in a subject, said method comprising administering the adjuvant composition described herein. The term "enhance" or "enhancing" as used herein means to increase or prolong the potency or duration a desired effect.

Use of an adjuvant composition according to the present invention in the manufacture of a medicament for adjuvanting an immune response in a subject.

The present invention also relates to a combination of the adjuvant composition of described herein, and a vaccine formulation comprising an antigen.

The present invention also provides a kit comprising i) the adjuvant composition as described herein, and an antigen.

The invention also provides a delivery device pre-filled with an adjuvant composition of the present invention. The invention also provides a sterile container (e.g., a vial) containing an adjuvant composition of the present invention (e.g., containing a unit dose or a multiple of unit doses). The invention also provides a hermetically sealed containing a pharmaceutical composition of the present invention.

### EXAMPLES

The invention will now be illustrated by way of the following non-limiting examples. While particular embodiments of the invention are described below a skilled artisan will appreciate that various changes and modifications can be made. References to preparations carried out in a similar manner to, or by the general method of, other preparations, may encompass variations in routine parameters such as time, temperature, work-up conditions, and minor changes in reagent amounts, etc.

### Reference Example: Synthesis of the STING agonist

The STING agonists used in the present invention can be prepared using the methods disclosed in WO 2017/175147 (International patent application number PCT/IB2017/051945), which can be readily adapted to prepare other compounds of the invention by drawing on the knowledge of a skilled organic chemist. For example, (E)-1-((E)-4-((E)-5-carbamoyl-2-((1-ethyl-3-methyl-1H-pyrazole-5-carbonyl)imino)-2,3-dihydro-1H-benzo[d]imidazol-1-yl)but-2-en-1-yl)-2-((1-ethyl-3-methyl-1H-pyrazole-5-carbonyl)imino)-7-(3-hydroxypropoxy)-2,3-dihydro-1H-benzo[d]imidazole-5-carboxamide can be prepared according to Example 10 of WO'147. (E)-1-((E)-4-((E)-5-carbamoyl-2-((1-ethyl-3-methyl-1H-pyrazole-5-carbonyl)imino)-2,3-dihydro-1H-benzo[d]imidazol-1-yl)but-2-en-1-yl)-2-((1-ethyl-3-methyl-1H-pyrazole-5-carbonyl)imino)-7-(3-morpholinopropoxy)-2,3-dihydro-1H-benzo[d]imidazole-5-carboxamide can be prepared according to Example 13 of WO'147. 3-(((E)-6-carbamoyl-3-((E)-4-((E)-5-carbamoyl-2-((1-ethyl-3-methyl-1H-pyrazole-5-carbonyl)imino)-7-methoxy-2,3-dihydro-1H-benzo[d]imidazol-1-yl)but-2-en-1-yl)-2-((1-ethyl-3-methyl-1H-pyrazole-5-carbonyl)imino)-2,3-dihydro-1H-benzo[d]imidazol-4-yl)oxy)propyl dihydrogen phosphate can be prepared according to Example 19 of WO'147. (E)-1-(4-(5-carbamoyl-2-(1-ethyl-3-methyl-1H-pyrazole-5-carboxamido)-7-methoxy-1H-benzo[d]imidazol-1-yl)but-2-en-1-yl)-7-(3-(dimethylamino)propoxy)-2-(1-ethyl-3-methyl-1H-pyrazole-5-carboxamido)-1H-benzo[d]imidazole-5-carboxamide can be prepared according to Example 39 of WO'147. (E)-1-((E)-4-((E)-5-carbamoyl-2-((1-ethyl-3-methyl-1H-pyrazole-5-carbonyl)imino)-7-(3-(4-(2-hydroxyethyl)piperazin-1-yl)propoxy)-2,3-dihydro-1H-benzo[d]imidazol-1-yl)but-2-en-1-yl)-2-((1-ethyl-3-methyl-1H-pyrazole-5-carbonyl)imino)-7-methoxy-2,3-dihydro-1H-benzo[d]imidazole-5-carboxamide can be prepared according to Example 43 of WO'147. (E)-4-((5-carbamoyl-1-(4-(5-carbamoyl-2-(1-ethyl-3-methyl-1H-pyrazole-5-carboxamido)-1H-benzo[d]imidazol-1-yl)but-2-en-1-yl)-2-(1-ethyl-3-methyl-1H-pyrazole-5-carboxamido)-1H-benzo[d]imidazol-7-yl)oxy)butanoic acid can be prepared according to Example 52 of WO'147.

### Example 1: Pre-formulation studies

1.1 An exemplified immunogenic composition of the invention has been tested to assess (i) the compatibility between the antigen and the STING agonist and (ii) the adsorption of the antigen and the STING agonist on alum, respectively. The HSV2 gE-gl heterodimer tested herein consisted of the HSV2 gE having the amino acid sequence shown in SEQ ID NO: 5 (ectodomain) associated in a non-covalent complex with the HSV2 gl having the amino acid sequence shown in SEQ ID NO: 6 (ectodomain). The alum used was aluminium hydroxide (Al(OH)₃). The STING agonist used was 3-(((E)-6-carbamoyl-3-((E)-4-((E)-5-carbamoyl-2-((1-ethyl-3-methyl-1H-pyrazole-5-carbonyl)imino)-7-methoxy-2,3-dihydro-1H-benzo[d]imidazol-1-yl)but-2-en-1-yl)-2-((1-ethyl-3-methyl-1H-pyrazole-5-carbonyl)imino)-2,3-dihydro-1H-benzo[d]imidazol-4-yl)oxy)propyl dihydrogen phosphate (referred to as Compound 1 hereafter):

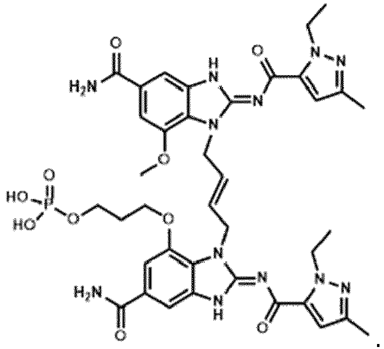

**Table 1**

| Groups | Components | Component concentration in the composition |
|---|---|---|
| 1 | HSV-2 gE-gI | - |
| 2 | HSV-2 gE-gI | 45 µg/ml STING agonist |
| 3 | | 15 µg/ml STING agonist |
| 4 | STING agonist (aqueous) | 5 µg/ml STING agonist |
| 5 | HSV-2 gE-gI | 45 µg/ml STING agonist |
| | | 111 µg/ml Al(OH)₃ |
| 6 | STING agonist - Al(OH)₃ | 15 µg/ml STING agonist |
| | | 111 µg/ml Al(OH)₃ |
| 7 | | 5 µg/ml STING agonist 111 µg/ml Al(OH)₃ |
| 8 | HSV-2 gE-gI | 15 µg/ml STING agonist |
| | STING agonist-Al(OH)₃ | 35 µg/ml Al(OH)₃ |

The compatibility between the antigen and the STING agonist was assessed, on the one hand, by determining the amount of the STING agonist recovered (using HPLC) in the supernatant after centrifugation of the formulations described in the above groups 2, 3and 4, and, on the other hand, by determining the amount and profile of the antigen recovered (using HPLC) in the supernatant after centrifugation of the formulations described in the above groups 2, 3 and 4. The adsorption of the STING agonist and the antigen on Al(OH)₃ was similarly assessed by determining the amount of STING agonist and amount and profile of the antigen, respectively, recovered) in the supernatant after centrifugation of the formulations described in the above groups 5, 6, 7 and 8. The concentration of the HSV-2 gE-gI antigen was 20 µg/mL in each group. The precise methodology for preparing the STING agonist and Al(OH)₃ formulation was as follows: a solution of Al(OH)₃ in water for injection was prepared, the required amount of STING agonist was added, the required amount of HSV-2 gE-gI to have 20 µg/mL in final solution was added. The solution was then mixed for 30 minutes at room temperature. NaCl was added, and the mixture was mixed for 5 minutes.

### Results

Considering the groups 2, 3 and 4 (STING agonist in aqueous formulation, with no added Al(OH)₃), the same amount of STING agonist was found in the non-centrifuged samples and in the supernatant of centrifuges samples indicating that no aggregation or precipitation occurs in the presence of the antigen. Likewise, the same profile of gE-gI was observed by both SDS PAGE and SEC HPLC on non-centrifuged samples and in the supernatant of centrifuges samples, indicating that no aggregation or precipitation occurs in the presence of the STING agonist.

Considering the groups 5, 6, 7 and 8 (STING agonist + Al(OH)₃), after centrifugation of the samples, no STING agonist was found in the supernatant, indicating that the entire amount of the STING agonist was adsorbed on Al(OH)₃ in the presence of the antigen. Likewise, for the groups 5, 6 and 7, after centrifugation of the samples, no antigen was detected in the supernatant, indicating that the entire amount of the antigen was adsorbed on Al(OH)₃ in the presence of the STING agonist. Only the group with a lower amount of Al(OH)₃ (group 8) showed a reduced adsorption (with 30% of the antigen being adsorbed).

1.2 Next, the minimal amount of Al(OH)₃ needed to fully adsorb the STING agonist was determined. Compound 1 was used. The amount of the STING agonist was fixed at 45 µg/ml and AL(OH)₃ varied from 0 to 200 µg/ml (by reference to Al³⁺), so that different ratios STING agonist:Al(OH)₃ were tested. The different formulations (as indicated in Table 2) were mixed under agitation for 1h. Samples were then centrifuged for 10 min at 8000 rpm. The supernatant of the centrifuged samples was then analysed to characterize the amount of STING agonist which was not adsorbed. The quantification was performed by fluorometry. Results are presented in Fig. 1.

**Table 2**

| STING agonist concentration (µg/ml) | Al³⁺ concentration (µg/ml) | STING agonist/Al³⁺ ratio |
|---|---|---|
| 45 | 200 | 0.225 |
| 45 | 150 | 0.3 |
| 45 | 100 | 0.45 |
| 45 | 75 | 0.6 |
| 45 | 60 | 0.75 |
| 45 | 50 | 0.9 |
| 45 | 40 | 1.125 |
| 45 | 30 | 1.5 |
| 45 | 20 | 2.25 |
| 45 | 10 | 4.5 |
| 45 | 50 | 9 |
| 45 | 0 | - |

### Results

It was demonstrated that the STING agonist is generally well adsorbed on Al(OH)₃. This study shows that the maximum ratio STING agonist to aluminium hydroxide allowing for an optimal adsorption is about 1.5. Ratios between 0.45 and 1.5 allow a quasi-complete adsorption. This indicates that, even at a higher concentration, a very low dose of Al(OH)₃ may be used (lower than what is usually found in alum-based vaccines).

### Example 2: Evaluation of the immunogenicity of a recombinant HSV-2 gE-gI antigen model adiuvanted with STING agonist and aluminium hydroxide

The same HSV-2 gE-gI antigen recombinantly expressed was formulated with different doses of either a soluble form of STING agonist alone or formulated with AI(OH)3. Total HSV-2 gE and gI IgG titers, Total HSV-1 gE-gI IgG titers, HSV-2 gE and gI CD4+ T cell responses, HSV-1 gE and gI CD4+ T cell responses, and antibody functionality were looked at, and compared with the same HSV-2 gE-gI antigen adjuvanted with AS01 (a liposome-based adjuvant comprising a saponin and a TLR4 agonist).

### Study design:

In this study, 9 groups of naïve female CB6F1 mice (n=8 per group, except group 1 (control) n=4), 6 to 8 weeks old at study start were injected intra-muscularly (IM) at 2 weeks interval in left gastrocnemius muscle with 50µL of either non-adjuvanted HSV-2 gE-gI protein, or different doses of STING agonist-adjuvanted gE-gI or different doses of STING agonist/Al(OH)₃ formulations with HSV-2 gE-gI or AS01-adjuvanted HSV-2 gE-gI.

Serum samples were collected 14 days (Gp1-Gp9) post one and two immunizations to evaluate the total anti- HSV-2 gE & gI antibody response. Cross-reactive HSV-1 gE-gI specific antibody responses and the antibody function (Competitive ELISA and mFcyRIV binding activity/ADCC like assay) were only assessed after two immunizations. Spleen were collected 14 days (Gp1-Gp9) after two immunizations to assess the frequencies of anti-HSV-2 gE & gI-specific CD4+/CD8+ T cells expressing IL-2, TNF-α IFN-γ , IL-13 and/or IL-17 cytokines. A summary of the study design and formulation is shown in the below table. Compound 1 was used as the STING agonist.

**Table 3: Study design**

| **Gp** | **Vaccine** | **Adjuvant dose/per animal** | | **Antigen dose**/ **Per animal** | **Sample collection and days** | **No. of mice** |
|---|---|---|---|---|---|---|
| 1 | HSV-2 gE-gI alone | - | | 1µg | **Sera** at 14dPI*and 14dpII** | 4 |
| 2 | HSV-2 gE-gI /STING agonist aqueous | 2.22µg STING agonist | | 1µg | | 8 |
| 3 | | 0.74µg STING agonist | | 1µg | | 8 |
| 4 | | 1.5µg STING agonist | | 1µg | | 8 |
| 5 | HSV-2 gE-gI /STING agonist/Al(OH)₃ | 2.22µg STING agonist | 5.55µg Al(OH)₃ | 1µg | **Spleen** at 14dPII** | 8 |
| 6 | | 0.74µg STING agonist | 5.55µg Al(OH)₃ | 1µg | | 8 |
| 7 | | 1.5µg STING agonist | 5.55µg Al(OH)₃ | 1µg | | 8 |
| 8 | | 0.74µg STING agonist | 1.85µg Al(OH)₃ | 1µg | | 8 |
| 9 | HSV-2 gE-gI /AS01 | 5µg AS01*** | | 1µg | | 8 |

| | | | | | | |
|---|---|---|---|---|---|---|
| * "PI" = Post-first immunization / "14dPI" means 14 days post-first immunization ** "PII" = Post-second immunization / "14dpII" means 14 days post-second immunization *** 5µg AS01 refers to a liposomal formulation containing 5µg MPL and 5µg QS_21 | | | | | | |

### 2.1 Detection of total anti-HSV-2 gE and gI IgG antibodies bv ELISA

Quantification of the total HSV-2 gE or gI-specific IgG antibodies was performed using indirect ELISA. Recombinant HSV-2 gE (∼51kDa) (BMP1291) or HSV-2 gI proteins (~46kDa) (BMP1292) were used as coating antigens. These proteins were produced using the ExpiHEK293FTM expression system.

Polystyrene 96-well ELISA plate (Nunc F96 Maxisorp cat 439454) were coated with 100µL/well of antigen diluted at a concentration of 2 µg/mL (HSV-2 gE) and 1 µg/mL (HSV-2 gI) in carbonate/bicarbonate 50mM pH 9.5 buffer and incubated overnight at 4°C. After incubation, the coating solution was removed and the plates were blocked with 200µL/well of Difkomilk 10% diluted in PBS (blocking buffer) (Becton Dickinson, USA) for 1 h at 37°C. The blocking solution was removed and a three-fold (sera 14dPI & 14dPII) sera dilutions (in PBS + 0.1% Tween20 + 1% BSA buffer) were added to the coated plates and incubated for 1h at 37°C. The plates were washed four times with PBS 0.1% Tween20 (washing buffer) and Peroxydase conjugated AffiniPure Goat anti-mouse IgG (H+L) (Jackson, USA) was used as a secondary antibody. One hundred microliters per well of the secondary antibody diluted at a concentration of 1:500 in PBS + 0.1% Tween20 + 1% BSA buffer was added to each well and the plates were incubated for 45min at 37°C.The plates were then washed four times with washing buffer and 2 times with deionised water and incubated for 10min at RT (room temperature) with 100 µL/well of a solution of 75% single-component TMB Peroxidase ELISA Substrate (Bio-Rad, USA) diluted in sodium Citrate 0.1M pH5.5 buffer. Enzymatic color development was stopped with 100µL of 0,4N Sulfuric Acid (H₂SO₄) per well and the plates were read at an absorbance of 450/620nm using the Versamax ELISA reader.

Optical densities (OD) were captured and analysed using the SoftMaxPro GxP v5.3 software. A standard curve was generated by applying a 4-parameter logistic regression fit to the reference standard results (reference standard anti-HSV-2 gE = 14PIII - Pool of mice 1.1 to 1.20 immunized with 5µg of HSV-2 gE/AS01 /dose; reference standard anti-HSV-2 gI = 14PII - Pool of mice 2.1 to 2.10. immunized with 5µg of HSV-2 gI/AS01/dose). Antibody titer in the samples was calculated by interpolation of the standard curve. The antibody titer of the samples was obtained by averaging the values from dilutions that fell within the 20-80% dynamic range of the standard curve. ELISA titers were normalized at the same starting dilution to allow titers comparisons.

### 2.2 Detection of the total anti-HSV-1 gE-gI-specific IgG antibodies bv ELISA

The total anti-HSV-1 gE-gI-specific IgG antibodies was assessed using indirect ELISA. Recombinant gE-gI heterodimer protein (BMP1299) from HSV-1 were used as coating antigen. This protein was produced using the ExpiCHO^{™} expression system.

The rest of procedure was the same as for the quantification of total HSV-2 gE or gI-specific IgG antibodies previously described, except that the polystyrene 96-well ELISA plate were coated with 100µL/well of recombinant HSV-1 gE-gI heterodimer.

### Results

All vaccine formulations were immunogenic as antibody responses were significantly higher than the one observed with the antigen alone (see Fig. 2, Fig. 3 and Fig. 4). A clear boost in the antibody response was observed between the first and second dose of the vaccine. No clear dose effect was observed with soluble STINGa or STINGa/Al(OH)₃. Moreover, no significant difference in terms of HSV-2 gE and HSV-2 gI antibody titers was observed between the STINGa/Al(OH)₃ ratios tested.

The comparison between the two STINGa-based vaccines after 2 immunizations suggests that STINGa/Al(OH)₃ formulation induced similar or higher HSV-2 gE specific IgG antibody titers than the soluble STINGa formulation (Fig. 3). For HSV-2 gI response, higher antibody titers were observed with STINGa/Al(OH)₃ formulations, as compared with the soluble STINGa formulations, with a fold-increase ranging from 3.3 to 4 (Fig. 2).

STINGa/Al(OH)₃ formulations tended to induce higher HSV-2 gE specific IgG titers in comparison to AS01 (Fig. 3). In terms of HSV-2 gI specific titers, STINGa formulations induced similar responses as the AS01 formulation (Fig. 2).

Similar observations were made for the anti-HSV-1 gE-gI cross-reactive IgG antibody response in terms of dose range (comparison within the two STINGa formulations and comparison with AS01 (see Fig. 4).

### 2.3 Evaluation of anti-HSV-2 and HSV-1 gE and gI CD4+ T cell responses by Intracellular cytokine staining (ICS)

The frequencies of vaccine-specific CD4+ producing IL-2 and/or IFN-γ and/or TNF-α and/or IL-13 and/or IL-17 were evaluated in splenocytes collected 14 days after second immunization after *ex-vivo* stimulation with HSV-2 gE or gI peptides pools or with HSV-1 gE or gI peptides pools.

**Isolation of splenocytes:** Spleens were collected from individual mouse 14 days after second immunization and placed in RPMI 1640 medium supplemented with RPMI additives (Glutamine, Penicillin/streptomycin, Sodium Pyruvate, non-essential amino-acids & 2- mercaptoethanol) (= RPMI/additives). Cell suspensions were prepared from each spleen using a tissue grinder. The splenic cell suspensions were filtered (cell stainer 100µm) and then the filter was rinsed with 35mL of cold RPMI/additives. After centrifugation (335g, 10min at 4°C), cells were resuspended in 5mL of cold RPMI/additives. Splenic cell suspensions were again filtered (cell stainer 100µm) before a second washing step was performed, as previously described, and the cells were finally resuspended in 2mL of RPMI/additives supplemented with 5% FCS. Cell suspensions were then diluted 20× (10µL) in PBS buffer (190µL) for cell counting (using MACSQuant Analyzer). After counting, cells were centrifuged (335 g, 10 min at RT) and resuspended at 10⁷ cells/mL in RPMI/additives supplemented with 5% FCS.

**Cell preparation:** Fresh splenocytes were seeded in round bottom 96-well plates at 10⁶ cells/well (100µL). The cells were then stimulated for 6 hours (37°C, 5% CO₂) with anti-CD28 (clone 37.51) and anti-CD49d antibodies (clone 9C10 (MFR4.B) at 1µg/mL per well, containing 100µL of either:
- 15 mers overlapping peptides pool covering the sequences of gE protein from HSV-2 (1µg/mL per peptide per well).
- 15 mers overlapping peptides pool covering the sequences of gI protein from HSV-2 (1µg/mL per peptide per well).
- 15 mers overlapping peptides pool covering the sequences of gE protein from HSV-1 (1µg/mL per peptide per well).
- 15 mers overlapping peptides pool covering the sequences of gI protein from HSV-1 (1µg/mL per peptide per well).
- 15 mers overlapping peptides pool covering the sequences of Human β-actin protein (1µg/mL per peptide per well) (irrelevant stimulation).
- RPMI/additives medium (as negative control of the assay).
- PMA - ionomycin solution at working concentrations of 0.25 µg/mL and 2.5 µg/mL respectively (as positive control of the assay).

After 2 hours of *ex vivo* stimulation, Brefeldin A (Golgi plug,BD Bioscience) diluted 1/200 in RPMI/additives supplemented with 5% FCS and Monensin (BD GolgiStop, BD Bioscience) diluted 1/300 in RPMI/additives supplemented with 5% FCS was added for 4 additional hours to inhibit cytokine secretion. Plates were then transferred at 4°C for overnight incubation.

**Intracellular Cytokine Staining:** After overnight incubation at 4°C, cells were transferred to V-bottom 96-well plates, centrifuged (189g, 5min at 4°C) and washed with 250µL of cold PBS +1% FCS (Flow buffer). After a second centrifugation (189g, 5min at 4°C), cells were resuspended to block unspecific antibody binding (10 min at 4°C) in 50µL of Flow buffer containing anti-CD16/32 antibodies (clone 2.4G2) diluted 1/50. Then, 50 µL Flow Buffer containing mouse anti-CD4-A700 antibodies (clone RM4-5, diluted at 1/100), and Live/Dead^{™} Fixable near-IR dead cell stain (diluted at 1/500) was added for 30min in obscurity at 4°C. After incubation, 100µL of Flow buffer was added into each well and cells were then centrifuged (189g for 5 min at 4°C). A second washing step was performed with 200µL of Flow buffer and after centrifugation, cells were fixed and permeabilized by adding 200µL of Cytofix-Cytoperm solution for 20min at 4°C in the obscurity. After plates centrifugation (500g for 5 min at 4°C), cells were washed with 200µL of Perm/Wash buffer, centrifuged (500g for 5 min 4°C) and resuspended in 50µL of Perm/Wash buffer containing mouse anti-IL2-FITC (clone JES6-5H4, diluted 1/400), anti- IFN-γ-APC (clone XMG1.2, diluted 1/200) and anti-TNF-α-PE (clone MP6-XT22, diluted 1/700) and anti-IL-13 PeCy7 (clone ebio13A ,diluted 1/50) and anti-IL-17 BV605 (clone TC11-18H10 , diluted 1/100) antibodies, for 1 hour at 4°C in the obscurity. After incubation, 100µL of Perm/wash buffer was added into each well and cells were then finally washed with 200µL of Perm/Wash buffer (centrifugation 500g for 5 min à 4°C) and resuspended in 220µL PBS.

**Cell acquisition and analysis** Stained cells were acquired by flow cytometry and analyzed using the FlowJo software. Live cells were identified with the Live/Dead staining and then lymphocytes were isolated based on Forward/Side Scatter lights (FSC/SSC) gating. The acquisition was performed on ∼ 20.000 CD4+T-cell events. The percentages of IFN-γ^{+/-} IL-2^{+/-} TNF-α^{+/-} IL-13^{+/-} and IL-17^{+/-} producing cells were calculated on CD4+ and cell populations. For each sample, unspecific signal detected after medium stimulation was removed from the specific signal detected after peptide pool stimulation.

### Results

Individual frequencies of CD4+ T cell expressing at least one cytokine among IL-2, TFN-α, IFN-γ, IL-13 or IL-17 observed after two injections of HSV-2 gE-gI adjuvanted with soluble STINGa, STINGa/Al(OH)₃, and AS01 are presented in Fig. 5.

While for soluble STINGa there is no dose dependency, an inverse dose range was observed with the STINGa/Al(OH)₃ formulations, with the higher CD4+ T cell responses observed with the lower STINGa dose (0.74 µg). In addition, no significant difference in terms of HSV-2 gE and gI CD4+ specific T cells can be observed between the two Al(OH)₃ ratios (Fig. 5 and Fig. 6, respectively).

When compared to AS01, HSV-2 gE-gI adjuvanted with STINGa (0.74 µg)/Al(OH)₃ (5.55µg) also showed significantly higher HSV-2 gE and gI specific CD4+ T cell responses (Fig. 5 and Fig. 6, respectively). However, no difference was observed between the soluble STINGa formulation and AS01 for any of the tested antigens.

Finally, similar observations were made for the anti-HSV-1 gI and gE cross-reactive CD4+ T cell responses in terms of dose range (comparison within the two STINGa formulations and comparison with AS01) (Fig. 7 and Fig. 8, respectively).

### 2.4 Evaluation of CD4+ T cell polvfunctionalitv profile

T-cell polyfunctionality was next looked at. The capacity of CD4+ T cells to produce one or multiple cytokines was assessed, and the T cell profile was compared between the different formulations. As the 0.74 µg STINGa dose induced the highest level of CD4+ T cells response, this dose was used for the comparison. Proportions of HSV-2 gE -specific CD4+ T cells expressing 1, 2, 3, or 4 cytokines after in vitro stimulation were determined and represented in Fig. 9. Pie charts represent the mean proportions of cells expressing single markers and any combination of IFN-γ, IL-2, TNF-α, IL-13 and IL-17 marker-positive CD4+ T cells out of the total HSV-2 specific CD4+ T cells.

### Results

It was observed that the profile obtained with AS01 and the soluble STINGa formulation were similar with a dominance of double positive cells. In contrast, with STINGa/Al(OH)₃, the proportion of cells expressing 3 cytokines was higher suggesting an increased CD4+ T cell polyfunctionality with STINGa'456/Al(OH)₃ as compared with soluble STINGa formulations and AS01. Similar results were obtained for HSV-2 gI (Fig. 10).

### Example 3: Evaluation of the innate immune response induced by a STINGa adiuvanted vaccine

The kinetic and the intensity of the systemic innate immune response induced by the STINGa formulations (soluble and alum-formulated) were also assessed to evaluate if STINGa/Al(OH)₃ formulations reduce the potential systemic effect. The recombinant gE protein of VZV (SEQ ID No. 7) was used as the antigen. Those data were compared with an AS01 formulation. The STINGa was Compound 1.

### Study design

4 groups of Female 6-8 week old C57BL/6JOIaHsd Mice were injected intra-muscularly (IM) in left gastrocnemius on days 0 with 50 µl/site containing 5 µg VZV gE only (negative control group), 5 µg VZV gE formulated either with soluble STINGa, with STINGa/Al(OH)₃ or with AS01 (positive control group). To assess the kinetic of the innate response induced by the different vaccine formulations, sera were taken at different time points (3h, 6h, 24h and 48h) post-immunization (n= 5 mice/group/time point). For the AS01 group (Gp 1), the responses were evaluated at 6h only, which corresponds to the peak of the response. To define a baseline for each cytokine measured, a bleeding was performed on 30 mice prior to vaccination and 5 pools of 6 mice were realized.

### Results

Innate cytokine responses induced by the two STINGa formulations (soluble and alum-formulated) peak at 6h for the majority of innate markers tested and returns to baseline within 24h or 48h (IL-6, IP-10 and IFN-γ in Fig. 11). For Type 1 IFNs (IFN-a and IFN-b), the peak was at 3h (Fig. 12).

The adsorption of STINGa on Al(OH)₃ decreases the innate cytokine response in serum with respect to the ones observed with the soluble STINGa formulation. This effect was mainly observed for IFN-a and IFN-b (Fig. 12) and IL-6 and IFN-γ (Fig. 11).

As compared with AS01 (6h), both STINGa formulations induced the following:
▪ High level of Type 1 IFNs (IFN-a and IFN-b) which were not induced by AS01 (STINGa specificity) (Fig. 12).
▪ Lower level of IFN-γ and IL-6 and (Fig. 11).
▪ Similar or slightly higher level of IP-10 (Fig. 11).

Altogether, these results suggest that the adsorption of STINGa on Al(OH)₃ decreases the systemic cytokine response induced by the STINGa. Both STINGa formulations, as compared with AS01, induce lower levels of markers known to be associated with systemic reactogenicity suggesting an acceptable tolerogenic profile for the two STINGa formulations.

### Overall Results

Altogether, the results showed a good immunogenicity of the recombinant HSV-2 gE-gI protein adjuvanted with different doses of STINGa (soluble or alum-formulated) after one and/or two injections, and an acceptable tolerogenic profile.

A benefit of the STINGa/Al(OH)₃ formulation was demonstrated, inducing higher functional antibodies and a higher CD4+ T cell response than both AS01 and soluble STINGa formulations. Moreover, STINGa/Al(OH)₃ formulations induced CD4+ T cells with a higher polyfunctionality, as compared with AS01 and soluble STINGa formulations.

No difference was observed between the two Al(OH)₃ ratios tested in terms of total IgG, functional antibodies or CD4+ T cells.

These results demonstrate that the adjuvant composition of the present invention has excellent immunogenicity when combined with an antigen, and presents some superiority in comparison to both soluble STINGa formulations and alternative adjuvants, such as AS01.

### Embodiments of the invention:

The present invention relates to the following embodiments:
1. An adjuvant composition comprising:
   (iii) a STING agonist of Formula (I) or a pharmaceutically acceptable salt thereof 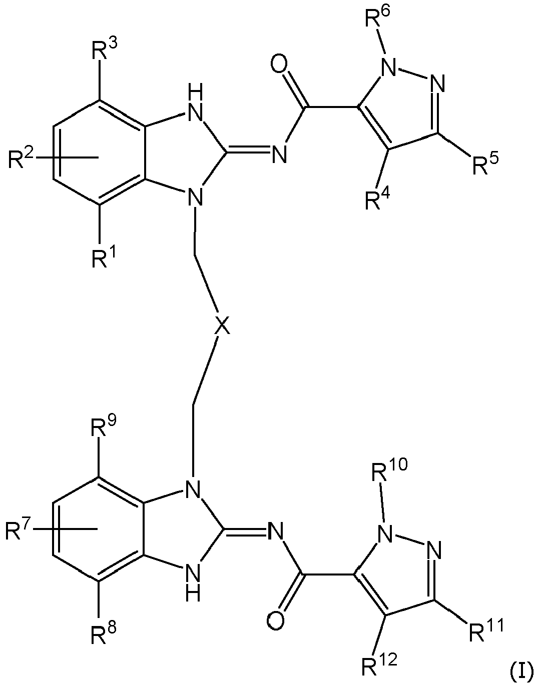 wherein
      X is ―halo(C₁-C₅)alkyl, unsubstituted ―C₁-C₅ alkyl, or unsubstituted ―C₂-C₅ alkenyl;
      R¹ and R⁹ are independently H, halogen, hydroxyl, ―O―P(O)(OH)₂, ―O―P(O)(R^{I}R^{II})₂, optionally substituted C₁-C₆ alkyl or optionally substituted C₁-C₆ alkyloxy,
         wherein optionally substituted means substituted with 1-4 substituents each independently selected from the group consisting of hydroxyl, ―O―P(O)(OH)₂, ―O-P(O)(R^{I}R^{II})₂, C₁-C₄ alkoxyl, -N(R^{A})₂, -CO₂(R^{B}), optionally substituted phenyl, and optionally substituted 5-6 membered heterocycloalkyl, wherein said optionally substituted phenyl, or optionally substituted 5-6 membered heterocycloalkyl is optionally substituted by 1-4 substituents each independently selected from halogen, hydroxy, ―O―P(O)(OH)₂, ―O― P(O)(R^{I}R^{II})₂, amino, (C₁-C₆ alkyl)amino-, (C₁-C₆ alkyl)(C₁-C₆ alkyl)amino―, halo(C₁-C₆ alkyl), hydroxy-(C₁-C₄ alkyl)―, ―(C₁-C₄ alkyl)-O-P(O)(OH)₂, -(C₁-C₄alkyl)-O-P(O)(R^{I}R^{II})₂, halo(C₁-C₄ alkoxy)―, C₁-C₄ alkoxy―, hydroxy―(C₂-C₄ alkoxy)―, ―(C₂-C₄ alkoxy)―O―P(O)(OH)₂, ―(C₂-C₄ alkoxy)-O-P(O)(R^{I}R^{II})₂, -(C₁-C₆ alkyl)-NH₂, -C1-C4 alkyl―(C₁-C₄ alkoxyl) and C1-C4 alkoxy-(C₁-C₄ alkoxy)-, wherein R^{A} and R^{B} are each independently selected from hydrogen, ―C₁-C₄ alkyl, ―CO(C₁-C₄ alkyl), ―OCO(C₁-C₄ alkyl), ―(C₁-C₄ alkyl)-NH2, ―(C₁-C₄ alkyl)-C1-C4 alkoxyl, or ―CO₂(C₁-C₄ alkyl),
      R² and R⁷ are each independently hydrogen, ―CON(R^{C})₂, -COOH, or CO₂(R^{D}), or one of R² and R⁷ is ―CON(R^{C})(R^{D}) and the other is H, -COOH, or CO₂(R^{E}), wherein R^{C} and R^{D} are each independently selected from hydrogen, ―C₁-C₄ alkyl, ―CO(C₁-C₄ alkyl),-OCO(C₁-C₄ alkyl), ―(C₁-C₄ alkyl)-NH2, ―(C₁-C₄ alkyl)-C₁-C₄ alkoxyl, or ―CO₂(C₁-C₄ alkyl); R³ and R⁸ are each independently H, halo(C₁-C₆alkyl), halo(C₁-C₆alkoxy)-, hydroxy, -O-P(O)(OH)₂, -O-P(O)(R^{I}R^{II})₂, -NR^{c}R^{d}, -COR^{c}, -CO₂R^{c}, -N(R^{d})COR^{c}, -N(R^{d})S O₂R^{c}, -N(R^{g})SO₂(C₁-C₂alkyl)-N(R^{h})(R^{f}), -N(R^{g})CO(C₁-C₂alkyl)-N(R^{h})(R^{f});
      R^{e}, R^{f}, R^{g}, and R^{h} are each independently H or C1-C4 alkyl;
      R⁴, R⁵, R¹¹ and R¹² are each independently H or C1-C4 alkyl;
      R⁶ and R¹⁰ are each C₁-C₄ alkyl; and
      each occurrence of R^{I} and R^{II} is independently C1-C6 alkyloxy-; and
   (iv) aluminium hydroxide, aluminium phosphate, aluminium oxyhydroxide, or aluminium hydroxyphosphate.
2. The adjuvant composition according to embodiment 1, wherein the STING agonist has the structure of Formula (II) or a pharmaceutically acceptable salt thereof 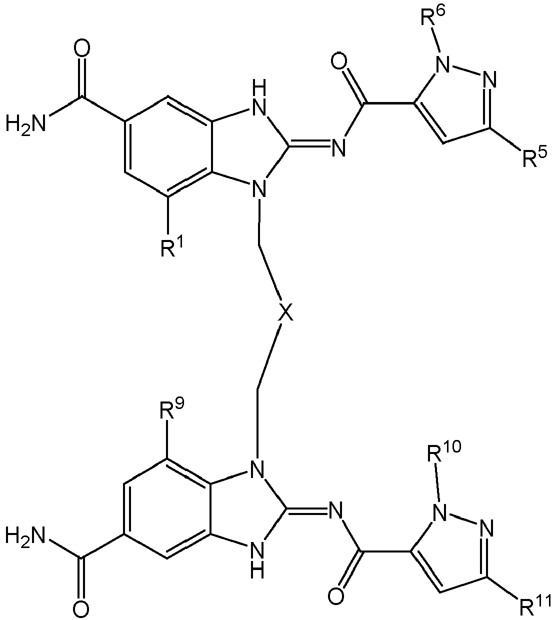 wherein X, R¹, R⁵, R⁶, R⁹, R¹⁰ and R¹¹ are as defined in embodiment 1.
3. The adjuvant composition according to embodiment 1 or 2, wherein R¹ and R⁹ are each independently H, halogen, optionally substituted (C₁-C₆alkyl), or optionally substituted (C₁-C₆alkyl)oxy-, and the C₁-C₆alkyl of said optionally substituted (C₁-C₆alkyl), optionally substituted (C₁-C₆alkyl)oxy- is optionally substituted with 1-4 substituents each independently selected from the group consisting of hydroxyl,―O-P(O)(OH)₂, ―O-P(O)(R^{I}R^{II})₂, -N(R^{e})(R^{f}), C₁-C₄alkoxyl, phenyl, optionally substituted 5-6 membered heterocycloalkyl containing at least one nitrogen or oxygen as a member of the ring, each R^{e} is independently selected from H, (C₁-C₄alkyl), -(C₁-C₄alkyl)-NH₂, or -(C₁-C₄alkyl) C₁-C₄alkoxy and each R^{f} is independently H or (C₁-C₄alkyl).
4. The adjuvant composition according to embodiment 1 or 2, wherein one of R¹ and R⁹ is ―O― P(O)(OH)₂, ―O-P(O)(R^{I}R^{II})₂, C₁-C₆ alkyl substituted with ―O―P(O)(OH)₂ or ―O-P(O)(R^{I}R^{II})₂, or a C₁-C₆ alkyloxy group.
5. The adjuvant composition according to any one of embodiments 1 to 4, wherein R³ and/or R⁹ is H.
6. The adjuvant composition according to any one of embodiments 1 to 5, wherein R⁴ and/or R¹³ is H.
7. The adjuvant composition according to any one of embodiments 1 to 6, wherein R⁶ and/or R¹⁰ is ethyl.
8. The adjuvant composition according to any one of embodiments 1 to 7, wherein at least one of R¹ and R⁹ is selected from the following groups: 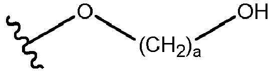 where a is a number from 1 to 6; 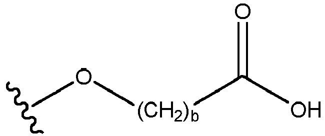 where b is a number from 1 to 6; 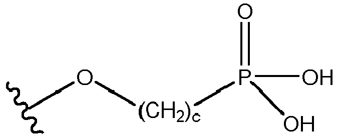 where c is a number from 1 to 6; 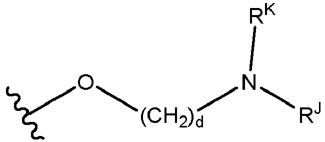 where d is a number from 1 to 6, and R^{J} and R^{K} are C1-C3 alkyl; or 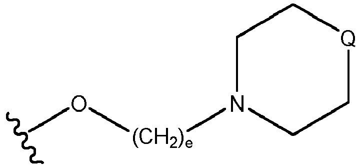 where e is a number from 1 to 6, and Q is selected from O or N(R^{X}) wherein R^{X} is a C1-C6 alkyl or C1-C6 alkoxy group.
9. The adjuvant composition according to any one of embodiments 1 to 8 wherein the STING agonist is selected from the group consisting of:
   (E)-1-((E)-4-((E)-5-carbamoyl-2-((1-ethyl-3-methyl-1H-pyrazole-5-carbonyl)imino)-2,3-dihydro-1H-benzo[d]imidazol-1-yl)but-2-en-1-yl)-2-((1-ethyl-3-methyl-1H-pyrazole-5-carbonyl)imino)-7-(3-hydroxypropoxy)-2,3-dihydro-1H-benzo[d]imidazole-5-carboxamide;
   4-(((E)-6-carbamoyl-3-((E)-4-((E)-5-carbamoyl-2-((1-ethyl-3-methyl-1H-pyrazole-5-carbonyl)imino)-2,3-dihydro-1H-benzo[d]imidazol-1-yl)but-2-en-1-yl)-2-((1-ethyl-3-methyl-1H-pyrazole-5-carbonyl)imino)-2,3-dihydro-1H-benzo[d]imidazol-4-yl)oxy)butanoic acid;
   (E)-1-((E)-4-((E)-5-carbamoyl-2-((1-ethyl-3-methyl-1H-pyrazole-5-carbonyl)imino)-7-methoxy-2,3-dihydro-1H-benzo[d]imidazol-1-yl)but-2-en-1-yl)-7-(3-(dimethylamino)propoxy)-2-((1-ethyl-3-methyl-1H-pyrazole-5-carbonyl)imino)-2,3-dihydro-1H-benzo[d]imidazole-5-carboxamide;
   (E)-1-((E)-4-((E)-5-carbamoyl-2-((1-ethyl-3-methyl-1H-pyrazole-5-carbonyl)imino)-7-(3-(4-(2-hydroxyethyl)piperazin-1-yl)propoxy)-2,3-dihydro-1H-benzo[d]imidazol-1-yl)but-2-en-1-yl)-2-((1-ethyl-3-methyl-1H-pyrazole-5-carbonyl)imino)-7-methoxy-2,3-dihydro-1H-benzo[d]imidazole-5-carboxamide;
   (E)-1-((E)-4-((E)-5-carbamoyl-2-((1-ethyl-3-methyl-1H-pyrazole-5-carbonyl)imino)-7-(3-hydroxypropoxy)-2,3-dihydro-1H-benzo[d]imidazol-1-yl)but-2-en-1-yl)-2-((1-ethyl-3-methyl-1H-pyrazole-5-carbonyl)imino)-7-methoxy-2,3-dihydro-1H-benzo[d]imidazole-5-carboxamide;
   (E)-1-(4-(5-carbamoyl-2-(1-ethyl-3-methyl-1H-pyrazole-5-carboxamido)-1H-benzo[d]imidazol-1-yl)but-2-en-1-yl)-2-(1-ethyl-3-methyl-1H-pyrazole-5-carboxamido)-7-(3-morpholinopropoxy)-1H-benzo[d]imidazole-5-carboxamide;
   (E)-1-((E)-4-((E)-5-carbamoyl-2-((1-ethyl-3-methyl-1H-pyrazole-5-carbonyl)imino)-2,3-dihydro-1H-benzo[d]imidazol-1-yl)but-2-en-1-yl)-2-((1-ethyl-3-methyl-1H-pyrazole-5-carbonyl)imino)-7-(3-morpholinopropoxy)-2,3-dihydro-1H-benzo[d]imidazole-5-carboxamide;
   (E)-1-((E)-4-((E)-5-carbamoyl-2-((1-ethyl-3-methyl-1H-pyrazole-5-carbonyl)imino)-7-(3-morpholinopropoxy)2,3-dihydro-1H-benzo[d]imidazol-1-yl)but-2-en-1-yl)-2-((1-ethyl-3-methyl-1H-pyrazole-5-carbonyl)imino)-7-methoxy-2,3-dihydro-1H-benzo[d]imidazole-5-carboxamide;
   3-(((E)-6-carbamoyl-3-((E)-4-((E)-5-carbamoyl-2-((1-ethyl-3-methyl-1H-pyrazole-5-carbonyl)imino)-7-methoxy-2,3-dihydro-1H-benzo[d]imidazol-1-yl)but-2-en-1-yl)-2-((1-ethyl-3-methyl-1H-pyrazole-5-carbonyl)imino)-2,3-dihydro-1H-benzo[d]imidazol-4-yl)oxy)propyl dihydrogen phosphate; or
   a pharmaceutically acceptable salt thereof.
10. The adjuvant composition according to any one of embodiments 1 to 9, wherein the STING agonist of Formula I is 3-(((E)-6-carbamoyl-3-((E)-4-((E)-5-carbamoyl-2-((1-ethyl-3-methyl-1H-pyrazole-5-carbonyl)imino)-7-methoxy-2,3-dihydro-1H-benzo[d]imidazol-1-yl)but-2-en-1-yl)-2-((1-ethyl-3-methyl-1H-pyrazole-5-carbonyl)imino)-2,3-dihydro-1H-benzo[d]imidazol-4-yl)oxy)propyl dihydrogen phosphate as represented by the below formula: 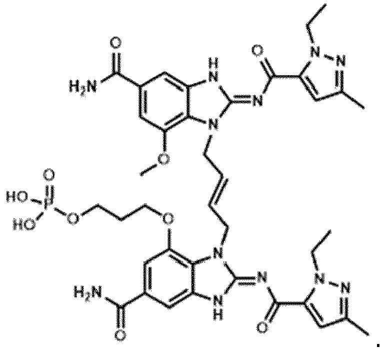
11. The adjuvant composition according to anyone of embodiments 1 to 10, wherein the adjuvant composition includes aluminium hydroxide.
12. The adjuvant composition according to embodiment 11, wherein the STING agonist is adsorbed on the aluminium hydroxide.
13. The adjuvant composition according to embodiment 12, wherein an amount of the STING agonist adsorbed on the aluminium hydroxide is greaterthan 80%, 85%, 90%, or 95%.
14. The adjuvant composition according to any one of embodiments 11 to 13, wherein the adjuvant composition includes aluminium hydroxide in an amount of 50 to 500 µg per dose.
15. The adjuvant composition according to any one embodiments 1 to 14, wherein the adjuvant composition includes an amount of STING agonist between 0.5 to 250 µg per dose.
16. The adjuvant composition according to anyone of embodiments 1 to 10, wherein the adjuvant composition includes aluminium phosphate.
17. The adjuvant composition according to embodiment 16, wherein the STING agonist is adsorbed on the aluminium phosphate.
18. The adjuvant composition according to embodiment 17, wherein an amount of the STING agonist adsorbed on the aluminium phosphate is greater than 80%, 85%, 90%, or 95%.
19. The adjuvant composition according to anyone of embodiments 1 to 10, wherein the adjuvant composition includes aluminium oxyhydroxide.
20. The adjuvant composition according to embodiment 19, wherein the STING agonist is adsorbed on the aluminium oxyhydroxide.
21. The adjuvant composition according to embodiment 20, wherein an amount of the STING agonist adsorbed on the aluminium oxyhydroxide is greater than 80%, 85%, 90%, or 95%.
22. The adjuvant composition according to anyone of embodiments 1 to 10, wherein the adjuvant composition includes aluminium hydroxyphosphate.
23. The adjuvant composition according to embodiment 22, wherein the STING agonist is adsorbed on the aluminium hydroxyphosphate.
24. The adjuvant composition according to embodiment 23, wherein an amount of the STING agonist adsorbed on the aluminium hydroxyphosphate is greater than 80%, 85%, 90%, or 95%.
25. An immunogenic composition comprising:
   (i) the adjuvant composition according to any one of embodiments 1 to 24; and
   (ii) an antigen.
26. The immunogenic composition according to embodiment 25, wherein the antigen is derived from a cancer cell.
27. The immunogenic composition according to embodiment 25, wherein the antigen is derived from a human pathogen.
28. The immunogenic composition according to embodiment 27, wherein the antigen is derived from a human pathogen selected from the group consisting of bacteria, virus, fungi, parasitic microorganisms and multicellular parasites.
29. The immunogenic composition according to any one of embodiments 25-28, wherein the antigen is a polypeptide-containing antigen.
30. The immunogenic composition according to embodiment 28, wherein the human pathogen is selected from coronavirus, Herpes simplex virus (HSV), HIV, hepatitis B virus, hepatitis C virus, meningitis B, Haemophilus influenza type B, pertussis, diphtheria, tetanus, influenza virus, RSV, HPV, measles, rubella virus, mumps virus, HCMV, VZV, Dengue virus, poliovirus, HBV, Ebola virus and rotavirus, or any combination of two or more thereof.
31. The immunogenic composition according to embodiment 30, wherein the antigen is a VZV antigen.
32. The immunogenic composition according to embodiment 30, wherein the antigen is a HSV antigen.
33. The immunogenic composition according to embodiment 32, wherein the antigen is a HSV-2 gE-gI antigen.
34. The immunogenic composition according to embodiment 30, wherein the antigen is derived from at least one coronavirus.
35. The immunogenic composition according to embodiment 34, wherein the SARS-CoV-2 antigen is a SARS-CoV-2 S protein.
36. The immunogenic composition according to embodiment 35, wherein the SARS-CoV-2 S protein is a pre-fusion stabilised S protein.
37. The immunogenic composition according to embodiment 36, wherein the SARS-CoV-2 S protein comprises an amino acid sequence having at least 90% identity to the amino acid sequence set forth in SEQ ID NO:1.
38. The immunogenic composition according to any of embodiments 25-37, wherein the adjuvant composition includes aluminium hydroxide and the antigen is adsorbed on the aluminium hydroxide.
39. The immunogenic composition according to embodiment 38, wherein the antigen and STING agonist are adsorbed on to the same aluminium hydroxide.
40. The immunogenic composition according to embodiment 38, wherein the antigen and STING agonist are adsorbed on to different aluminium hydroxide.
41. The immunogenic composition according to any one of embodiments 38 to 40, wherein an amount of the antigen bound to the aluminium hydroxide is greater than 80%, 85%, 90%, or 95%.
42. The immunogenic composition according to any of embodiments 25-37, wherein the adjuvant composition includes aluminium phosphate and the antigen is adsorbed on the aluminium phosphate.
43. The immunogenic composition according to embodiment 42, wherein the antigen and STING agonist are adsorbed on to the same aluminium phosphate.
44. The immunogenic composition according to embodiment 42, wherein the antigen and STING agonist are adsorbed on to different aluminium phosphate.
45. The immunogenic composition according to any one of embodiments 42 to 44, wherein an amount of the antigen bound to the aluminium phosphate is greater than 80%, 85%, 90%, or 95%.
46. The immunogenic composition according to any of embodiments 25-37, wherein the adjuvant composition includes aluminium oxyhydroxide and the antigen is adsorbed on the aluminium oxyhydroxide.
47. The immunogenic composition according to embodiment 46, wherein the antigen and STING agonist are adsorbed on to the same aluminium oxyhydroxide.
48. The immunogenic composition according to embodiment 46, wherein the antigen and STING agonist are adsorbed on to different aluminium oxyhydroxide.
49. The immunogenic composition according to any one of embodiments 46 to 48, wherein an amount of the antigen bound to the aluminium oxyhydroxide is greater than 80%, 85%, 90%, or 95%.
50. The immunogenic composition according to any of embodiments 25-37, wherein the adjuvant composition includes aluminium hydroxyphosphate and the antigen is adsorbed on the aluminium hydroxyphosphate.
51. The immunogenic composition according to embodiment 50, wherein the antigen and STING agonist are adsorbed on to the same aluminium hydroxyphosphate.
52. The immunogenic composition according to embodiment 50, wherein the antigen and STING agonist are adsorbed on to different aluminium hydroxyphosphate.
53. The immunogenic composition according to any one of embodiment 50 to 52, wherein an amount of the antigen bound to the aluminium hydroxyphosphate is greater than 80%, 85%, 90%, or 95%.
54. The immunogenic composition according to any one of embodiments 25 to 52 for use in therapy.
55. The adjuvant composition according to any one of embodiments 1 to 24 for use in a method of immunizing a host comprising administering to the host an adjuvant composition of any one of embodiments 1 to 24 and an antigen.
56. A method of immunizing a host comprising administering to the host an adjuvant composition according to any one of embodiments 1 to 24 and an antigen.
57. The method of embodiment 56, wherein the antigen is formulated in a separate composition to the adjuvant composition, and is administered separately.
58. A method of immunizing a host comprising administering to the host an immunogenic composition according to any one of embodiments 25 to 53.
59. A combination of an adjuvant composition according to any one of embodiments 1 to 24, and a vaccine formulation comprising an antigen.
60. A method of adjuvanting an immune response in a subject, said method comprising administering the adjuvant composition according to any one of embodiments 1 to 24.
61. Use of an adjuvant composition according to any one of embodiments 1 to 21 in the manufacture of a medicament for adjuvanting an immune response in a subject.
62. A kit comprising i) a first container comprising the adjuvant composition as defined in any one of embodiments 1 to 14 and ii) a second container comprising an antigen as defined in embodiments 26 to 36.

## Claims

1. An adjuvant composition comprising:
(v) a STING agonist of Formula (I) or a pharmaceutically acceptable salt thereof 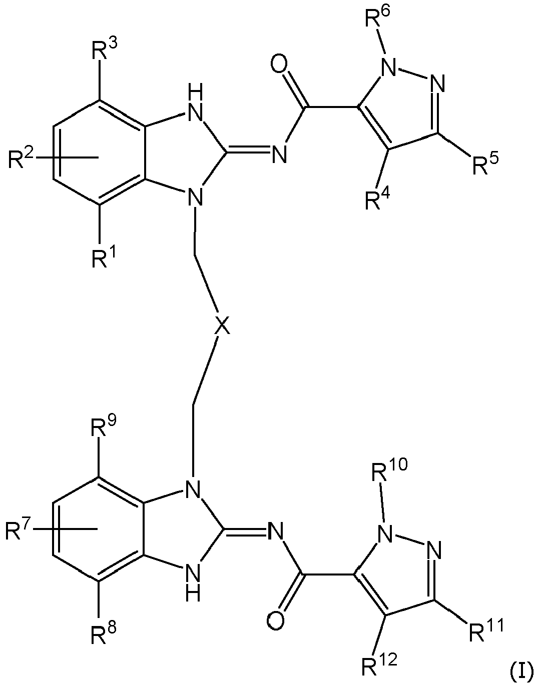 wherein
X is ―halo(C₁-C₅)alkyl, unsubstituted ―C₁-C₅ alkyl, or unsubstituted ―C₂-C₅ alkenyl;
R¹ and R⁹ are independently H, halogen, hydroxyl, ―O―P(O)(OH)₂, ―O―P(O)(R^{I}R^{II})₂, optionally substituted C₁-C₆ alkyl or optionally substituted C₁-C₆ alkyloxy,
wherein optionally substituted means substituted with 1-4 substituents each independently selected from the group consisting of hydroxyl, ―O―P(O)(OH)₂, ―O-P(O)(R^{I}R^{II})₂, C₁-C₄ alkoxyl, -N(R^{A})₂, -CO₂(R^{B}), optionally substituted phenyl, and optionally substituted 5-6 membered heterocycloalkyl, wherein said optionally substituted phenyl, or optionally substituted 5-6 membered heterocycloalkyl is optionally substituted by 1-4 substituents each independently selected from halogen, hydroxy, ―O―P(O)(OH)₂, ―O― P(O)(R^{I}R^{II})₂, amino, (C₁-C₆ alkyl)amino-, (C₁-C₆ alkyl)(C₁-C₆ alkyl)amino-, halo(C₁-C₆ alkyl), hydroxy-(C₁-C₄ alkyl)-, ―(C₁-C₄ alkyl)-O-P(O)(OH)₂, -(C₁-C₄alkyl)-O-P(O)(R^{I}R^{II})₂, halo(C₁-C₄ alkoxy)-, C₁-C₄ alkoxy-, hydroxy―(C₂-C₄ alkoxy)-, ―(C₂-C₄ alkoxy)―O―P(O)(OH)₂, ―(C₂-C₄ alkoxy)-O-P(O)(R^{I}R^{II})₂, -(C₁-C₆ alkyl)-NH2, -C1-C4 alkyl―(C₁-C₄ alkoxyl) and C1-C4 alkoxy-(Ci-C4 alkoxy)-, wherein R^{A} and R^{B} are each independently selected from hydrogen, ―C₁-C₄ alkyl, ―CO(C₁-C₄ alkyl), ―OCO(C₁-C₄ alkyl), ―(C₁-C₄ alkyl)-NH2, ―(C₁-C₄ alkyl)-C1-C4 alkoxyl, or ―CO₂(C₁-C₄ alkyl),
R² and R⁷ are each independently hydrogen, ―CON(R^{C})₂, -COOH, or CO₂(R^{D}), or one of R² and R⁷ is ―CON(R^{C})(R^{D}) and the other is H, -COOH, or CO₂(R^{E}), wherein R^{C} and R^{D} are each independently selected from hydrogen, ―C₁-C₄ alkyl, ―CO(C₁-C₄ alkyl),-OCO(C₁-C₄ alkyl), ―(C₁-C₄ alkyl)-NH2, ―(C₁-C₄ alkyl)-C₁-C₄ alkoxyl, or ―CO₂(C₁-C₄ alkyl); R³ and R⁸ are each independently H, halo(C₁-C₆alkyl), halo(C₁-C₆alkoxy)-, hydroxy, -O-P(O)(OH)₂, -O-P(O)(R^{I}R^{II})₂, -NR^{c}R^{d}, -COR^{c}, -CO₂R^{c}, -N(R^{d})COR^{c}, -N(R^{d})S O₂R^{c}, -N(R^{g})SO₂(C₁-C₂alkyl)-N(R^{h})(R^{f}), -N(R^{g})CO(C₁-C₂alkyl)-N(R^{h})(R^{f});
R^{e}, R^{f}, R^{g}, and R^{h} are each independently H or C1-C4 alkyl;
R⁴, R⁵, R¹¹ and R¹² are each independently H or C1-C4 alkyl;
R⁶ and R¹⁰ are each C₁-C₄ alkyl; and
each occurrence of R^{I} and R^{II} is independently C1-C6 alkyloxy-; and
(vi) aluminium hydroxide, aluminium phosphate, aluminium oxyhydroxide, or aluminium hydroxyphosphate.

2. The adjuvant composition according to claim 1, wherein the STING agonist has the structure of Formula (II) or a pharmaceutically acceptable salt thereof 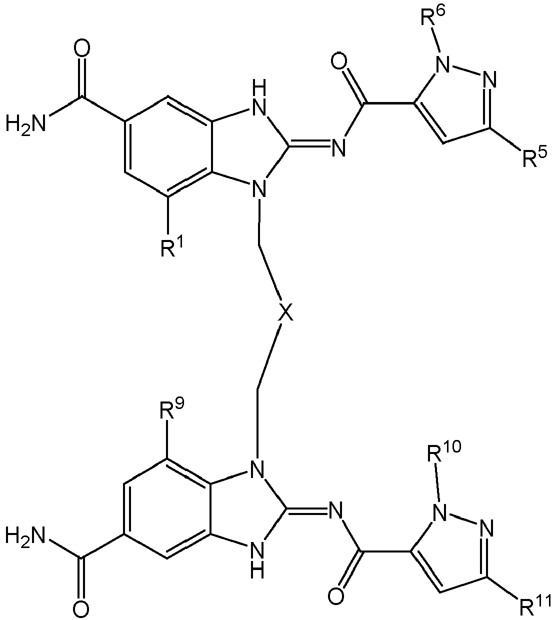 wherein X, R¹, R⁵, R⁶, R⁹, R¹⁰ and R¹¹ are as defined in claim 1.

3. The adjuvant composition according to claim 1 or 2, wherein R¹ and R⁹ are each independently H, halogen, optionally substituted (C₁-C₆alkyl), or optionally substituted (C₁-C₆alkyl)oxy-, and the C₁-C₆alkyl of said optionally substituted (C₁-C₆alkyl), optionally substituted (C₁-C₆alkyl)oxy-is optionally substituted with 1-4 substituents each independently selected from the group consisting of hydroxyl,―O-P(O)(OH)₂, ―O-P(O)(R^{I}R^{II})₂, -N(R^{e})(R^{f}), C₁-C₄alkoxyl, phenyl, optionally substituted 5-6 membered heterocycloalkyl containing at least one nitrogen or oxygen as a member of the ring, each R^{e} is independently selected from H, (C₁-C₄alkyl),-(C₁-C₄alkyl)-NH₂, or -(C₁-C₄alkyl) C₁-C₄alkoxy and each R^{f} is independently H or (C₁-C₄alkyl); or
wherein one of R¹ and R⁹ is ―O―P(O)(OH)₂, ―O-P(O)(R^{I}R^{II})₂, C1-C6 alkyl substituted with ―O―P(O)(OH)₂ or ―O-P(O)(R^{I}R^{II})₂, or a C₁-C₆ alkyloxy group; or
wherein at least one of R¹ and R⁹ is selected from the following groups: 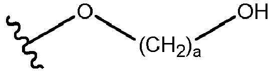 where a is a number from 1 to 6; 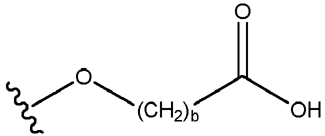 where b is a number from 1 to 6; 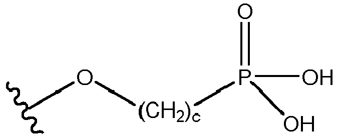 where c is a number from 1 to 6; 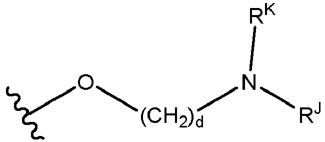 where d is a number from 1 to 6, and R^{J} and R^{K} are C1-C3 alkyl; or 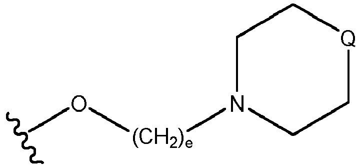 where e is a number from 1 to 6, and Q is selected from O or N(R^{X}) wherein R^{X} is a C1-C6 alkyl or C1-C6 alkoxy group.

4. The adjuvant composition according to any one of claims 1 to 3, wherein the STING agonist is selected from the group consisting of:
(E)-1-((E)-4-((E)-5-carbamoyl-2-((1-ethyl-3-methyl-1H-pyrazole-5-carbonyl)imino)-2,3-dihydro-1H-benzo[d]imidazol-1-yl)but-2-en-1-yl)-2-((1-ethyl-3-methyl-1H-pyrazole-5-carbonyl)imino)-7-(3-hydroxypropoxy)-2,3-dihydro-1H-benzo[d]imidazole-5-carboxamide;
4-(((E)-6-carbamoyl-3-((E)-4-((E)-5-carbamoyl-2-((1-ethyl-3-methyl-1H-pyrazole-5-carbonyl)imino)-2,3-dihydro-1H-benzo[d]imidazol-1-yl)but-2-en-1-yl)-2-((1-ethyl-3-methyl-1H-pyrazole-5-carbonyl)imino)-2,3-dihydro-1H-benzo[d]imidazol-4-yl)oxy)butanoic acid;
(E)-1-((E)-4-((E)-5-carbamoyl-2-((1-ethyl-3-methyl-1H-pyrazole-5-carbonyl)imino)-7-methoxy-2,3-dihydro-1H-benzo[d]imidazol-1-yl)but-2-en-1-yl)-7-(3-(dimethylamino)propoxy)-2-((1-ethyl-3-methyl-1H-pyrazole-5-carbonyl)imino)-2,3-dihydro-1H-benzo[d]imidazole-5-carboxamide;
(E)-1-((E)-4-((E)-5-carbamoyl-2-((1-ethyl-3-methyl-1H-pyrazole-5-carbonyl)imino)-7-(3-(4-(2-hydroxyethyl)piperazin-1-yl)propoxy)-2,3-dihydro-1H-benzo[d]imidazol-1-yl)but-2-en-1-yl)-2-((1-ethyl-3-methyl-1H-pyrazole-5-carbonyl)imino)-7-methoxy-2,3-dihydro-1H-benzo[d]imidazole-5-carboxamide;
(E)-1-((E)-4-((E)-5-carbamoyl-2-((1-ethyl-3-methyl-1H-pyrazole-5-carbonyl)imino)-7-(3-hydroxypropoxy)-2,3-dihydro-1H-benzo[d]imidazol-1-yl)but-2-en-1-yl)-2-((1-ethyl-3-methyl-1H-pyrazole-5-carbonyl)imino)-7-methoxy-2,3-dihydro-1H-benzo[d]imidazole-5-carboxamide;
(E)-1-(4-(5-carbamoyl-2-(1-ethyl-3-methyl-1H-pyrazole-5-carboxamido)-1H-benzo[d]imidazol-1-yl)but-2-en-1-yl)-2-(1-ethyl-3-methyl-1H-pyrazole-5-carboxamido)-7-(3-morpholinopropoxy)-1H-benzo[d]imidazole-5-carboxamide;
(E)-1-((E)-4-((E)-5-carbamoyl-2-((1-ethyl-3-methyl-1H-pyrazole-5-carbonyl)imino)-2,3-dihydro-1H-benzo[d]imidazol-1-yl)but-2-en-1-yl)-2-((1-ethyl-3-methyl-1H-pyrazole-5-carbonyl)imino)-7-(3-morpholinopropoxy)-2,3-dihydro-1H-benzo[d]imidazole-5-carboxamide;
(E)-1-((E)-4-((E)-5-carbamoyl-2-((1-ethyl-3-methyl-1H-pyrazole-5-carbonyl)imino)-7-(3-morpholinopropoxy)-2,3-dihydro-1H-benzo[d]imidazol-1-yl)but-2-en-1-yl)-2-((1-ethyl-3-methyl-1H-pyrazole-5-carbonyl)imino)-7-methoxy-2,3-dihydro-1H-benzo[d]imidazole-5-carboxamide;
3-(((E)-6-carbamoyl-3-((E)-4-((E)-5-carbamoyl-2-((l-ethyl-3-methyl-1H-pyrazole-5-carbonyl)imino)-7-methoxy-2,3-dihydro-1H-benzo[d]imidazol-1-yl)but-2-en-1-yl)-2-((1-ethyl-3-methyl-1H-pyrazole-5-carbonyl)imino)-2,3-dihydro-1H-benzo[d]imidazol-4-yl)oxy)propyl dihydrogen phosphate; or
a pharmaceutically acceptable salt thereof.

5. The adjuvant composition according to any one of claims 1 to 4, wherein the STING agonist of Formula I is 3-(((E)-6-carbamoyl-3-((E)-4-((E)-5-carbamoyl-2-((1-ethyl-3-methyl-1H-pyrazole-5-carbonyl)imino)-7-methoxy-2,3-dihydro-1H-benzo[d]imidazol-1-yl)but-2-en-1-yl)-2-((1-ethyl-3-methyl-1H-pyrazole-5-carbonyl)imino)-2,3-dihydro-1H-benzo[d]imidazol-4-yl)oxy)propyl dihydrogen phosphate as represented by the below formula:

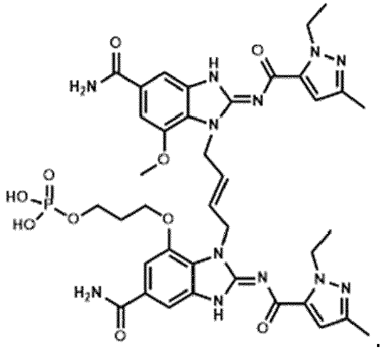

6. The adjuvant composition according to any one of claims 1 to 5, wherein the adjuvant composition includes aluminium hydroxide.

7. The adjuvant composition according to claim6, wherein the STING agonist is adsorbed on the aluminium hydroxide.

8. The adjuvant composition according to any one of claims 6 or 7, wherein the adjuvant composition includes aluminium hydroxide in an amount of 50 to 500 µg per dose; and/or
wherein the adjuvant composition includes an amount of STING agonist between 0.5 to 250 µg per dose.

9. An immunogenic composition comprising:
(iii) the adjuvant composition according to any one of claims 1 to8; and
(iv) an antigen.

10. The immunogenic composition according to claim 9, wherein the antigen is derived from a cancer cell; or
wherein the antigen is derived from a human pathogen; or
wherein the antigen is derived from a human pathogen selected from the group consisting of bacteria, virus, fungi, parasitic microorganisms and multicellular parasites.

11. The immunogenic composition according to claim 10, wherein the antigen is derived from a human pathogen and the human pathogen is selected from coronavirus, Herpes simplex virus (HSV), HIV, hepatitis B virus, hepatitis C virus, meningitis B, Haemophilus influenza type B, pertussis, diphtheria, tetanus, influenza virus, RSV, HPV, measles, rubella virus, mumps virus, HCMV, VZV, Dengue virus, poliovirus, HBV, Ebola virus and rotavirus, or any combination of two or more thereof.

12. The immunogenic composition according to claim 11, wherein the antigen is a VZV antigen; or
wherein the antigen is a HSV antigen; or
wherein the antigen is a HSV-2 gE-gI antigen.

13. The immunogenic composition according to any of claims 9-12, wherein the adjuvant composition includes aluminium hydroxide and the antigen is adsorbed on the aluminium hydroxide; or
wherein the antigen and STING agonist are adsorbed on to the same aluminium hydroxide; or
wherein the antigen and STING agonist are adsorbed on to different aluminium hydroxide.

14. A method of immunizing a host comprising administering to the host: i) an adjuvant composition according to any one of claims 1 to 8 and an antigen, optionally wherein the antigen is formulated in a separate composition to the adjuvant composition, and is administered separately; or
ii) an immunogenic composition according to any one of claims 9 to 13.

15. A kit comprising i) a first container comprising the adjuvant composition as defined in any one of claims 1 to 8 and ii) a second container comprising an antigen as defined in claims 10 to 12.
